# EUROPEAN PATENT APPLICATION

(11) **EP 2 199 937 A1**
(43) Date of publication of application: **23.06.2010**
(21) Application number: 10157206.3
(22) Date of filing: 20.10.2003
(51) Int. Cl.: G06F 19/00

(54) **Medical image radiographing system, medical image management method and portable terminal**

(30) Priority: 31.10.2002 JP 2002317258; 24.03.2003 JP 2003079711; 24.03.2003 JP 2003079769; 28.03.2003 JP 2003091018
(62) Divisional of application: 03256590.5
(71) Applicant: Konica Minolta Business Technologies, Inc., Tokyo 100-0005 (JP)
(72) Inventor: Moriyama, Naoto, Tokyo 192-8505 (JP); Motoki, Wataru, Tokyo 192-8505 (JP); Shiibashi, Takao, Tokyo 192-8505 (JP)
(74) Representative: Brown, George Laurence

(57) **Abstract**

A medical image radiograping system capable of modifying radiographing order information suitably. In the system, a control apparatus has: an obtaining section for obtaining identification information of a cassette; a storage for storing the identification information related to radiographing order information, and storing the radiographing order information renewed according to radiographing; and a communication section for transmitting the radiographing order information and the identification information, and the control apparatus has: a storage for storing radiographing order information; a communication section for receiving the radiographing order information and the identification information; a determination section for determining whether the radiographing order information received agrees with the radiographing order information stored or not; and a management section for controlling both the radiographing order information stored and the radiographing order information received and the identification information of the cassete, according to a result determined by the determination section.

## Description

### BACKGROUND OF THE INVENTION

### Field of the Invention

The invention relates to a medical image radiographing system, a medical image management method and a portable terminal used in the medical image radiographing system, for radiographing a medical image with radiation such as X-ray or the like.

### Description of Related Art

In recent years, a user can easily input and output information with a portable information terminal such as a PDA (Personal Digital Assistance) or the like, even when moving. As technique using the PDA, for example, technique capable of storing financial and personal information of a user such as credit card information or the like in a memory of the PDA and writing the information stored in the memory of the PDA in a smart card usable in POS transactions, ATM transactions or consumer transactions, the smart card inserted after the user is identified, is disclosed. Further, as an applied example, technique capable of writing medical information stored in a PDA for a doctor in a smart media and accessing to the medical information stored in the smart media with a PDA for a patient when the patient is identified is disclosed (for example, with reference to Japanese Translation Publication of PCT International Application NO. Tokuhyo 2001-512876).

Further, in the field of medicine, in order to diagnose patient's illness, a medical image radiographing system for radiographing a medical image of the patient as an object to be examined with radiation such as X-ray or the like and obtaining the medical image as digital image data, is used.

In the medical image radiographing system, a control apparatus is connected to a information management apparatus for inputting radiographing reservation through a network, receives radiographing order information for a predetermined period from the information management apparatus, and manages the radiographing order information. Then, the control apparatus controls a medical image reading apparatus on the basis of the radiographing order information, reads radiation image information of the examined object accumulated in a photostimulable phosphor substance plate by radiographing the object with X-ray, and manages the read medical image.

Further, the medical image radiographing system comprises, for example, a CT (Computed Tomography), a CR (Computed Radiography), a MRI (Magnetic Resonance Imaging) or the like.

The above-described radiation image radiographing apparatus uses a medical image conversion panel wherein a photostimulable phosphor substance layer is formed on a supporting member. In the radiation image radiographing apparatus, radiation which has been transmitted through the object is absorbed, radiation energy corresponding to radiation transmissibility of each part of the object is accumulated, and thereby a latent image is formed, in the photostimulable phosphor substance layer of the conversion panel. Then, when the phosphor substance layer is scanned with stimulated excitation light such as infrared rays or the like, the accumulated radiation energy is radiated as phosphor light, the phosphor light is converted photoelectrically, and thereby a medical image signal is obtained. The medical image obtained as described above is processed, outputted to an output apparatus such as a film, a CRT or the like to be visible, stored with patient information in a filing apparatus such as a server or the like, and used in medical operations.

The medical image radiographing system using the above-described radiation image radiographing apparatus is classified into two system structures roughly.

One of two system structures is a general medical image radiographing system for providing the radiographing apparatus of images (for example, a X-ray apparatus), the reading apparatus and the photostimulable phosphor substance plate in a radiographing room of a radiation department or near the radiographing room, and radiographing images in the radiographing room. The system can radiograph and read the images in the radiographing room at the same time.

The other is a medical image radiographing system for using a portable radiographing apparatus with X-ray or the like and a portable cassette incorporating the phosphor substance plate, and radiographing images of a patient who has broken, has a cerebrovascular disorder, or the like in a ward, a patient in an intensive care unit, a patient who is under medical treatment in an emergency room or an operating room (for example, with reference to Japanese Patent Application (Unexamined) No. Tokukai 2000-139888). In the system, when the cassette is inserted in the reading apparatus only for the cassette after the image is radiographed, the radiographed image is read.

In the above-described medical image radiographing system for radiographing the image in the ward or the room, a process for an operator of radiographing images, will be explained as follows. First, the operator prints radiographing order information prepared according to an instruction of a doctor on an order sheet in a HIS (Hospital Information system) in the hospital, a RIS (Radiology Information System) in the radiation department or the like. The radiographing order information includes information (Hereinafter, it will be patient information.) on a patient such as a name, a sex of the like of the patient to be radiographed or information (Hereinafter, it will be radiographing information.) on radiographing such as a radiographic part, a radiographing direction or the like. The radiographing order information indicates an instruction to radiograph the patient.

Then, the operator carries the portable radiographing apparatus, the cassette, the order sheet or the like to the room for the patient, and confirms the patient as the object to be radiographed on the basis of the radiographing order information printed on the order sheet. In order to make the cassette used to radiograph the patient clear, the operator records identification information peculiar to the cassette to be used so as to relate the identification information to the radiographing order information on the order sheet. When confirming the patient and recording the identification information of the cassette, the operator operates the radiographing apparatus, makes the radiographing apparatus radiograph the patient, and records the medical image in the cassette.

Then, after radiographing the patient, the operator returns to the radiographing room, and inputs the identification information of the cassette used for radiographing the patient so as to be related to the radiographing order information to the control apparatus on the basis of the record of the order sheet. The operator inserts the cassette in which the medical image is recorded into the reading apparatus for the cassette, and makes the recording apparatus read the medical image out of the cassette. Therefore, the control apparatus relates the medical image read by the reading apparatus to the radiographing order information.

In the above-described case of radiographing the medical image in the ward or the room, there is a case it is impossible to radiograph the patient according to the instruction of the radiographing order information due to an unexpected state. For example, there is a case it is impossible to radiograph the patient because the patient is absent, a case it is impossible to radiograph the patient in a specific radiographing direction due to patient's condition, a case the operator is instructed to add the radiographic part or the number of images to be radiograped by the doctor, or the like.

X-ray radiographing requires instructions by a doctor or a dentist, as prescribed in Article 26 of the Radiologist Law, "the radiologist cannot apply radiation to the human body in case they are not specifically instructed by a doctor or a dentist.". The X-ray radiographing in the medical image radiographing system is carried out according to the radiographing order information inputted as instructed by the doctor on the basis of the Law. Therefore, the radiographing cannot be carried out in principle if the radiographing order information is not prepared. According to an earlier development, in order to add new radiographing, the operator has to go through a complicated process of returning from the ward or the room to the location at which the control apparatus is provided, adding the radiographing order information, going to the ward or the room, and photographing the patient. As a result, extremely long time is required. Further, the operator is required to record additional or changed content on the order sheet, and modify the radiographing order information added or changed on the basis of the record of the order sheet after radiographing the patient.

After radiographing the patient, the operator is required to input the identification information of the cassette recorded on the order sheet in the control apparatus, and relate the medical image to the radiographing order information on the basis of the inputted identification information of the cassette. In case of modifying the content variously, the operator is required to carry out hard operations and complicated input processing. As a result, there occurs a case the operator makes input mistakes. In the case, there is a problem that consistency between the radiographing order information and the medical image cannot be kept.

Further, the medical image radiographing system can have a structure for adding additional information to image data so that the image data radiographed by an image data output apparatus can be outputted by an image forming apparatus such as a printer or the like (for example, with reference to Japanese Patent Application Publication (Unexamined) No. Tokukai 2002-133394).

For example, in order to radiograph the medical image with a portable X-ray radiographing apparatus disclosed in Japanese Patent Application Publication (Unexamined) No. Tokukai 2002-139888, it is required to relate the cassette in which the medical image is radiographed or to be radiographed to the medical image, and to manage the related information. However, there is a case it is impossible to obtain the related information sufficiently.

For example, in the structure that information for identifying the cassette or the like related to the radiographing order information is inputted in the portable terminal, the portable terminal transmits the information to the management apparatus, and the management apparatus receives and manages the information, when the communication between the portable terminal and the management apparatus has trouble, there is a case wherein the management apparatus cannot obtain the related information.

### SUMMARY OF THE INVENTION

A first object of the present invention is to provide a medical image radiograping system and a medical image management method capable of modifying radiographing order information easily and suitably in case radiographing different from one as instructed based on the radiographing order information is carried out, and of accurately managing radiographing order information for radiographing which is not carried out.

A second object of the present invention is to efficiently add radiographing order information on medical image radiographing with a portable terminal.

A third object of the present invention is to accurately obtain information necessary to manage radiographing with a portable radiographing appartus in a management side.

In accordance with a first aspect of the present invention, a medical image radiographing system comprises: a control apparatus for managing a medical image and radiographing order information by relating the medical image to the radiographing order information; and a portable terminal for obtaining the radiographing order information from the control apparatus, the portable terminal comprises: an obtaining section for obtaining identification information of a cassette for recording the medical image radiographed based on the radiographing order information obtained from the control apparatus; a storage for storing the identification information of the cassette obtained by relating the identification information of the cassette to the radiographing order information, and storing the radiographing order information renewed according to radiographing; and a communication section for transmitting the radiographing order information and the identification information of the cassette stored in the storage, and the control apparatus comprises: a storage for storing radiographing order information; a communication section for receiving the radiographing order information and the identification information of the cassette; a determination section for determining whether the radiographing order information received agrees with the radiographing order information stored in the storage or not; and a management section for controlling both the radiographing order information stored and the radiographing order information received and the identification information of the cassette thereto, according to a result determined by the determination section.

Preferably, the management section stores the identification inforamtion of the casete realting to the radiographing order information stored in the storage, when the radiographing order information received agrees with the radiographing order information stored.

In accordance with a second aspect of the present invention, a medical image management method for a medical image radiographing system comprising a control apparatus for managing a medical image and radiographing order information by relating the medical image to the radiographing order information and a portable terminal for obtaining the radiographing order information from the control apparatus, comprises: obtaining identification information of a cassette for recording the medical image radiographed based on the radiographing order information obtained from the control apparatus; storing the identification information of the cassette obtained by relating the identification information of the cassette to the radiographing order information, and storing the radiographing order information renewed according to radiographing; transmitting the radiographing order information and the identification information of the cassette stored; storing radiographing order information in a storage; receiving the radiographing order information and the identification information of the cassette; determining whether the radiographing order information received agrees with the radiographing order information stored in the storage or not; and controlling both the radiographing order information stored and the radiographing order information received and the identification information of the cassette thereto, according to a result determined.

Preferably, the method further comprises storing the identification inforamtion of the casete realting to the radiographing order information stored in the storage, when the radiographing order information received agrees with the radiographing order information stored.

According to the system or the method, only when the radiographing order information stored in the storage of the control apparatus agrees with the radiographing order information transmitted from the portable terminal to the control apparatus, it is possible that the control apparatus relates the identification information of the cassette ID transmitted from the portable terminal to the control apparatus to the radiographing order information and stores them in the storage. Consequently, it is possible to prevent the trouble that the control apparatus stores the radiographing order information which disagrees with the radiographing order information stored in the storage, and to relate and manage the radiographing order information and the identification information of the cassette efficiently.

Preferably, in the system of the first aspect of the present invention, the control apparatus further comprises: a display control section for displaying a message for confirming whether to renew the radiographing order information stored in the storage or not, when determining that the radiographing order information received disagrees with the radiographing order information stored in the storage; and an input control section for inputting an instruction to renew the radiographing order information stored in the storage or not, and the management section stores the radiographing order information received in the storage by renewing the radiographing order information stored in the storage to the radiographing order information received, and stores the identification information of the cassette in the storage by relating the identification information of the cassette to the radiographing order information, when the instruction to renew the radiographing order information stored in the storage is inputted.

Preferably, the method of the second aspect of the present invention further comprises:
displaying a message for confirming whether to renew the radiographing order information stored in the storage or not, when determining that the radiographing order information received disagrees with the radiographing order information stored in the storage; inputting an instruction to renew the radiographing order information stored in the storage or not; and storing the radiographing order information received in the storage by renewing the radiographing order information stored in the storage to the radiographing order information received, and storing the identification information of the cassette in the storage by relating the identification information of the cassette to the radiographing order information, when the instruction to renew the radiographing order information stored in the storage is inputted.

According to the system or the method, when the radiographing order information stored in the storage of the control apparatus disagrees with the radiographing order information transmitted from the portable terminal to the control apparatus, it is possible to display the message for confirming to renew the radiographing order information stored in the storage or not, and to make an operator or the like input the instruction to renew the radiographing order information or not. Consequently, when the radiographing order information stored in the storage disagrees with the radiographing order information transmitted from the portable terminal to the control apparatus, it is possible to renew the radiographing order information stored in the storage according to decision of the operator. Even when radiographing is not carried out according to the instruction based on the radiographing order information, it is possible to modify and manage the radiographing order information according to the change.

Preferably, in the control apparatus of the above-described system, the input control section inputs modification to the radiographing order information received, and the management section stores the radiographing order information modified in the storage by renewing the radiographing order information stored in the storage to the radiographing order information modified, and stores the identification information of the cassette in the storage by relating the identification information of the cassette to the radiographing order information, when the instruction to renew the radiographing order information stored in the storage is inputted.

Preferably, the above-described method further comprises: inputting modification to the radiographing order information received; and storing the radiographing order information modified in the storage by renewing the radiographing order information stored in the storage to the radiographing order information modified, and storing the identification information of the cassette in the storage by relating the identification information of the cassette to the radiographing order information, when the instruction to renew the radiographing order information stored in the storage is inputted.

According to the system or the method, when the radiographing order information stored in the storage disagrees with the radiographing order information transmitted from the portable terminal to the control apparatus, for example, when the radiographing order information is changed according to a radiographing state, or when the radiographing order information stored in the portable terminal is changed due to the factor undesired by the operator, the control apparatus can modify or add the changed part, and can manage the radiographing order information suitably.

Preferably, in the control apparatus of the above-described system, the storage stores a transmission history for indicating whether the radiographing order information is transmitted to the portable terminal or not, the display control section displays a message for confirming whether to cancel the radiographing order information received or not, when determining that the radiographing order information received disagrees with the radiographing order information stored in the storage, the input control section inputs an instruction to cancel the radiographing order information received or not, and the management section transmits the instruction to cancel the radiographing order information received to the portable terminal by controlling the communication section, and stores the transmission history of the radiographing order information stored, renewed to be not transmitted in the storage, when the instruction to cancel the radiographing order information received is inputted, and in the portable terminal, the communication section receives the instruction to cancel the radiographing order information transmitted from the control apparatus, and the storage deletes the radiographing order information corresponding to the instruction to cancel the radiographing order information received.

Preferably, the above-described method further comprises: storing a transmission history for indicating whether the radiographing order information is transmitted to the portable terminal or not; displaying a message for confirming whether to cancel the radiographing order information received or not, when determining that the radiographing order information received disagrees with the radiographing order information stored in the storage; inputting an instruction to cancel the radiographing order information received or not; transmitting the instruction to cancel the radiographing order information received to the portable terminal, and storing the transmission history of the radiographing order information stored, renewed to be not transmitted in the storage, when the instruction to cancel the radiographing order information received is inputted; receiving the instruction to cancel the radiographing order information transmitted; and deleting the radiographing order information corresponding to the instruction to cancel the radiographing order information received.

According to the system or the method, when the radiographing order information stored in the storage disagrees with the radiographing order information transmitted from the portable terminal to the control apparatus, the control apparatus displays the message for confirming to cancel the radiographing order information or not, and makes the operator or the like input the instruction to cancel the radiographing order information or not. When the instruction to cancel the radiographing order information is inputted, the control apparatus sends the instruction to cancel the radiographing order information to the portable terminal, and deletes the radiographing order information stored in the portable terminal. Consequently, for example, when the identification information is not related to the radiographing order information transmitted from the portable terminal to the control apparatus, it is possible that the operator determines that the radiographing is not carried out, and that the radiographing order information stored in the portable terminal is deleted. As a result, the radiographing order information for the radiographing which is not carried out is not kept in the portable terminal for a long time. Consequently, it is possible to prevent the operator or the like from misunderstanding the radiographing order information for the radiographing which is forgotten to be carried out and preparing the radiographing.

Preferably, the system of the first aspect of the present invention, further comprises an information management apparatus for transmitting radiographing order information to the control apparatus, wherein the management section in the control apparatus transmits the radiographing order information and the identification information of the cassette stored in the storage to the information management apparatus, by controlling the communication section, and the information management apparatus comprises: a communication section for receiving the radiographing order information and the identification information of the cassette; and a storage for storing the radiographing order information and the identification information of the cassette received.

Preferably, the method of the second aspect of the present invention further comprises:
transmitting the radiographing order information and the identification information of the cassette stored in the storage to an information management apparatus; receiving the radiographing order information and the identification information of the cassette; and
storing the radiographing order information and the identification information of the cassette received.

According to the system or the method, the control apparatus can transmit the radiographing order information and the identification information on the cassette stored in the storage to the information management apparatus, and the information management apparatus can manage the radiographing order information and the identification information on the caste received from the control apparatuses in a concentrated way. That is, the information management apparatus can receive the radiographing order information and the identification information which are newest information, and manage the whole medical image radiographing system efficiently on the basis of the information.

In accordance with a third aspect of the present invention, a medical image management method for a medical image radiographing system comprising a control apparatus for managing a medical image and radiographing order information by relating the medical image to the radiographing order information and a portable terminal for obtaining the radiographing order information from the control apparatus, comprises: obtaining identification information of a cassette for recording the medical image radiographed based on the radiographing order information obtained from the control apparatus; storing the identification information of the cassette obtained by relating the identification information of the cassette to the radiographing order information in the portable terminal, and storing the radiographing order information renewed according to radiographing in the potable terminal; transmitting the radiographing order information and the identification information of the cassette stored in the portable, from the portable terminal to the control apparatus; storing radiographing order information in a storage of the control apparatus; determining whether the radiographing order information received by the control apparatus agrees with the radiographing order information stored in the storage or not; and storing the identification information of the cassette received by the control apparatus in the storage by relating the identification information of the cassette to the radiographing order information stored in the storage when determining that the radiographing order information received by the control apparatus agrees with the radiographing order information stored in the storage.

In accordance with a fourth aspect of the present invention , a medical image radiographing system comprises: a control apparatus for managing a medical image and radiographing order information by relating the medical image to the radiographing order information; and a portable terminal for obtaining the radiographing order information from the control apparatus, the portable terminal comprises: an obtaining section for obtaining identification information of a cassette for recording the medical image radiographed based on the radiographing order information obtained from the control apparatus; a storage for storing the identification information of the cassette obtained by relating the identification information of the cassette to the radiographing order information; an editing section for editing the radiographing order information stored in the storage; and a communication section for transmitting the radiographing order information and the identification information of the cassette stored in the storage, and the control apparatus comprises: a storage for storing radiographing order information; a communication section for receiving the radiographing order information and the identification information of the cassette; and a management section for storing the radiographing order information received in the storage by renewing the radiographing order information stored in the storage to the radiographing order information received, and storing the identification information of the cassette in the storage by relating the identification information of the cassette to the radiographing order information.

In accordance with a fifth aspect of the present invention, a medical image management method for a medical image radiographing system comprising a control apparatus for managing a medical image and radiographing order information by relating the medical image to the radiographing order information and a portable terminal for obtaining the radiographing order information from the control apparatus, comprises: obtaining identification information of a cassette for recording the medical image radiographed based on the radiographing order information obtained from the control apparatus; storing the identification information of the cassette obtained by relating the identification information of the cassette to the radiographing order information; editing the radiographing order information stored; transmitting the radiographing order information and the identification information of the cassette stored; storing radiographing order information in a storage; receiving the radiographing order information and the identification information of the cassette; and storing the radiographing order information received in the storage by renewing the radiographing order information stored in the storage to the radiographing order information received, and storing the identification information of the cassette in the storage by relating the identification information of the cassette to the radiographing order information.

According to the system of the fourth aspect or the method of the fifth aspect of the present invention, for example, when the radiographing state is changed according to a condition of a patient or the like, it is possible to edit the radiographing order information according to the changed content by the portable terminal. Further, because the portable terminal transmits the edited radiographing order information to the control apparatus, and the control apparatus renews the radiographing order information on the basis of the received radiographing order information, it is possible to manage the identification information of the cassette on the basis of the newest radiographing order information.

Preferably, in the system of the fourth aspect of the present invention, the control apparatus further comprises a determination section for determining whether the radiographing order information received agrees with the radiographing order information stored in the storage or not, the communication section transmits a message for confirming whether to renew the radiographing order information stored in the storage or not to the portable terminal, and receives an instruction to renew the radiographing order information stored in the storage or not from the portable terminal, when the radiographing order information received disagrees with the radiographing order information stored in the storage, and the management section stores the radiographing order information received in the storage by renewing the radiographing order information stored in the storage to the radiographing order information received, and stores the identification information of the cassette in the storage by relating the identification information of the cassette to the radiographing order information, when receiving the instruction to renew the radiographing order information stored in the storage from the portable terminal.

Preferably, the method of the fifth aspect of the present invention, further comprises:
determining whether the radiographing order information received agrees with the radiographing order information stored in the storage or not; transmitting a message for confirming whether to renew the radiographing order information stored in the storage or not, when the radiographing order information received disagrees with the radiographing order information stored in the storage; receiving an instruction to renew the radiographing order information stored in the storage or not from the portable terminal; and storing the radiographing order information received in the storage by renewing the radiographing order information stored in the storage to the radiographing order information received, and storing the identification information of the cassette in the storage by relating the identification information of the cassette to the radiographing order information, when receiving the instruction to renew the radiographing order information stored in the storage from the portable terminal.

According to the system or the method, when the radiographing order information received from the portable terminal disagrees with the radiographing order information stored in the storage, the control apparatus can send the confirmation message to the portable terminal, renew the radiographing order information stored in the storage according to the instruction received from the portable terminal, and store it in the storage. For example, when the radiographing order information is edited according to the radiographing state, the operator can confirm the edited radiographing order information, send the instruction to renew the radiographing order information, and make the control apparatus renew the radiographing order information. As a result, it is possible to improve confidence in the radiographing order information.

Preferably, in the control apparatus of the above-described system, the management section stores the identification information of the cassette in the storage by relating the identification information of the cassette to the radiographing order information stored in the storage when receiving the instruction not to renew the radiographing order information stored in the storage from the portable terminal.

Preferably, the above-described method further comprises: storing the identification information of the cassette in the storage by relating the identification information of the cassette to the radiographing order information stored in the storage when receiving the instruction not to renew the radiographing order information stored in the storage from the portable terminal.

According to the system or the method, when the control apparatus receives the instruction not to renew the radiographing order information stored in the storage, the control apparatus does not renew the radiographing order information. Consequently, in case the radiographing order information stored in the portable terminal is edited according to the factor undesired by the operator, it is possible to manage the radiographing order information properly without renewing the radiographing order information stored in the control apparatus by mistake.

Preferably, in the control apparatus of the system of the fourth aspect of the present invention, the storage stores a transmission history for indicating whether the radiographing order information is transmitted to the portable terminal or not, the communication section transmits a message for confirming whether to cancel the radiographing order information received or not, and receives an instruction to cancel the radiographing order information received or not from the portable terminal, when the radiographing order information received disagrees with the radiographing order information stored in the storage, and the management section stores the transmission history for the radiographing order information stored in the storage, in the storage by renewing the transmission history to be not transmitted when receiving the instruction to cancel the radiographing order information received from the portable terminal.

Preferably, the method of the fifth aspect of the present invention further comprises:
storing a transmission history for indicating whether the radiographing order information is transmitted to the portable terminal or not; transmitting a message for confirming whether to cancel the radiographing order information received or not, when the radiographing order information received disagrees with the radiographing order information stored in the storage; receiving an instruction to cancel the radiographing order information received or not from the portable terminal; and storing the transmission history for the radiographing order information stored in the storage, in the storage by renewing the transmission history to be not transmitted when receiving the instruction to cancel the radiographing order information received from the portable terminal.

According to the system or the method, when the radiographing order information received disagrees with the radiographing order information stored, the control apparatus sends the confirmation message for confirming whether to cancel the radiographing order information or not to the portable terminal, and receives the instruction to cancel the radiographing order information or not from the portable terminal. When receiving the instruction to cancel the radiographing order information received, the control apparatus renews the transmission history of the corresponding radiographing order information stored to be not transmitted, and stores the transmission history. Consequently, for example, it is possible that the control apparatus again manages the radiographing order information for the radiographing which is not carried out, prevent the operator or the like from forgetting to radiographing, and distribute the operation again.

Preferably, the system of the fourth aspect of the present invention, further comprises an information management apparatus for transmitting radiographing order information to the control apparatus, the management section in the control apparatus transmits the radiographing order information and the identification information of the cassette stored in the storage to the information management apparatus, by controlling the communication section, and the information management apparatus comprises: a communication section for receiving the radiographing order information and the identification information of the cassette; and a storage for storing the radiographing order information and the identification information of the cassette received.

Preferably, the method of the fifth aspect of the present invention, further comprises:
transmitting the radiographing order information and the identification information of the cassette stored in the storage to an information management apparatus; receiving the radiographing order information and the identification information of the cassette; and
storing the radiographing order information and the identification information of the cassette received.

According to the system or the method, when the control apparatus transmits the radiographing order information and the identification information of the cassette stored in the storage to the information management apparatus, the information management apparatus can manage the radiographing order information and the identification information of the cassette received from the control apparatus in a concentrated way. That is, because the information management apparatus receives the radiographing order information and the identification information on the cassette which are renewed to newest information, the information management apparatus can manage the whole medical image radiographing system efficiently.

In accordance with a sixth aspect of the present invention, a medical image management method for a medical image radiographing system comprising a control apparatus for managing a medical image and radiographing order information by relating the medical image to the radiographing order information and a portable terminal for obtaining the radiographing order information from the control apparatus, comprises: obtaining identification information of a cassette for recording the medical image radiographed based on the radiographing order information obtained from the control apparatus; storing the identification information of the cassette obtained in the portable terminal by relating the identification information of the cassette to the radiographing order information; editing the radiographing order information stored in the portable terminal; transmitting the radiographing order information and the identification information of the cassette stored in the portable terminal, from the portable terminal to the control apparatus; storing radiographing order information in a storage of the control apparatus; and storing the radiographing order information received by the control apparatus in the storage by renewing the radiographing order information stored in the storage to the radiographing order information received by the control apparatus, and storing the identification information of the cassette in the storage by relating the identification information of the cassette to the radiographing order information.

In accordance with a seventh aspect of the present invention, a medical image radiographing system comprises: a control apparatus for holding radiographing order information in a readable state, and transmitting the radiographing order information to an external apparatus according to a reading instruction; and a portable terminal connectable to the control apparatus through a communication network, and the portable terminal comprises: a storage for storing the radiographing order information obtained from the control apparatus; a display for displaying an addition input screen for inputting to add radiographing order information when permitted doctor identification information is inputted; an addition processing section for adding the radiographing order information inputted on the addition input screen to the radiographing order information stored in the storage; and a transmission section for transmitting the radiographing order information added to the control apparatus through the communication network.

According to the system of the seventh aspect of the present invention, when X-ray radiographing is carried out in a hospital room, in case it is necessary to add radiographing order information in the hospital room, it is possible to add radiographing order information rapidly and efficiently. Further, if the permitted doctor identification information is not inputted, the screen for adding the radiographing order information is not displayed. Consequently, it is possible to prevent the operator or the like from adding the radiographing order information without the permission of the doctor, and to prevent illegal X-ray radiographing against the Radiologist Law without the permission of the doctor in the hospital room.

Preferably, the system of the seventh aspect of the present invention further comprises an information management apparatus for generating and storing radiographing order information on the basis of radiographing reservation input, wherein the control apparatus further comprises an obtaining section for obtaining the radiographing order information by reading the radiographing order information out of the information management apparatus through the communication network.

According to the system, the medical image radiographing system comprises the information management apparatus for generating radiographing order information on the basis of radiographing reservation input, and the control apparatus reads the radiographing order information from the information management apparatus through the communication network. Consequently, it is possible to input radiographing order information by the external information management apparatus.

Preferably, in the above-described system, the control apparatus further comprises: a receiving section for receiving the radiographing order information added transmitted from the portable terminal; an addition processing section for adding the radiographing order information received to the radiographing order information held; and a transmission section for transmitting the radiographing order information added to the information management apparatus through the communication network, and the information management apparatus comprises: a receiving section for receiving the radiographing order information added transmitted from the control apparatus; and an addition processing section for adding the radiographing order information received in the radiographing order information stored.

According to the system, when receiving the added radiographing order information transmitted from the portable terminal, the control apparatus adds the received radiographing order information to the held radiographing order information, and transmits the radiographing order information to the information management apparatus through the network. When receiving the added radiographing order information transmitted from the control apparatus, the information management apparatus adds the received radiographing order information to the stored radiographing order information. Consequently, it is possible to add the radiographing order information added by the portable terminal in the hospital room to the information management apparatus easily and rapidly.

Preferably, the system of the seventh aspect of the present invention, further comprises an information management apparatus for generating radiographing order information on the basis of radiographing reservation input, wherein the portable terminal has a structure capable of reading the radiographing order information out of the information management apparatus through the communication network.

According to the system, the medical image radiographing system comprises the information management apparatus for generating radiographing order information on the basis of radiographing reservation input, and the portable terminal can read the radiographing order information out of the information management apparatus through the communication network. Consequently, it is possible that the portable terminal obtains the radiographing order information without the control apparatus.

Preferably, the system of the seventh aspect of the present invention, further comprises an X-ray radiographing apparatus which is movable and radiographs with X-ray by using a cassette on the basis of the radiographing order information, wherein the control apparatus comprises: an extracting section for extracting radiographing order information using the cassette from the radiographing order information held; and a transmission section for transmitting the radiographing order information extracted by the extracting section to the portable terminal.

According to the system, the control apparatus extracts radiographing order information for the radiographing using the cassette from the radiographing order information, and transmits the extracted radiographing order information to the portable terminal. Consequently, because only the radiographing order information for the radiographing using the cassette is transmitted to the portable terminal, it is possible to limit data to be processed by the portable terminal to information required to radiographing with X-ray in the hospital room. As a result, it is possible that the portable terminal performs the processing efficiently.

In accordance with an eighth aspect of the present invention, a portable terminal capable of being connected to a control apparatus for holding radiographing order information in a readable state and transmitting the radiographing order information to an external apparatus according to a reading instruction, through a communication network, comprises: a receiving section for receiving the radiographing order information from the control apparatus; a storage for storing the radiographing order information received; an input section for inputting doctor identification information; a display for displaying an addition input screen for inputting to add radiographing order information when the doctor identification information which is permitted is inputted; a radiographing order information addition processing section for adding the radiographing order information inputted on the addition input screen to the radiographing order information stored in the storage; and a transmission section for transmitting the radiographing order information inputted on the addition input screen to the control apparatus.

Accordingly, when radiographing with X-ray is to be carried out in the hospital room, in case it is necessary to add radiographing order information in the hospital room, it is possible to provide the portable terminal capable of adding radiographing order information rapidly and efficiently. Further, according to the portable terminal, if the permitted doctor identification information is not inputted, the screen for adding radiographing order information is not displayed. Consequently, it is possible to prevent the operator or the like from adding radiographing order information without the permission of the doctor.

In accordance with a ninth aspect of the present invention, a medical image radiographing system comprises: a control apparatus for managing a medical image and radiographing order information on medical radiographing by relating the medical image to the radiographing order information; and a portable terminal for receiving the radiographing order information from the control apparatus, the portable terminal comprises: a first communication section for communicating with the control apparatus; and a first control section for transmitting radiographing history information related to the radiographing order information to the control apparatus through the first communication section, and the control apparatus comprises: a second communication section for communicating with the portable terminal; an input section for receiving input of information; and a second control section for determining whether it is possible to communicate with the portable terminal through the second communication section or not, and receiving input of the radiographing history information through the input section when determining that it is impossible to communicate with the portable terminal.

In accordance with a tenth aspect of the present invention, a medical image management method for a medical image radiographing system comprising a control apparatus for managing a medical image and radiographing order information on medical radiographing by relating the medical image to the radiographing order information and a portable terminal for receiving the radiographing order information from the control apparatus, comprises: transmitting radiographing history information related to the radiographing order information from the portable terminal to the control apparatus; and determining whether it is possible to communicate with the portable terminal or not, and receiving input of the radiographing history information when determining that it is impossible to communicate with the portable terminal, in the control apparatus.

According to the system of the ninth aspect or the method of the tenth aspect of the present invention, when it is impossible to communicate with the portable terminal, because the control apparatus obtains the radiographing history information related to the radiographing order information by receiving the input of the radiographing history information, it is possible that the control apparatus obtain the radiographing history information certainly.

Preferably, in the system of the ninth aspect of the present invention, the second control section receives the radiographing history information from the portable terminal through the second communication section when determining that it is possible to communicate with the portable terminal.

Preferably, the method of the tenth aspect of the present invention, further comprises receiving the radiographing history information from the portable terminal when determining that it is possible to communicate with the portable terminal, in the control apparatus.

According to the system or the method, when the communication with the portable terminal is established, the control apparatus can obtain the radiographing history information certainly by receiving it from the portable terminal.

Preferably, the system of the ninth aspect of the present invention further comprises a communication terminal for mediating communication between the portable terminal and the control apparatus when the communication terminal is connected to the second communication section and the portable terminal is connected to the communication terminal, wherein the second control section determines whether it is possible to communicate with the portable terminal through the second communication section or not in a state the portable terminal is connected to the communication terminal.

Preferably, in the method of the tenth aspect of the present invention, the medical image radiographing system further comprises a communication terminal for mediating communication between the portable terminal and the control apparatus when the communication terminal is connected to the control apparatus and the portable terminal is connected to the communication terminal, and the determining whether it is possible to communicate with the portable terminal or not includes determining whether it is possible to communicate with the portable terminal or not in a state the portable terminal is connected to the communication terminal, in the control apparatus.

According to the system or the method, because the control apparatus determines whether the communication with the portable terminal is established or not in the state the portable terminal is connected to the communication terminal, it is possible that the control apparatus certainly determines whether the communication is established or not.

Preferably, in the system of the ninth aspect of the present invention, the control apparatus further comprises a display for displaying information, and the second control section displays the radiographing history information inputted.

Preferably, the method of the tenth aspect of the present invention, further comprises displaying the radiographing history information inputted in the control apparatus.

According to the system or the method, it is possible to make the operator or the like input the radiographing history information certainly with reference to the display of the inputted radiographing history information. Consequently, it is possible that the control apparatus obtains it certainly.

In accordance with an eleventh aspect of the present invention, a medical image radiographing system comprises: a control apparatus for managing a medical image and radiographing order information by relating the medical image to the radiographing order information; a portable terminal comprising an obtaining section for obtaining the radiographing order information from the control apparatus, and a transmission section for transmitting a radiographing result corresponding to the radiographing order information obtained by the obtaining section to the control apparatus; a determination section for determining relationship between the radiographing order information transmitted from the control apparatus to the portable terminal and the radiographing result corresponding to the radiographing order information by the portable terminal; and a control section for controlling transmission of the radiographing result from the portable terminal to the control apparatus on the basis of a determination result determined by the determination section.

Preferably, the system of the eleventh aspect of the present invention, further comprises at least one of the control apparatus connected through a network.

More preferably, the above-described system further comprises at least one of the portable terminal capable of communicating with the at least one of the control apparatus through the network, or at least one of the portable terminal capable of communicating with a specific control apparatus among the at least one of the control apparatus through the network.

In accordance with a twelfth aspect of the present invention, a medical image radiographing system comprises: a radiographing order information generating apparatus for generating radiographing order information; a control apparatus for obtaining the radiographing order information, relating the radiographing order information to at least one of related information of the radiographing order information and a medical image, and managing the radiographing order information and the at least one related to the radiographing order information; a portable terminal comprising an obtaining section for obtaining the radiographing order information from the control apparatus, and a transmission section for transmitting a radiographing result corresponding to the radiographing order information obtained by the obtaining section to the control apparatus; a determination section for determining relationship between the radiographing order information transmitted from the control apparatus to the portable terminal and the radiographing result corresponding to the radiographing order information by the portable terminal; and a control section for controlling transmission of the radiographing result from the portable terminal to the control apparatus on the basis of a determination result determined by the determination section.

Preferably, the system of the twelfth aspect of the present invention further comprises at least one of the control apparatus connected to the radiographing order information generating apparatus through a network.

More preferably, the above-described system further comprises at least one of the portable terminal capable of communicating with the at least one of the control apparatus through the network, or at least one of the portable terminal capable of communicating with a specific control apparatus among the at least one of the control apparatus through the network.

### BRIEF DESCRIPTION OF THE DRAWINGS

The present invention will become more fully understood from the detailed description given hereinafter and the accompanying drawing given by way of illustration only, and thus are not intended as a definition of the limits of the present invention, and wherein:
FIG. 1 is a view showing a system structure of a medical image radiographing system 100 according to a first embodiment to which the present invention is applied;
FIG. 2 is a block diagram showing a main structure of a portable terminal 110 shown in FIG. 1;
FIG. 3 is a table showing a data structure of a radiographing order information file 1161 stored in a storage unit 116 shown in FIG. 2;
FIG. 4 is a block diagram showing a main structure of a control apparatus 120 shown in FIG. 1;
FIG. 5A is a flowchart showing radiographing preparation processing performed by a CPU 121 of the control apparatus 120, and FIG. 5B is a flowchart showing radiographing preparation processing performed by a CPU 111 of the portable terminal 110;
FIG. 6 is a view showing an example of a menu screen 1231 displayed on a display unit 123 of the control apparatus 120;
FIG. 7 is a view showing an example of a portable list screen 1232 displayed on the display unit 123 of the control apparatus 120;
FIG. 8A is a view showing an example of an input screen 1233 for inputting patient information displayed on the display unit 123 of the control apparatus 120, and FIG. 8B is a view showing an example of an input screen 1234 for inputting radiographing information displayed on the display unit 123 of the control appartus 120;
FIG. 9 is a flowchart showing radiographing start processing performed by the CPU 111 of the portable terminal 110;
FIG. 10A is a view showing an example of a patient list screen 1231 displayed on a display unit 113 of the portable terminal 110, and FIG. 10B is a view showing an example of a display screen 1232 displayed on the display unit 113 of the portable terminal 110;
FIGS. 11A and 11B are flowcharts showing radiographing termination processing performed by the CPU 121 of the control apparatus 120;
FIG. 12 is a flowchart showing radiographing termination processing performed by the CPU 111 of the portable terminal 110;
FIG. 13A is a view showing an example of a portable list screen 1235 displayed on the display unit 123 of the control apparatus 120, and FIG. 13B is a view showing an example of a portable list screen 1236 displayed on the display unit 123 of the control apparatus 120;
FIG. 14 is a view showing an example of a portable processing screen 1237 displayed on the display unit 123 of the control apparatus 120;
FIG. 15 is view showing a system structure of a medical image radiographing system 500 according to another embodiment to which the present invention is applied;
FIG. 16 is a view showing a system structure of a medical image radiographing system 200 according to a second embodiment to which the present invention is applied;
FIG. 17 is a flowchart showing radiographing start processing performed by a CPU 211 of a portable terminal 210;
FIGS. 18A and 18B are flowcharts showing radiographing termination processing performed by the CPU 211 of the portable terminal 210;
FIGS. 19A and 19B are flowcharts showing radiographing termination processing performed by a CPU 221 of a control apparatus 220;
FIG. 20A is a view showing an example of a display screen 2133 of a confirmation message displayed on a display unit 213 of the portable terminal 210, and FIG. 20B is a view showing an example of a display screen 2134 of the confirmation message displayed on the display unit 213 of the portable terminal 210;
FIG. 21 is a view conceptually showing a system structure of a medical image radiographing system 300 according to a third embodiment to which the present invention is applied;
FIG. 22 is a block diagram showing a functional structure of a control apparatus 320 shown in FIG. 21;
FIG. 23 is a table showing a data structure of a radiographing order information file 3261 stored in a storage unit 326 shown in FIG. 22;
FIG. 24 is a block diagram showing a functional structure of a portable terminal 310 shown in FIG. 21;
FIG. 25 is a flowchart showing raiographing order information transmission processing performed by the control apparatus 320 and the portable terminal 310 shown in FIG. 21;
FIG. 26 is a flowchart showing radiographing order information addition processing A performed by a CPU 311 of the portable terminal 310 shown in FIG. 24;
FIG. 27A is a view showing an example of a radiographing order information addition screen 3131, and FIG. 27B is a view showing an example of a radiographing condition addition screen 3132;
FIG. 28 is a flowchart showing radiographing order information addition processing B for adding radiographing order information added by the portable terminal 310 shown in FIG. 21 to the control apparatus 320 and an information management apparatus 360;
FIG. 29 is a view showing a system structure of a medical image radiographing system 400 according to a fourth embodiment to which the present invention is applied;
FIG. 30 is a table showing a data structure of a radiographing order information file 4161 stored in a storage unit 416 of a portable terminal 410 shown in FIG. 29;
FIG. 31 is a block diagram showing a main structure of a control apparatus 420 shown in FIG. 29;
FIG. 32 is a table showing a data structure of a radiographing history management file 4262 stored in a storage unit 426 shown in FIG. 31;
FIG. 33 is a flowchart showing radiographing start processing performed by the portable terminal 410;
FIG. 34A is a view showing an example of a patient list screen 4131 displayed on a display unit 413 of the portable terminal 410, and FIG. 34B is a view showing an example of a display screen 4132 displayed on the display unit 413 of the portable terminal 410;
FIG. 35 is a flowchart showing radiographing termination processing performed by the control appartus 420;
FIG. 36 is a view showing an example of a time-out screen 4231 displayed on a display unit 423 of the control apparatus 420; and
FIG. 37 is a view showing an example of a portable processing screen 4232 displayed on the display unit 423 of the control apparatus 420.

### EMBODIMENTS OF THE INVENTION

### [First Embodiment]

Hereinafter, the first embodiment of the present invention will be explained in detail, with reference to figures. A definition of the present invention is not limited to the figures. As an example of a characteristic embodiment of the present invention, a medical image radiographing system 100 for radiographing in a ward with a portable terminal which is portable and a radiographing apparatus which is portable will be explained.

First, a structure of the first embodiment will be explained.

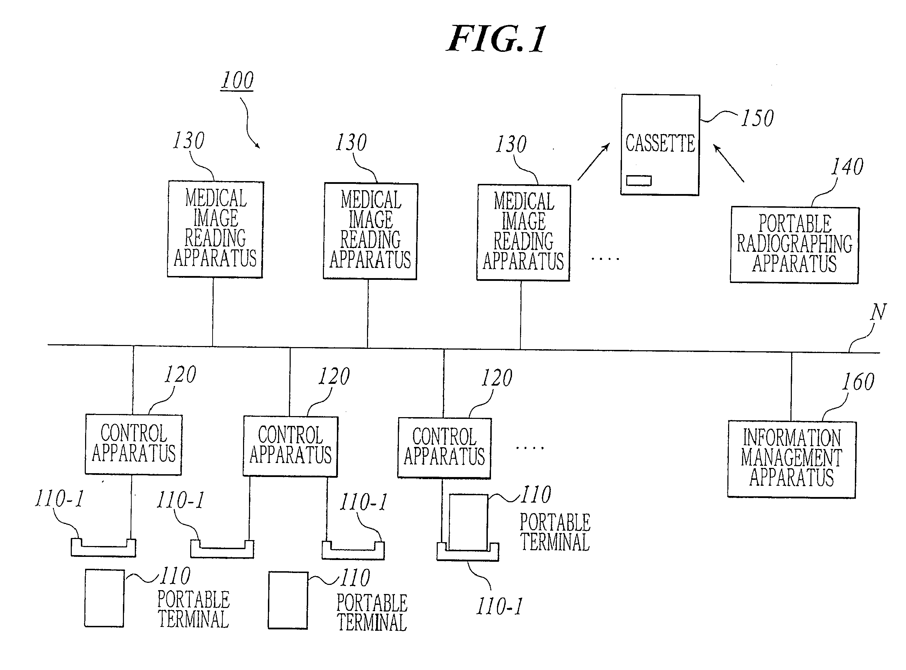

FIG. 1 is a conceptual view showing a system structure of the medical image radiographing system 100 of the present invention. As shown in FIG. 1, the medical image radiographing system 100 comprises a portable terminal 110, a communication terminal 110-1, a control apparatus 120, a medical image reading apparatus 130, a portable radiographing apparatus 140, a cassette 150, an information management apparatus 160 or the like. The control apparatus 120, the medical image reading apparatus 130 and the information management apparatus 160 are connected to each other through a network N. The portable terminal 110 and the control apparatus 120 can be connected to each other through the communication terminal 110-1. At least one portable terminal 110, at least one communication terminal 110-1, at least one control apparatus 120 and at least one medical image reading apparatus 130 are provided and are not limited to specific numbers.

The portable terminal 110 is a portable information terminal apparatus which an operator or the like for operating the portable radiographing apparatus 140 takes with him. The portable terminal 110 communicates with the control apparatus 120 through the communication terminal 110-1 as follows. The portable terminal 110 obtains radiographing order information from the control apparatus 120 and displays it thereon. In case the radiographing order information includes radiographing orders of a plurality of patients, the portable terminal 110 searches radiographing order information corresponding to a desired patient ID from the obtained radiographing order information and displays the searched radiographing order information thereon.

The communication terminal 110-1 is connected to the control apparatus 120 through a cable or the like, and controls transmission of data between the portable terminal 110 connected to the communication terminal 110-1 and the control apparatus 120. For example, the communication terminal 110-1 controls transmission of radiographing order information from the control apparatus 120 to the portable terminal 110, and transmission of radiographing order information and a cassette ID from the portable terminal 110 to the control apparatus 120.

The control apparatus 120 receives radiographing order information from the information management apparatus 160, and transmits the received radiographing order information to the portable terminal 110 through the communication terminal 110-1. When radiographing a medical image, the control apparatus 120 receives identification information of the cassette 150 related to the radiographing order information from the portable terminal 110, relates the medical image to the radiographing order information on the basis of the identification information of the cassette 150, and manages the medical image. The control apparatus 120 relates the identification information of the cassette 150 to the radiographing order information, and transmits them to the information management apparatus 160.

The medical image reading apparatus 130 is a medical image reading apparatus for reading the medical image recorded in the cassette 150. The medical image reading apparatus 130 irradiates excitation light to a photostimulable phosphor substance sheet of the cassette 150, converts stimulated light emitted from the sheet photoelectrically, converts an analog image signal obtained to a digital signal, and obtains a medical image. The medical image reading apparatus 130 reads a cassette ID accompanying the cassette 150, relates the medical image to the cassette ID, and transmits them to the control apparatus 120.

The portable radiographing apparatus 140 is a medical image radiographing apparatus which is movable. The portable radiographing apparatus 140 radiographs a patient in a ward, and records the medical image in the cassette 150 which is attachable to and detachable from the apparatus body.

The cassette 150 incorporates the photostimulable phosphor substance sheet in which a part of radiation energy is accumulated, and accumulates a part of radiation energy which is irradiated from a radiation source and transmitted through an object placed between the radiation source and the cassette 150 so as to be examined in the photostimulable phosphor substance sheet. A barcode or the like for recording the cassette ID which is identification information of the cassette 150 is attached on a surface of the cassette 150.

The information management apparatus 160 is a terminal for managing radiographing order information instructed by a doctor collectively. The information management apparatus 160 extracts radiographing order information according to an instruction requested by the control apparatus 120, and transmits the extracted radiographing order information to the control apparatus 120. The information management apparatus 160 may adopt an apparatus (which is not shown in figures) for accepting reservation of radiographing order information, or an information management system such as the HIS (Hospital Information system), the RIS (Radiology Information system) or the like, as another information management apparatus.

The network N can adopt various line states such as a LAN (Local Area Network), a WAN (Wide Area Network), the Internet or the like. A wireless LAN or the like which has a bad influence on a medical apparatus in a medical institution such as a hospital or the like, is excluded from the network N. However, if it is permitted, the network N may use radio communication or infrared rays communication. When radiographing order information is transmitted through the network N, because the radiographing order information includes important patient information, it is preferable to encode the radiographing order information.

Next, each apparatus which is one of main components in the present invention will be explained in detail.

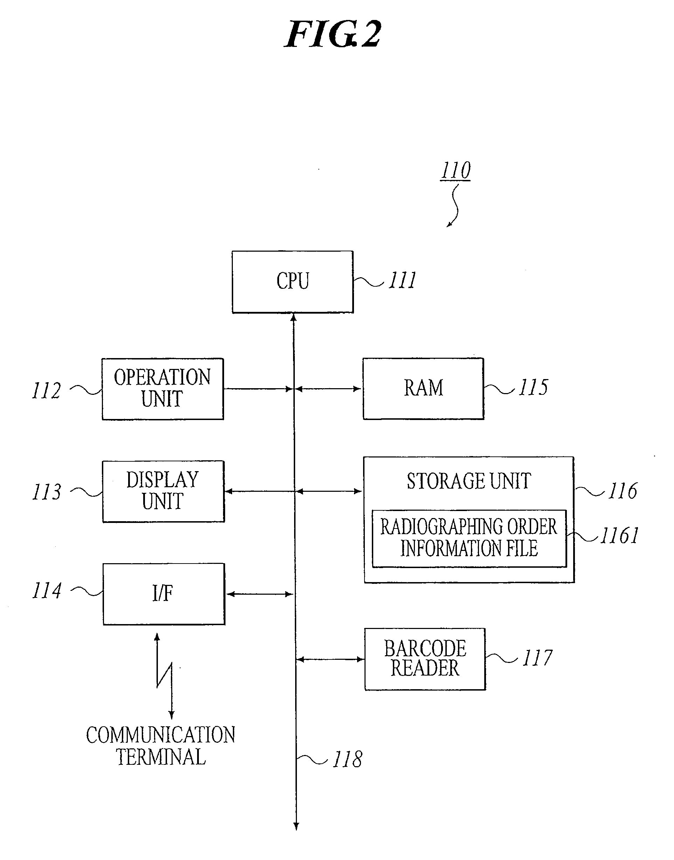

FIG. 2 is a block diagram showing a functional structure of the portable terminal 110. As shown in FIG. 2, the portable terminal 110 comprises a CPU 111, an operation unit 112, a display unit 113, an I/F 114 which is the communication section, a RAM 115, a storage unit 116 which is the storage, a barcode reader 117 which is the obtaining section and so on, which are connected to each other through a bus 118.

The CPU (Central Processing Unit) 111 expands a program specified from a system program and various application programs stored in the storage unit 116 in the RAM 115, and controls each unit of the portable terminal 110 according to the program in a concentrated way, as the control section.

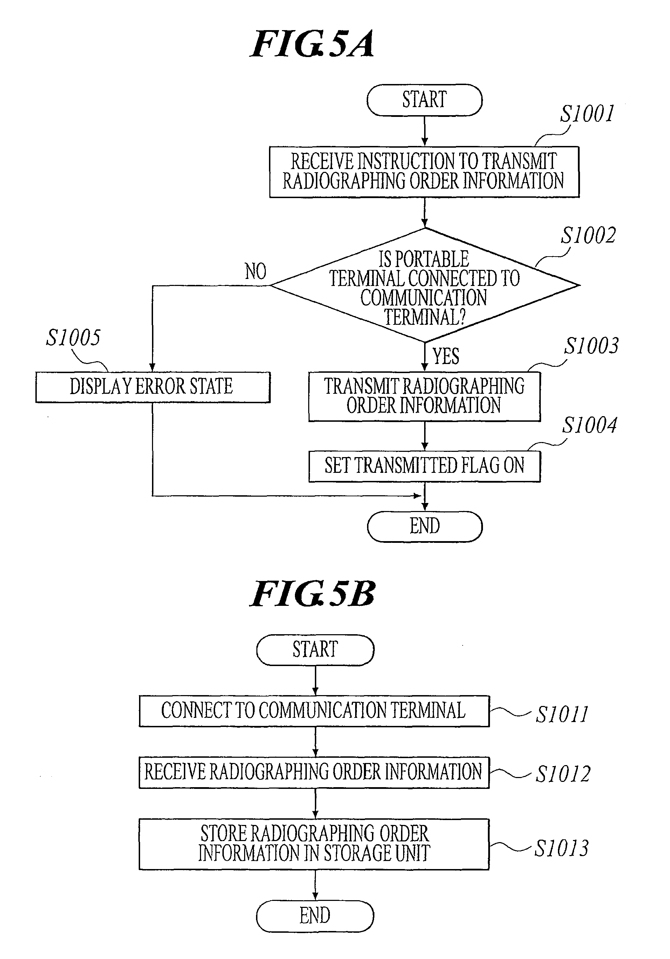

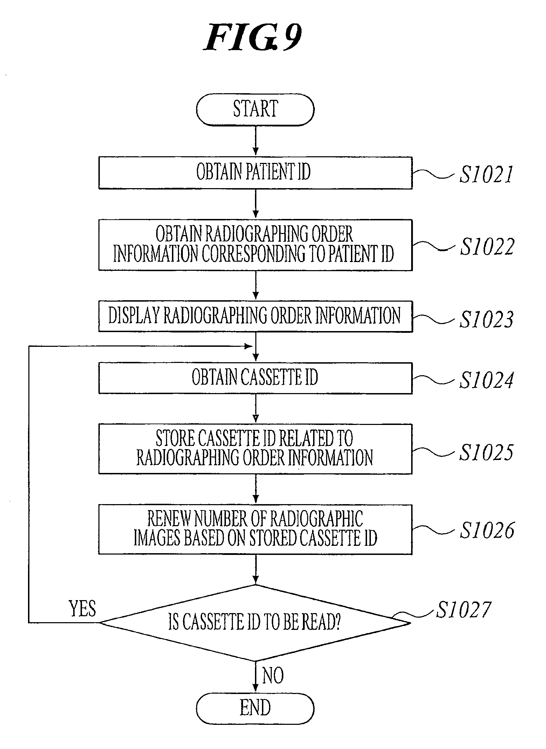

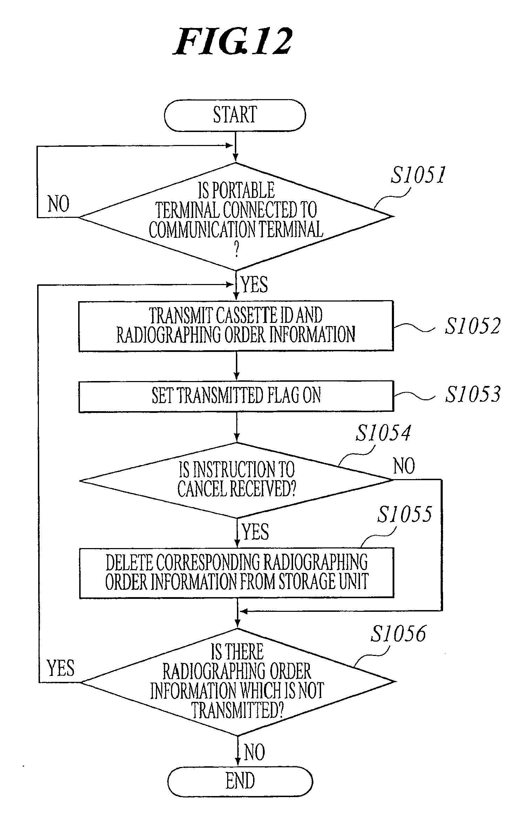

More specifically, the CPU 111 reads a radiographing preparation processing program, a radiographing start processing program and a radiographing termination processing program out of the storage unit 116, and executes radiographing preparation processing (FIG. 5B), radiographing start processing (FIG. 9) and radiographing termination processing (FIG. 12) according the programs, as follows. The detail of each processing will be explained as follows.

The operation unit 112 comprises a cursor key, numeric keys, various function keys or the like, and outputs a push signal corresponding to a key pushed by a radiographer to the CPU 111. Further, the operation unit 112 comprises a jog dial key capable of being operated with a finger of a hand holding the portable terminal 110, and outputs an instruction signal for scrolling (moving) information displayed on the display unit 113 according to operation of the joy dial key to the CPU 111. When the jog dial key is pushed, the operation unit 112 outputs a push key corresponding to information displayed on the display unit 113 to the CPU 111. The operation unit 112 may comprise a pointing device such as a touch panel or the like, another input device or the like, as necessary. Further, the operation unit 112 may comprise a touch key or the like instead of the jog dial key.

The display unit 113 is the display such as a LCD (Liquid Crystal Display), an EL display (Electro Luminance Display), or the like. The display unit 113 displays various information such as radiographing order information, an obtained patient ID, a cassette ID or the like, on the basis of a display instruction outputted from the CPU 111.

The I/F 114 is an interface for connecting the portable terminal 110 to the communication terminal 110-1, and outputs a detection signal to the CPU 111 when the portable terminal 110 is connected to the communication terminal 110-1. The I/F 114 adjusts a transmission speed of data, converts a data format, and mediates data between the portable terminal 110 and the control apparatus 120, through the communication terminal 110-1.

For example, the I/F 114 receives radiographing order information from the control apparatus 120, and transmits the cassette ID related to the radiographing order information to the control apparatus 120 after radiographing the patient. When a portable telephone such as a PHS or the like is connected to the I/F 114 as necessary, the I/F 114 may establish wireless communication and transmit data. For example, when a doctor instructs the operator to add radiographing order information, the portable telephone such as a PHS or the like may display a confirmation message for confirming that the radiographing order information is to be added to the doctor.

The RAM (Random Access Memory) 115 has a work memody area for storing the application program specified as described above, inputted instructions, inputted data, processing results or the like.

The storage unit 116 comprises a storage medium in which programs, data or the like are stored previously. The storage medium stores a system program, various application programs corresponding to the system program, data processed according to various processing programs, or the like. The storage medium consists of a magnetic storage medium, an optical storage medium or a semiconductor memory. The storage medium may be fixed at or connected so as to be attachable to and detachable from the storage unit 116.

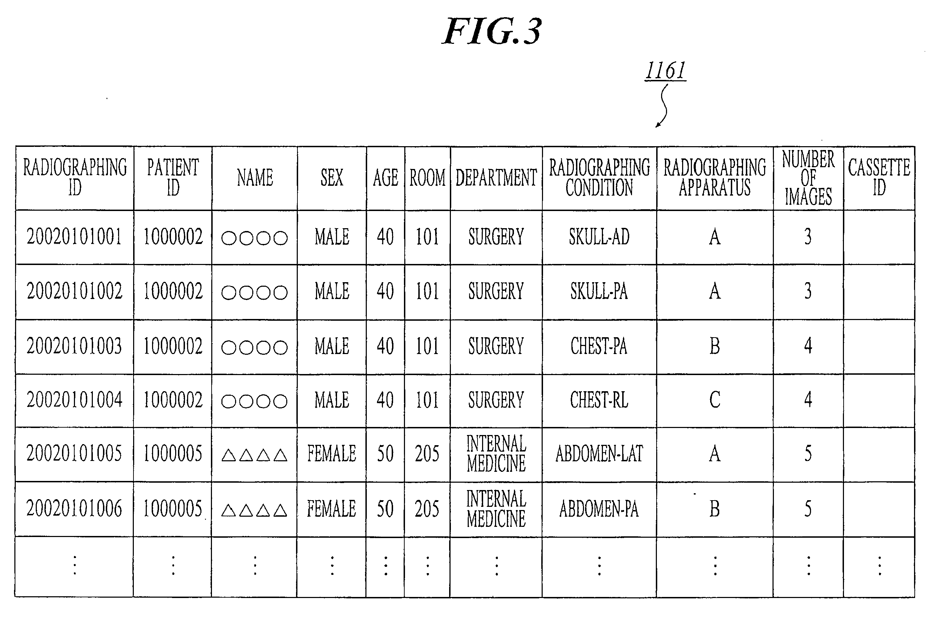

Further, the storage unit 116 stores a radiographing order information file 1161 for storing radiographing order information received from the control apparatus 120. The radiographing order information file 1161 will be explained with reference to FIG. 3.

FIG. 3 is a table showing an example of a data structure of the radiographing order information file 1161. As shown in FIG. 3, the radiographing order information file 1161 has items for storing a radiographing ID, a patient ID, a name, a sex, an age, a room, a department, a radiographing condition, a radiographing apparatus, a number of images, and identification information of the cassette 150 (hereinafter, it will be a cassette ID.), respectively. The radiographing order information file 1161 stores data corresponding to each item for every radiographing order information.

An identification code (for example, 20020101001, 20020101002, 20020101003, ...) assigned for every radiographing to specify radiographing is stored in the item for the radiographing ID. An identification code (for example, 10000002, 1000005, ...) assigned for every radiographing to specify a patient to be radiographed is stored in the item for the patient ID. Character information indicating the name of the patient as the object to be radiographed is stored in the item for the name. Character information indicating the sex of the patient as the object to be radiographed is stored in the item for the sex. Numeral information indicating the age of the patient as the object to be radiographed is stored in the item for the age. Character information indicating the room as a radiographing location is stored in the item for the room.

Character information indicating the department which is requested to radiograph the patient is stored in the item for the department. Information (for example, skull-AP, skull-PA, chest-PA, ...) indicating the radiographic part is stored in the item for the radiographing condition. Character information (for example, A, B, C, ...) indicating the apparatus for radigraphing the patient is stored in the item for the radiographing apparatus. Numeral information (for example, 3, 4, 5, ...) indicating the number of medical images to be radiographed is stored in the item for the number of images.

The cassette ID (for example, information read out of the barcode attached to the cassette 150 or the like) assigned for every radiographing to specify the cassette used for radiographing is stored in the item for the cassette ID. When preparing for radiographing, data of the cassette ID are not stored in the item for the cassette ID. When radiographing the patient in the bed side, the cassette ID read by the barcode reader 117 incorporated in the portable terminal 110 is stored in the item for the cassette ID.

The radiographing order information may include various patient information, for example, a name of a doctor in charge, warning information for warning infection or the like, drug allergy, pregnancy, additional medical history, special care necessity such as a wheelchair, a stretcher or the like, clinical diagnosis, privileged items or the like, besides the patient ID, the name, the sex and the age, as the patient information. Further, the radiographing order information may include various radiographing information, for example, a radiographing method (simple radiography, contrast radiography or the like), the appointed radiographing date or the like, besides the radiographing condition, the radiographing apparatus and the number of images, as the radiographing information,

The barcode reader 117 is an example of the obtaining section for obtaining the caste ID, and comprises a scanner which is an optical reading apparatus. The barcode reader 117 reads the barcode with the scanner, decodes the read barcode according to a predetermined protocol, and obtains information indicated by the barcode. The predetermined protocol is JAN code, UPC code, CODE 39, CDE93, CODE 128, NW-7, INDUSTRIAL 2of5, ITF physical distribution code, or the like.

More specifically, the barcode reader 117 reads the barcode attached to the cassette 150 recording the medical image therein when radiographing the patient, and obtains the cassette ID. Further, the barcode reader 117 reads a barcode attached to the bed side of the patient or a part of a body of the patient, identifies the patient ID, and relates the cassette ID corresponding to the radiographing order information of the patient.

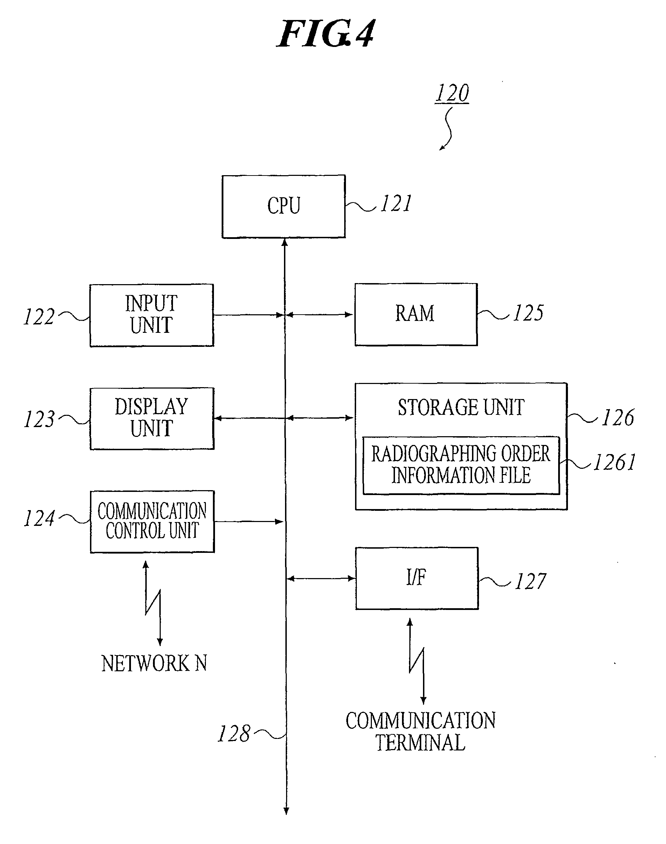

FIG. 4 is a block diagram showing a functional structure of the control apparatus 120. As shown in FIG. 4, the control apparatus 120 comprises a CPU 121, an input unit 122, a display unit 123, a communication control unit 124, a RAM 125, a storage unit 126, an I/F 127 and so on, which are connected to each other through a bus 128.

The CPU 121 reads a system program or various control programs stored in the storage unit 126, expands the program in the RAM 125, and controls each unit according to the control program in a concentrated way, as the control section. Further, the CPU 121 performs various processing according to the program expanded in the RAM 125, stores processing results in the RAM 125 temporarily, and displays the processing results on the display 123.

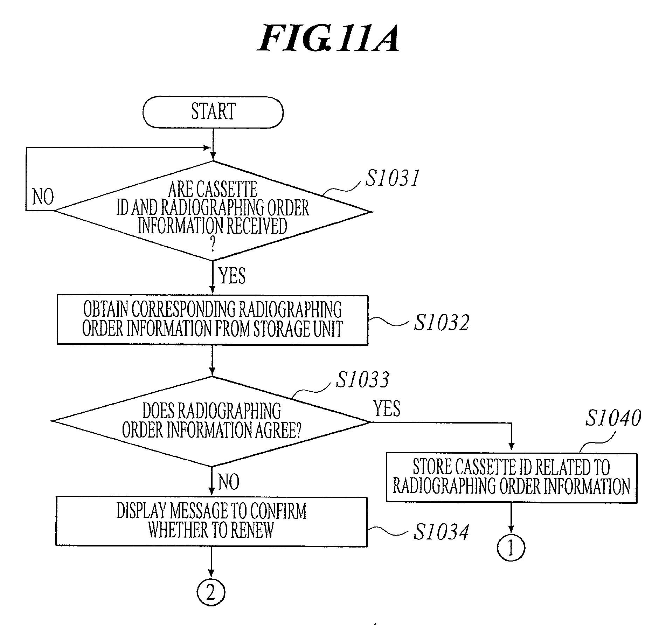

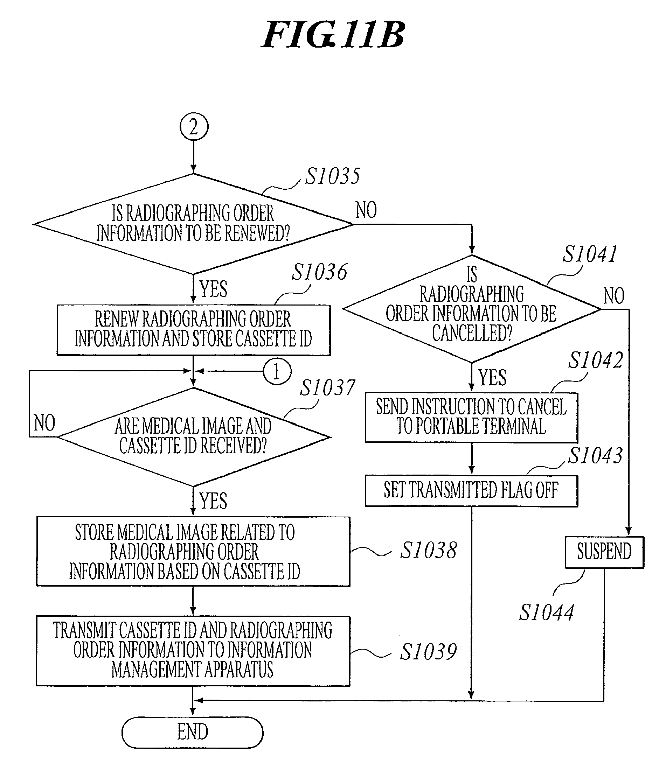

More specifically, the CPU 121 reads a radiographing preparation processing program and a radiographing termination processing program out of the storage unit 126, and executes radiographing preparation processing (FIG. 5A) and radiographing termination processing (FIGS. 11A and 11B) according the programs, as follows.

The input unit 122 comprises a keyboard comprising a cursor key, numeric input keys, various function keys or the like, and outputs a push signal corresponding to a key pushed in the keyboard to the CPU 121. Further, the input unit 122 may comprise a pointing device such as a mouse, a so-called touch panel or the like, another input device or the like, as necessary, the touch panel for outputting position information inputted by touching a transparent sheet panel covering a display screen of the display unit 123 with a finger or an exclusive stylus pen to the CPU 121, as an input signal.

The display unit 123 comprises a LCD (Liquid Crystal Display), a CRT (Cathode Ray Tube), an EL display (Electro Luminance Display) or the like. The display unit 123 displays instructions, data or the like inputted through the input unit 122 according to an instruction by a display signal outputted from the CPU 121.

The communication control unit 124 comprises a modem, a LAN adapter, a router, a TA (Terminal Adapter) or the like. The communication control unit 124 controls communication with each apparatus connected to the network N through a communication line such as a private line, ISDN network or the like.

The RAM 125 forms a storage area for storing a system program or a control program read out of the storage unit 126 and executable by the CPU 121, inputted or outputted data, parameters or the like, temporarily.

The storage unit 126 comprises a HDD (Hard Disc Drive), a non-volatile semiconductor memory or the like. The storage unit 126 stores a system program to be executed by the CPU 121, various processing programs corresponding to the system program, processing results or the like. The storage unit 126 comprises a storage medium (which is not shown in figures.) in which programs or data are stored previously. The storage medium is a non-volatile semiconductor memory consisting of a magnetic storage medium, an optical storage medium or a semiconductor memory. The storage medium may be fixed at or connected so as to be attachable to and detachable from the storage unit 126. The storage medium stores various programs in a format of readable program codes, and the CPU 121 performs operation according to the program codes in order.

Further, the storage unit 126 stores a radiographing order information file 1261 for storing radiographing order information received from the information management apparatus 160. The radiographing order information file 1261 has substantially the same structure as one of the above-described radiographing order information file 1161 (shown in FIG. 3), and it is omitted to show it in figures and explain it in detail. Further, the storage unit 126 stores the medical image received from the medical image reading apparatus 140 related to the radiographing order information.

The I/F 127 is an interface for connecting the control apparatus 120 to the communication terminal 110-1, and outputs a detection signal to the CPU 121 when detecting that the portable terminal 110 is connected to the communication terminal 110-1. The I/F 127 adjusts a transmission speed of data, converts a data format, and mediates data between the control apparatus 120 and the portable terminal 110 through the communication terminal 110-1. For example, the I/F 127 transmits radiographing order information to the portable terminal 110 before starting radiographing, and receives radiographing order information related to the cassette ID from the portable terminal 110 after radiographing.

Next, the operation according to the first embodiment will be explained.

The program realizable of each function described in the following flowcharts is stored in the storage unit 116 of the portable terminal 110 or the storage unit 126 of the control apparatus 120 in a format of program codes readable by a computer. Therefore, the CPU 111 of the portable terminal 110 or the CPU 121 of the control apparatus 120 performs the operation according to the program codes in order.

First, radiographing preparation processing for transmitting radiographing order information from the control apparatus 120 to the portable terminal 110 in a radiographing room will be explained as preparation processing for radiographing.

FIG. 5A is a flowchart showing radiographing preparation processing performed by the CPU 121 of the control apparatus 120. As shown in FIG. 5A, when the CPU 121 receives an instruction to transmit radiographing order information through the input unit 1220 by the operator (Step S1001), the CPU 121 controls the I/F 127 and determines whether the portable terminal 110 is connected to the communication terminal 110-1 or not (Step S1002). When the portable terminal 110 is not connected to the communication terminal 110-1 (Step S1002; NO), the CPU 121 displays an error state on the display unit 123 (Step S1005), and terminates the radiographing preparation processing.

On the other hand, when the portable terminal 110 is connected to the communication terminal 110-1 (Step S1002; YES), the CPU 121 obtains radiographing order information out of the radiographing order information file 1261, and transmits the radiographing order information to the portable terminal 110 (Step S1003). Then, the CPU 121 sets a transmitted flag for the transmitted radiographing order information ON (Step S1004), and terminates the radiographing preparation processing.

FIG. 5B is a flowchart showing radiographing preparation processing performed by the CPU 111 of the portable terminal 110. As shown in FIG. 5B, the portable terminal 110 is connected to the communication terminal 110-1 in order to obtain radiographing order information (Step S1011).

Then, the CPU 111 controls the I/F 114, and receives rediographing order information transmitted from the control apparatus 120 (Step S1012). The CPU 111 stores the received rediographing order information in the radiographing order information file 1161 of the storage unit 116 (Step S1013), and terminates the radiographing preparation processing.

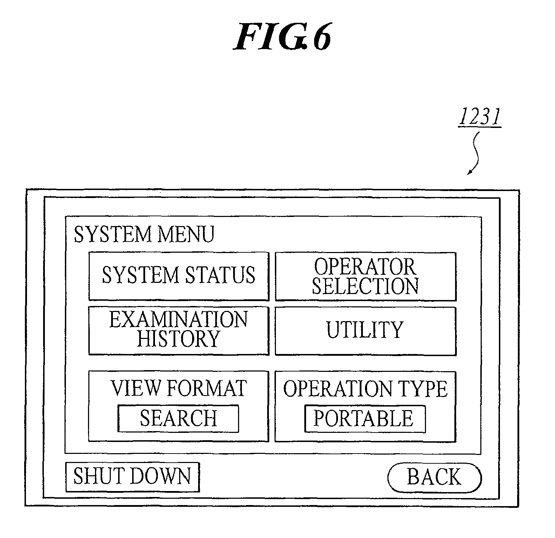

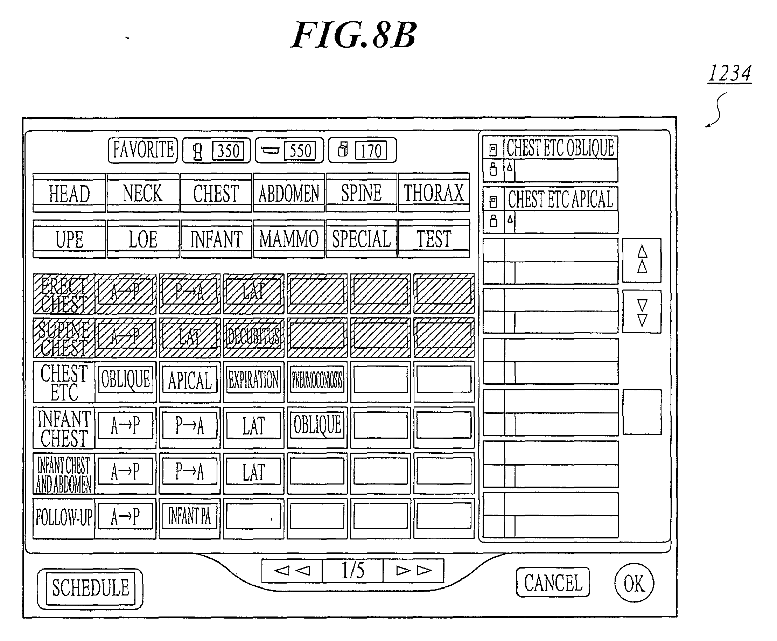

Screens displayed on the display unit 123 of the control apparatus 120 according to the above-described radiographing preparation processing, will be explained with reference to FIGS. 6 and 8B.

FIG. 6 is a view showing a menu screen 1231 for selecting desired processing. As shown in FIG. 6, as a system menu, instruction buttons for instructing menus of "system state", "operator selection", "examination history", "utility", "view format", and "operation type" are provided on the menu screen 1231. Further, input areas are provided in the "view format" and "operation type". For example, when "portable" is inputted in the input area of "operation type", and the instruction button of "operation type" is pushed, a portable mode screen for radiographing with the portable radiographing apparatus 140 is displayed. When "normal" is inputted in the input area of "operation type", a normal mode screen for radiographing in the radiographing room is displayed.

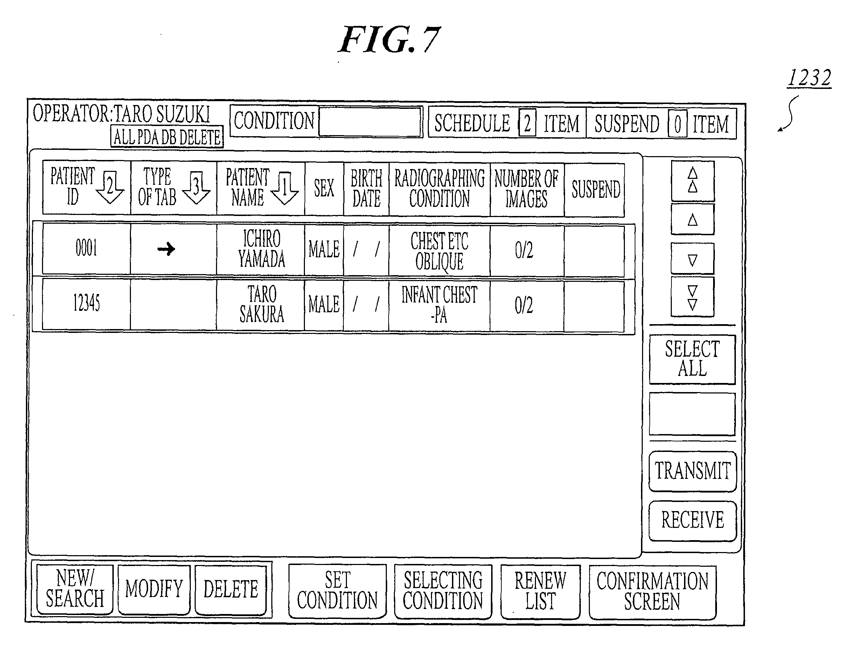

FIG. 7 is a view showing a portable list screen 1232 on which radiographing order information is listed and displayed in the portable mode. As shown in FIG. 7, an area for displaying radiographing order information and an area for displaying instruction buttons for inputting various instructions are provided on the portable list screen 1232. Items for displaying the patient ID, a type of tab, the name, the sex, the birth date, the radiographing condition, the number of radiographic images and suspend included in the radiographing order information are provided in the area for displaying the radiographing order information, and corresponding data are displayed on each item.

Instruction buttons on which text data are displayed, such as "transmit", "receive", "new/search", "modify", "delete", ... or "confirmation screen", are provided, besides selection keys in the area for displaying the instruction buttons for inputting various instructions at a right end section and a lower end section of the portable list screen 1232. When each instruction button is operated through the input unit 122, the instruction corresponding to the instruction button is inputted. For example, when radiographing order information displayed on the portable list screen 1232 is selected with the selection key, and the "transmit" button is operated, the selected radiographing order information is transmitted to the portable terminal 110. Herein, when the radiographing order information is transmitted to the portable terminal 110, the transmitted flag for the radiographing order information is set ON, and a transmitted check flag "→" is displayed on the item for "type of tab". When the instruction button of "new/search" is operated, input screens 1233 and 1234 for registering radiographing order information newly are displayed.

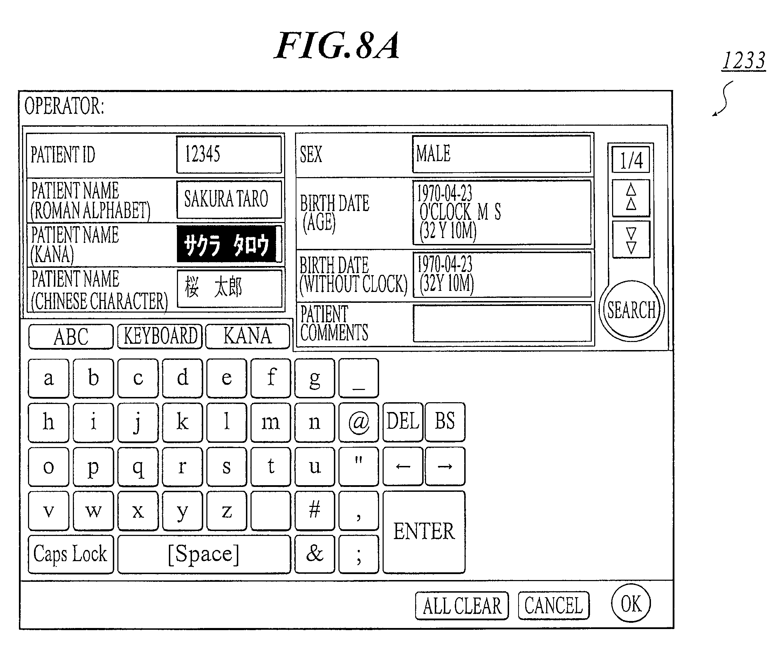

FIG. 8A and 8B are views showing input screens 1233 and 1234 displayed when the "new/search" instruction buttons are operated in the portable list screen 1232.

FIG. 8A is a view showing an input screen 1233 for inputting patient information of the radiographing order information newly. As shown in FIG. 8A, an area for inputting patient information and an area on which character keys for inputting characters are displayed are provided on the input screen 1233 for the patient information.

Items for inputting the patient ID, the name (roman alphabet, kana, Chinese cahracter), the sex, the birth date (age, without clock) and patient comments are provided in the area for inputting patient information displayed on an upper section of the input screen 1233. Data inputted according to operation through the input unit 122 are displayed on the corresponding item. Further, keys corresponding to the displayed character keys are inputted through a mouse or a touch panel of the input unit 122 in the area for displaying the character keys displayed on a lower section of the input screen 1233. The key may be inputted through a keyboard of the input unit 122.

FIG. 8B is a view showing an input screen 1234 for inputting radiographing information of the radiographing order information newly. As shown in FIG. 8B, an area for displaying instruction buttons for instructing a radiographic part as the radiographing condition, an area for displaying instruction buttons for instructing a radiographing direction of the radiographic part, and an area for displaying the radiographic part and the radiographing direction inputted are provided on the input screen 1234 for the radiographing information.

Instruction buttons on which text data are displayed, such as "head", "neck", ... or "TEST" are provided in the area for displaying the instruction buttons for the radiographic part displayed on a left upper section of the input screen 1234. When each instruction button is instructed through the input unit 122, a corresponding radiographic part is selected. Further, the area for displaying the instruction buttons for the radiographing direction displayed on a left middle section of the input screen 1234, is displayed with a mesh. Therefore, in the portable mode, the area displayed with a mesh cannot be selected. That is, the radiographic parts displayed in the area shows that they cannot be radiographed by the portable radiographing apparatus 140. A method for displaying the area which cannot be selected is not only to display it with a mesh, but also not to display is or to display it inactively. Further, in case the instruction button which cannot be selected is instructed, warning may be outputted as sounds or images.

Instruction buttons on which text data are displayed, such as "PA", ... or "pneumoconiosis" as the radiographing direction for the radiographic part "chest etc", are provided in the area for displaying the instruction buttons for the radiographing direction displayed on a left lower section of the input screen 1234. When each instruction button is instructed through the input unit 122, a corresponding radiographing direction is selected. Further, the radiographic part and the radiographing direction selected by instructing the above-described instruction buttons, for example, as "chest etc oblique" are displayed in the area for displaying the radiographic part and the rardiographing direction displayed on a right side of the input screen 1233.

Next, radiographing start processing for relating the patient ID and the cassette ID to radiographing order information performed by the portable terminal 110 in a room for radiographing before starting radiographing will be explained.

FIG. 9 is a flowchart showing radiographing start processing performed by the CPU 111 of the portable terminal 110. As shown in FIG. 9, the CPU 111 controls the barcode reader 117, reads the barcode attached to the bed side of the patient or a part of the body of the patient, and obtains the patient ID (Step S1021). Then, the CPU 111 obtains radiographing order information corresponding to the read patient ID from the radiographing order information file 1161 (Step S1022).

Then, the CPU 111 displays the obtained radiographing order information on the display unit 113 (Step S1023), controls the barcode reader 117, reads the barcode attached to the cassette 150 used for radiographing, and obtains the cassette ID (Step S1024). Then, the CPU 111 relates the obtained cassette ID to the radiographing order information and stores them in the radiographing order information file 1161 (Step S1025).

Thereafter, the CPU 111 renews and stores the number of radiographic images on the basis of the cassette ID related to the radiographing order information and stored in the radiographing order information file 1161 (Step S 1026). That is, the CPU 111 increments the number of radiographic images of the corresponding radiographing order information for every cassette ID read by 1 (+1), and renews the number of radiographic images. Then, the CPU 111 determines whether the cassette ID is to be read or not (Step S1027). When the cassette ID is to be read continuously (Step S1027; YES), the CPU 111 returns to Step S 1024, and performs the above-described processing continuously. On the other hand, when the cassette ID is not to be read (Step S1027; NO), the CPU 111 terminates the radiographing start processing. When the same cassette ID is inputted by mistake, the CPU 111 detects it and warns the operator or the like of it.

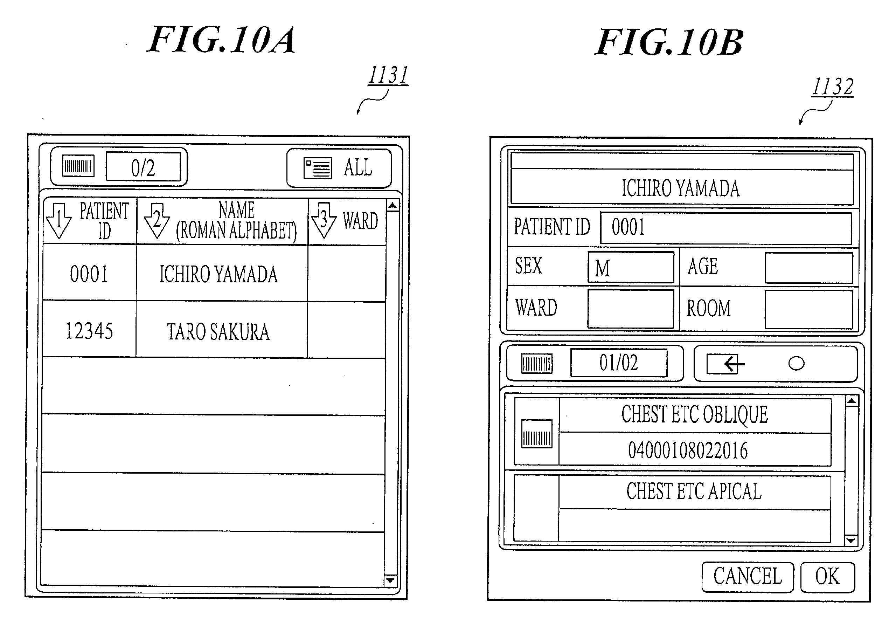

Screens displayed on the display unit 113 of the portable terminal 110 according to the above-described radiographing start processing will be explained with reference to FIGS. 10A and 10B.

FIG. 10A is a view showing a patient list screen 1131 for displaying a list of patients registered in the portable terminal 110. As shown in FIG. 10A, an area for showing patient information is provided on the patient list screen 1131. Items for displaying the patient ID, the name and the ward are provided in the area. More specifically, numeral data "0001" are displayed in the item of the patient ID, and text data "Ichiro Yamada" are displayed in the item for the name.

FIG. 10B is a view showing a display screen 1132 for displaying radiographing order information of the patient corresponding to the patient ID read by the barcode reader 117 of the potable terminal 110. As shown in FIG. 10B, an area for displaying patient information and an area for displaying radiographing information are provided on the display screen 1132. Items for displaying the name, the patient ID, the sex, the age, the ward and the room are provided in the area for displaying patient information, and corresponding data are displayed on each item.

Further, items for displaying the radiographing condition and the cassette ID are provided in the area for displaying radiographing information, and data obtained by the barcode reader 117 of the portable terminal 110 are displayed in the item for the cassette ID at the same time the barcode is read by the barcode reader 117. More specifically, text data "chest etc oblique" are displayed in the item for the radiographing condition, and numeral data "04000108022016" are displayed in the item for the cassette ID.

Next, radiographing termination processing for transmitting radiographing order information and the cassette ID from the portable terminal 110 to the control apparatus 120 in the room after radiographing will be explained, as characteristic processing of the present invention.

FIGS. 11A and 11B are flowcharts showing radiographing termination processing performed by the CPU 121 of the control apparatus 120. As shown in FIGS. 11A and 11B, when the CPU 121 receives the cassette ID related to radiographing order information from the portable terminal 110 through the communication terminal 110-1 (Step S1031; YES), the CPU 121 obtains radiographing order information corresponding to the received radiographing order information from the radiographing order information file 1261 (Step S1032). Then, the CPU 121 compares the radiographing order information received from the portable terminal 110 with the radiographing order information obtained from the radiographing order information 1261, and determines whether the radiographing order information agrees with each other or not (Step S1033).

Herein, the case the radiographing order information does not agree means the case the number of radiographic images stored in the received radiographing order information is different from the number of radiographic images stored in the radiographing order information obtained from the radiographing order information file 1261, and in the case, it is determined that the radiographing order information does not agree. Further, in the case the number of radiographic images stored in the received radiographing order information is 0 because radiographing is not carried out at all, it is determined that the radiographing order information does not agree. That is, in case the cassette ID related to the radiographing order information is not stored, the CPU 121 determines that radiographing is not carried out, and that the radiographing order information does not agree. The data as an object of determination is not limited to the number of radiographic images. The radiographing order information is determined to agree or not on the basis of data stored in various items.

When the radiographing order information agrees (Step S1033; YES), the CPU 121 relates the cassette ID related to the received radiographing order information to the radiographign order information stored in the radiographing order information file 1261, stores them (Step S1040), and moves to Step S1037.

On the other hand, when the radiographing order information does not agree (Step S1033; NO), the CPU 121 displays a message for confirming whether to renew content of the radiographing order information stored in the radiographing order information file 1261 and to store content of the received radiographing order information or not, on the display unit 123 (Step S1034). In case the instruction to modify the radiographing order information is inputted through the input unit 122, the CPU 121 displays the above-described input screens 1233 and 1234 for inputting radiographing order information on the display unit 123, to modify the radiographing order information.

Then, the CPU 121 determines whether the instruction to renew the radiographing order information is inputted through the input unit 122 or not (Step S1035). When the instruction to renew the radiographing order information is inputted (Step S1035; YES), the CPU 121 renews the radiographing order information stored in the radiographing order information file 1261 of the storage unit 126 to content of the radiographing order information received from the portable terminal 110 or the modified radiographing order information. Then, the CPU 121 relates the received cassette ID to the renewed rediographing order information and stores them in the radiographing order information file 1261 (Step S1036).

Thereafter, the CPU 121 determines whether to receive the medical image and the cassette ID from the medical image reading apparatus 140 or not (Step S1037). When receiving the medical image and the cassette ID (Step S1037; YES), the CPU 121 relates the medical image to the radiographing order information based on the received cassette ID, and stores them in the radiographing order information file 1261 (Step S1038). Then, when the CPU 121 obtains the radiographing order information and the cassette ID from the radiographing order information file 1261, and transmits them to the information management apparatus 160 according to the instruction by the operator (Step S1039), the CPU 121 terminates the radiographing termination processing.

On the other hand, in Step S1035, when the instruction not to renew the radiograping order information is inputted (Step S1035; NO), the CPU 121 displays a message for confirming whether to cancel the radiographing order information in the portable terminal 110 or not on the display unit 123, and determines whether the instruction to cancel the radiographing order information is inputted or not (Step S1041). When the instruction to cancel the radiographing order information is inputted (Step S1041; YES), the CPU 121 sends an instruction to delete corresponding rediographing order information to the portable terminal 110, and makes the portable terminal 110 delete the radiographing order information (Step S1042). Then, the CPU 121 sets the transmitted flag corresponding to the radigraphing order information OFF (Step S1043), and terminates the radiographing termination processing.

On the other hand, when the instruction to cancel the radiographing order information is not inputted (Step S1042; NO), the CPU 121 suspends corresponding radiographing order information (Step S1044), and terminates the radiographing termination processing.

The CPU 121 of the control apparatus 120 which has terminated the radiographing termination processing transmits the radiographing order information and the cassette ID stored in the radiographing order information file 1261 to the information management apparatus 160. The information management apparatus 160 receives the radiographing order information and the cassette ID from each of a plurality of control apparatuses 120, and stores them in a storage unit which is not shown in figures. Further, the information management apparatus 160 receives the medical image and the cassette ID from the medical image reading apparatus 140, and relates the medical image to the radiographing order information. Therefore, the information management apparatus 160 manages the medical images of the system collectively.

FIG. 12 is a flowchart showing radiographing termination processing performed by the CPU 111 of the portable terminal 110. As shown in FIG. 12, the CPU 111 determines whether the portable terminal 110 is connected to the communication terminal 110-1 or not according to a detection signal outputted from the communication terminal 110-1 (Step S1051). When the portable terminal 110 is connected to the communication terminal 110-1 (Step S1051; YES), the CPU 111 obtains the radiographing order information and the cassette ID from the radiographing order information file 1161, and transmits them to the control apparatus 120 (Step S1052).

Then, the CPU 111 sets the transmitted flag for the radiographing order information including the transmitted cassette ID ON (Step S1053). The CPU 111 determines whether the instruction to cancel the transmitted radiographing order information is received from the control apparatus 120 or not (Step S1054). When the CPU 111 receives the instruction to cancel the radiographing order information from the control apparatus 120 (Step S1054; YES), the CPU 111 deletes corresponding radiographing order information from the radiographing order information file 1161 (Step S1055).

Then, the CPU 111 determines whether radiographing order information which is not transmitted is included in the radiographing order information file 1161 or not (Step S1056). When the radiographing order information which is not transmitted is included (Step S1056; YES), the CPU 111 moves to Step S 1052, and performs the above-described processing continuously. On the other hand, when all radiographing order information included in the radiographing order information file 1161 and the cassette IDs for the radiographing order information are transmitted (Step S1056; NO), the CPU 111 terminates the radiographing termination processing.

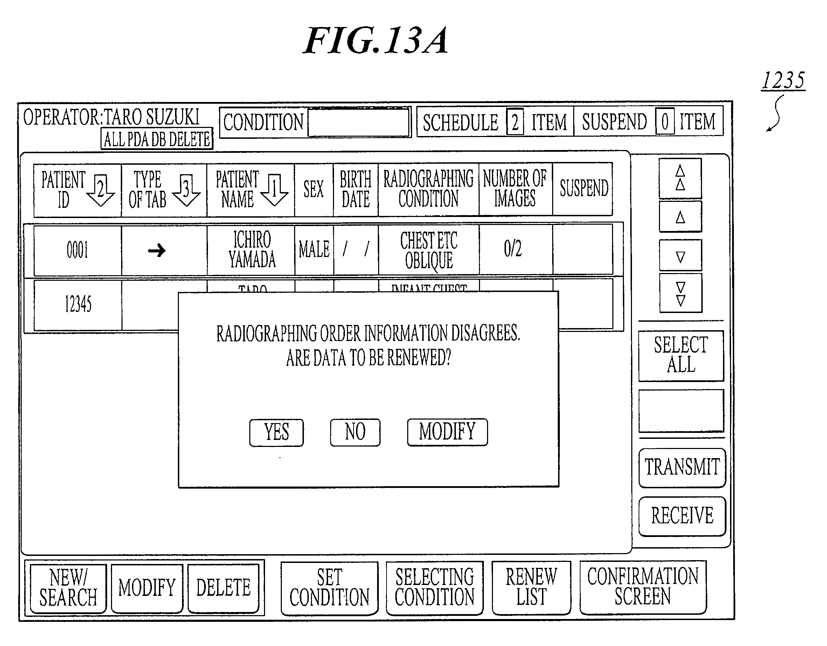

Screens displayed on the display unit 123 of the control apparatus 120 according to the above-described radiographing termination processing will be explained with reference to FIGS. 13A to 14.

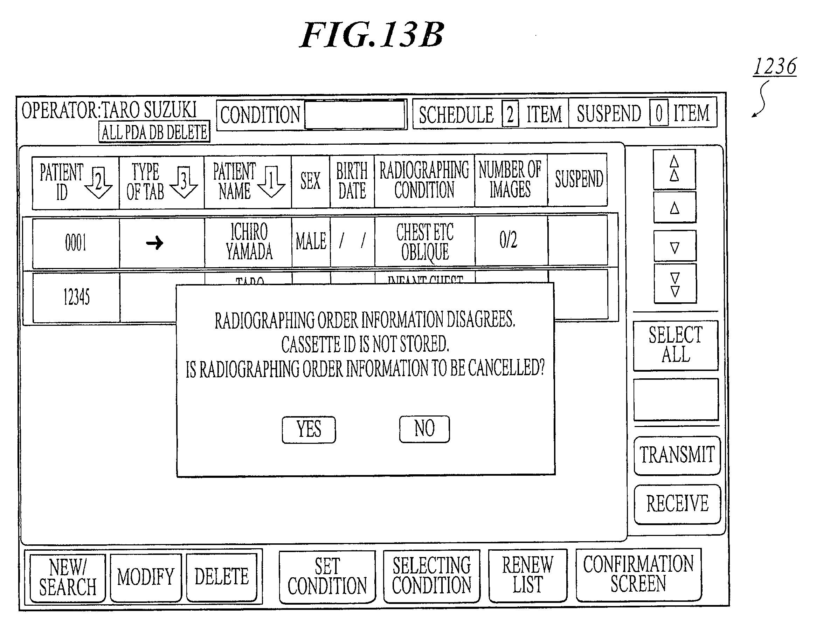

FIGS. 13A and 13B are views showing portable list screens 1235 and 1236 displayed on the display unit 123 when the control apparatus 120 receives the radiographing order information from the portable terminal 110.

FIG. 13A is a view showing a portable list screen 1235 on which a massage for confirming whether to renew the radiographing order information or not is displayed. As shown in FIG. 13A, the potable list screen 1235 has the same screen structure as one of the portable list screen 1232 shown in FIG. 7. As described above, the area for displaying the radiographing order information and the area for displaying the instruction buttons for inputting various instructions are provided on the portable list screen 1235. when the stored radiographing order information disagrees with the radiograping order information received from the portable terminal 110, the confirmation message is displayed on the area on which the corresponding radiographing order information is displayed.

More specifically, the confirmation message shows text data "RADIOGRAPHING ORDER INFORMATION DISAGREES. ARE DATA TO BE RENEWED?". Further, text data "YES", "NO" and "MODIFY" are displayed on a lower section to input the instruction to confirm the renew. When "YES" is operated, the radiographing order information is renewed, and the cassette ID is related to the radiographing order information. When "NO" is operated, the radiographing order information is not renewed, and the cassette ID is related to the radiographing order information. When "MODIFY" is operated, the input screens 1233 and 1234 (with reference to FIGS. 8A and 8B) for inputting the radiographing order information are displayed.

FIG. 13B is a view showing a display screen 1236 on which a massage for confirming whether to cancel the radiographing order information or not is displayed. As shown in FIG. 13B, the area for displaying the patient information and the area for displaying the radiographing order information are provided on the display screen 1236. The confirmation message is displayed on the area on which the corresponding radiographing order information is displayed.

More specifically, the confirmation message shows text data "RADIOGRAPHING ORDER INFORMATION DISAGREES. CASSETE ID IS NOT STORED. IS RADIOGRAPHING ORDER INFORMATION TO BE CANCELLED?". Further, text data "YES" and "NO" are displayed on a lower section to input the instruction to confirm the cancel. When "YES" is operated, the cancel instruction is sent to the portable terminal 110.

When "NO" is operated, that is, when the instruction to renew or cancel the radiographing order information is not inputted in case the radiographing order information received from the portable terminal 110 and the radiographing order information stored in the control apparatus 120 do not agree with each other, the radiographing order information is suspended, and the text data "suspend" is displayed on the item for the suspend.

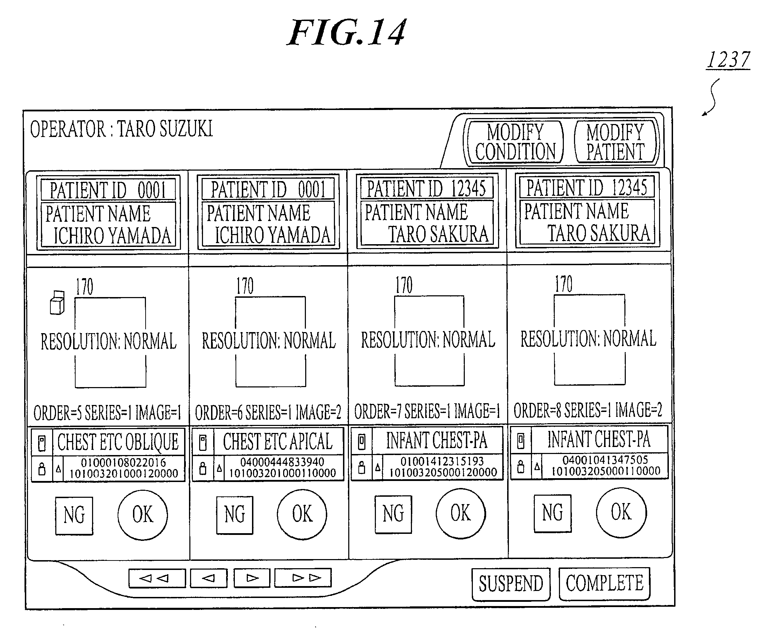

Next, the medical image displayed on the display unit 123 of the control apparatus 120 will be explained with reference to FIG. 14. FIG. 14 is a view showing a portable processing screen 1237 displayed on the display unit 123 in case of radiographing by using the portable terminal 110. As shown in FIG. 14, medical images for a plurality of patients registered in one portable terminal 110 are displayed on one portable processing screen 1237.

That is, an area for displaying operator information, and an area for displaying the patient information, the medical image and the radiographing information for every patient are provided on the portable processing screen 1237. Four types of medical images can be displayed on one portable processing screen 1237. More specifically, an item for displaying text data "TARO SUZUKI" as a name of the operator is provided in the area for displaying operator information at a left upper end section of the portable processing screen 1237. The medical image displayed at a left end section will be explained as the medical image displayed for every patient. An item for displaying numeral data "0001" as the patient ID and an item for displaying text data "ICHIRO YAMADA" as the name are provide in the area for displaying patient information.

An area for displaying the medical image is provided at a lower section. The medical image, an item for displaying numeral data "170" as the reading apparatus ID, and an item for displaying text data "NORMAL" as the resolution are provided in the area. Further, an area for displaying the radiographing information is provided at a much lower section. An item for displaying text data "CHEST ETC OBLIQUE" as the radiographing condition, and an item for displaying numeral data "01000108022016" as the cassette ID are provided in the area.

As described above, according to the medical image radiographing system 100 in the first embodiment, after radiographing, when the portable terminal 110 transmits the cassette ID related to the radiographing order information to the control apparatus 120, the control apparatus 120 determines whether the received radiographing order information agrees with the radiographing order information stored in the radiographing order information file 1261 or not. When the received radiographing order information agrees with the stored radiographing order information, the control apparatus 120 relates the received cassette ID to the radiographing order information and stores them. When the received radiographing order information does not agree with the stored radiographing order information, the control apparatus 120 makes the portable terminal 110 input whether to renew the radiographing order information or not. When the instruction to renew the radiographing order information is inputted, the control apparatus 120 renews the content of the stored radiographing order information to the content of the received radiographing order information, relates the cassette ID to the renewed radiographing order information and stores them.

Consequently, when the radiographing order information stored in the control apparatus 120 and the radiographing order information received from the portable terminal 110 do no agree with each other, because the message for confirming whether to renew the radiographing order information or not is displayed and then the instruction can be inputted, it is possible to deal with a state where the radiographing order information disagrees rapidly by demanding the operator to instruct even when the state is caused. As a result, it is possible to increase efficiency in processing without suspending the processing even when the radiographing order information disagrees. Further, because it is unnecessary to input to add, change or the like the radiographing order information, it is possible to preventing the operator from making input mistake and to manage the radiographing order information accurately. Furthermore, it is possible to save trouble and time required to input the radiographing order information, and to increase efficiency in operations of the operator.

Further, when the radiographing order information disagrees, because the control apparatus 120 can modify and renew the radiographing order information, it is possible to manage the radiographing order information according to the radiographing state. Furthermore, when the radiographing order information disagrees, for example, when the radiographing order information stored in the portable terminal 110 is added or changed due to a factor undesired by the operator such as an erroneous push of the button or the like of the portable terminal 110 while the operator is moving from the hospital room to the radiographing room, it is possible to modify the radiographing order information added or changed and to manage the radiographing order information suitably.

Further, when the radiographing order information agrees, because the received cassette ID is related to the radiographing order information stored in the radiographing order information gile 1261, and stored in the radiographing order information file 1261, it is possible to manage the radiographing order information and the caste ID so as to relate them to each other without the instruction by the operator. When the medical image and the cassette ID related to the medical image are received from the medical image reading apparatus 140, the medical image is related to the radiographing order information and they are managed on the basis of the cassette ID. Consequently, when there occurs no trouble, it is possible to relate the cassette ID to the radiographing order information, to relate the medical image to the radiographing order information and to manage them efficiently with saving trouble of the operator.

Further, when the instruction to cancel the radiographing order information without renewing the radiographing order information is inputted, the control apparatus 120 sends the instruction to cancel the radiographing order information to the portable terminal 110 and makes the portable terminal 110 delete the corresponding radiographing order information. On the other hand, when the instruction to renew and cancel the radiographing order information is not inputted, the control apparatus 120 suspends the corresponding radiographing order information.

Consequently, when the radiographing is not carried out on the basis of the radiographing order information transmitted to the portable terminal 110, because the message for confirming whether to cancel the radiographing order information or not is displayed, it is possible that the operator again confirms that there is radiographing order information for the radiographing which has not been carried out. As a result, it is possible to call an attention to the operator and to prevent the operator from forgetting to radiograph. Further, because the radiographing order information for the radiographing which has not been carried out is canceled in the portable terminal 110, the radiographing order information for the radiographing which has not been carried out cannot be kept stored in the portable terminal 110 for long time. That is, because the radiographing order information for the radiographing which has not been carried out stored in the portable terminal 110 is cancelled, and the transmitted flag is set OFF, it is possible that the control apparatus 120 manages the radiographing order information again. As a result, it is possible to prevent the operator from forgetting to radiographing, to distribute the operation, and to increase efficiency in the operation.

Further, the control apparatus 120 transmits the radiographing order information and the cassette ID stored in the radiographing order information file 1261 according to the above-described processing to the information management apparatus 160. Accordingly, when the radiographing is carried out actually, because the radiographing order information is renewed and stored, the control apparatus 120 can transmit the radiographing order information having the renewed content to the information management apparatus 160. Further, because the information management apparatus 160 can manage the radiographing order information or the medical image on the basis of the newest information, the information management apparatus can share the information in the whole system, and to use the system efficiently.

Although the present invention has been explained according to the above-described first embodiment which is an example of the medical image photographing system or the medical image management method suitable to the present invention, it should also be understood that the present invention is not limited to the embodiment.

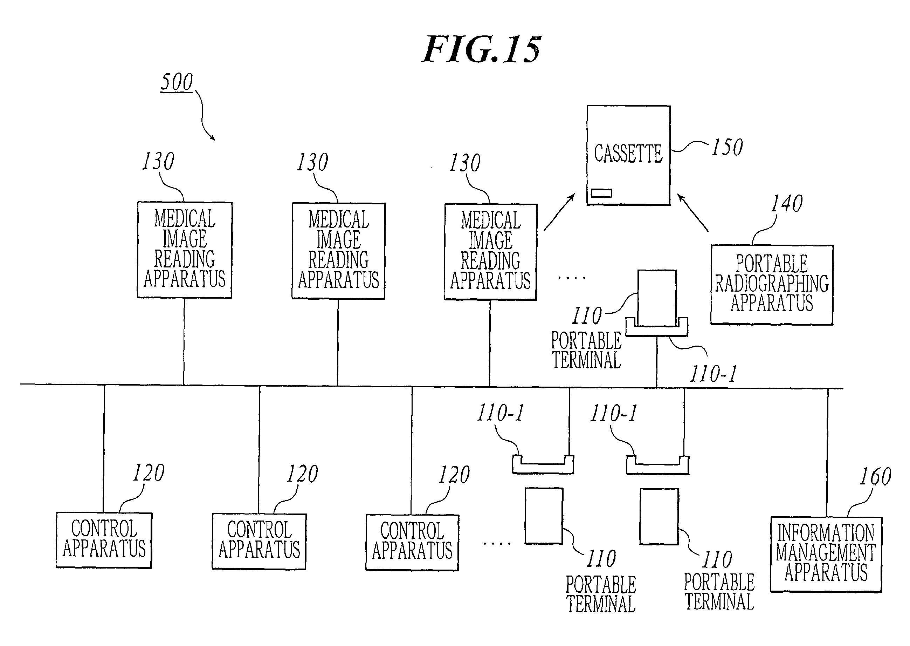

For example, the system structure of the medical image radiographing system 100 as described above is an example of the present invention, and the present invention is not limited to the system structure. As shown in a medical image radiographing system 500 shown in FIG. 15, the communication terminal 110-1 for controlling the communication between the portable terminal 110 and the control apparatus 120 may not be connected to the control apparatus 120 directly. The communication terminal 110-1 may be connected to the network N, and communicate with a plurality of control apparatuses 120 through the network N arbitrarily.

In the medical image radiographing system 500, the radiographing order information transmitted from the control apparatus 120 to the portable terminal 110 may include information (for example, an IP address or the like for identifying a computer on the network) on an apparatus to which the cassette ID related to the radiographing order information is to be transmitted. That is, because arbitrary information can be transmitted to a plurality of control apparatuses 120 connected to the network N, the CPU 11 of the portable terminal 110 detected to be connected to the communication terminal 110-1 obtains information on an apparatus to which the cassette ID is to be transmitted from the radiographing order information, and transmits the cassette ID related to the radiographing order information to the control apparatus 120 corresponding to the obtained information of the apparatus.

Further, the portable terminal 110 may store identification information on the control apparatus 120 from which the radiographing order information is received, and may transmit the cassette ID related to the radiographing order information to the control apparatus 120 from which the radiographing order information is received in case the radiographing order information received from the control apparatus 120 does not include information on an apparatus to which the cassette ID is to be transmitted. Further, an apparatus to which the cassette ID is to be transmitted may be specified through the operation unit 112 of the portable terminal 110, and necessary information may be transmitted to the specified apparatus.

According to the structure, because the portable terminal 110 can transmit information to the desired control apparatus 120 even if the operator or the like is anywhere in the hospital, for example, in case information becomes necessary immediately, it is possible to transmit necessary information quickly. Further, when the storage unit 116 of the portable terminal 110 exceeds the capacity, it is unnecessary that the operator or the like goes to the radiographing room, and it is possible to transmit the information quickly, to back-up data and to increase convenience.

Further, although the present invention has been explained according to the first embodiment which is an example of the case the control apparatus 120 stores the number of photographic images radiographed actually and determines whether the radiographing order information agrees not according to the stored number of photographic images, the present invention is not limited to the embodiment. When radiographing different from content instructed by the radiographing order information is carried out, data indicating the content of the different radiographing may be stored in a corresponding item of the radiographing order information file 1161. Accordingly, it is possible to determine whether the radiographing order information stored in the control apparatus 120 agrees with the radiographing order information stored in the portable terminal 110 for every item, and to renew, cancel or the like the radiographing order information.

Further, it should also be understood that the detailed structure and the detailed operation of each element of the medical image radiographing system 100 according to the embodiment are not limited to the embodiment and various changed and modifications may be made to the invention without departing from the gist thereof.

According to the present invention, only when the radiographing order information stored in the storage unit of the control apparatus agrees with the radiographing order information received from the portable terminal, it is possible that the control apparatus relates the identification information of the cassette ID received from the portable terminal to the radiographing order information and stores them in the storage unit. Consequently, it is possible to prevent the state that the control apparatus stores the radiographing order information which disagrees with the radiographing order information stored in the storage unit, and to relate and manage the radiographing order information and the identification information of the cassette efficiently.

Further, when the radiographing order information stored in the storage unit of the control apparatus disagrees with the radiographing order information received from the portable terminal, it is possible to display the message for confirming to renew the radiographing order information or not, and to make the operator or the like input the instruction to renew the radiographing order information or not. Consequently, when the radiographing order information disagrees, it is possible to renew the radiographing order information according to the decision of the operator. Even when the radiographing is not carried out according to the instruction of the radiographing order information, it is possible to modify and manage the radiographing order information according to the change.

Further, when the radiographing order information disagrees, for example, when radiographing order information is changed according to the radiographing state, or when the radiographing order information stored in the portable terminal is changed due to the factor undesired by the operator, the control apparatus can modify or add the changed part, and manage the radiographing order information suitably.

Further, when the radiographing order information disagrees, the control apparatus displays the message for confirming to cancel the radiographing order information or not, and makes the operator or the like input the instruction to cancel the radiographing order information or not. When the instruction to cancel the radiographing order information is inputted, the control apparatus sends the instruction to cancel the radiographing order information to the portable terminal, and deletes the corresponding radiographing order information stored in the portable terminal. Consequently, for example, when the identification information of the cassette is not related to the radiographing order information received from the portable terminal, it is possible that the operator determines that the radiographing is not carried out, and that the radiographing order information stored in the portable terminal is deleted. As a result, the radiographing order information for the radiographing which is not carried out is not kept in the portable terminal for a long time. Consequently, it is possible to prevent the operator or the like from misunderstanding the radiographing order information for the radiographing which is forgotten to be carried out and preparing the radiographing.

Further, the control apparatus can transmit the radiographing order information and the identification information on the cassette stored in the storage unit to the information management apparatus, and the information management apparatus can manage the radiographing order information and the identification information on the caste received from a plurality of control apparatuses in a concentrated way. That is, the information management apparatus can receive the radiographing order information and the identification information of the cassette which are newest information, and manage the whole medical image radiographing system efficiently on the basis of the information.

### [Second Embodiment]

According to the above-described embodiment, the medical image radiographing system capable of properly modifying the radiographing order information with the control apparatus in case the radiographing different from the instruction of the radiographing order information is carried out, in case the radiographing based on the radiographing order information is not carried out or the like, has been explained. According to a second embodiment, a medical image radiographing system capable of properly modifying the radiographing order information with the portable terminal in the above-described case, will be explained.

Hereinafter, the second embodiment of the present invention will be explained in detail, with reference to figures. Like the case of the first embodiment, a definition of the present invention is not limited to the figures. As an example of a characteristic embodiment of the present invention, a medical image radiographing system 200 for radiographing in a ward with a portable terminal which is portable and a radiographing apparatus which is portable will be explained.

First, a structure of the second embodiment will be explained.

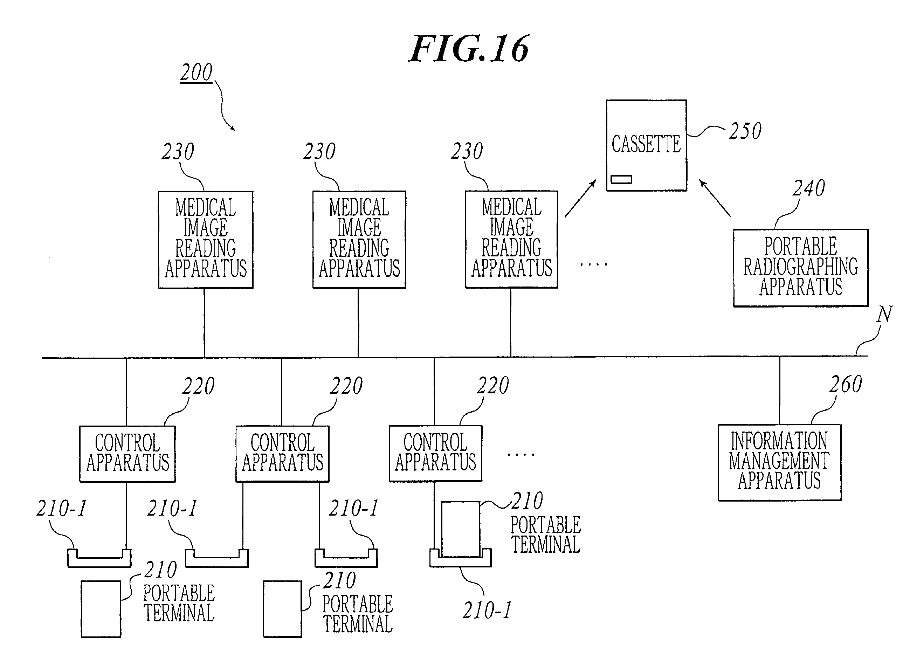

FIG. 16 is a conceptual view showing a system structure of the medical image radiographing system 200 of the present invention. As shown in FIG. 16, the medical image radiographing system 200 comprises a portable terminal 210, a communication terminal 210-1, a control apparatus 220, a medical image reading apparatus 230, a portable radiographing apparatus 240, a cassette 250, an information management apparatus 260 or the like, like the medical image radiographing system 100 according to the first embodiment. Each apparatus of the medical image radiographing system 200 comprises substantially the same structure and function as those of each corresponding apparatus of the medical image radiographing system 100 according to the first embodiment, and it is omitted to explain them.

Next, the operation according to the second embodiment will be explained.

The program realizable of each function described in the following flowcharts is stored in a storage unit 216 of the portable terminal 210 or a storage unit 226 of the control apparatus 220 in a format of program codes readable by a computer. Therefore, a CPU 211 of the portable terminal 210 or a CPU 221 of the control apparatus 220 performs the operation according to the program codes in order.

The CPU 221 of the control apparatus 220 and the CPU 221 of the portable terminal 210 performs radiographing preparation processing for transmitting radiographing order information from the control apparatus 220 to the portable terminal 210 in the radiographing room will be explained as preparation processing for radiographing. The radiographing preparation processing is the same as the radiographing preparation processing (with reference to FIGS. 5A and 5B) executed by the CPU 121 of the control apparatus 120 and the CPU 111 of the portable terminal 110 according to the first embodiment, and it is omitted to explain it. Further, screens displayed on a display unit 223 of the control apparatus 220 according to the radiographing preparation processing are the same as the screens (shown in FIGS. 6 to 8B) displayed on the display unit 123 of the control apparatus 120 according to the first embodiment, and it is omitted to explain them.

Next, radiographing start processing for relating the patient ID and the cassette ID to radiographing order information performed by the portable terminal 210 in a room for radiographing before starting radiographing will be explained.

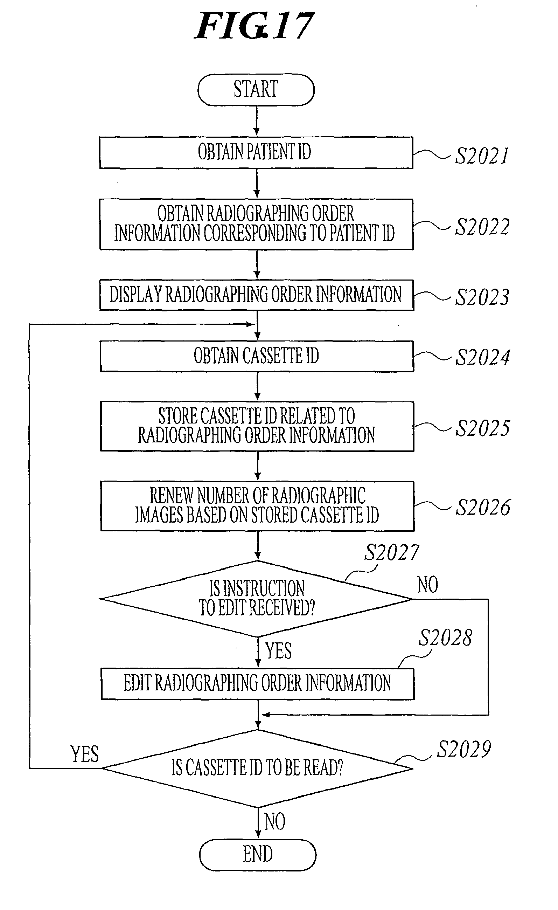

FIG. 17 is a flowchart showing radiographing start processing performed by the CPU 211 of the portable terminal 210. As shown in FIG. 17, the CPU 211 executes the same processing as Steps S 1021 to S 1026 in the radiographing start processing (FIG. 9) according to the first embodiment. That is, the CPU 211 obtains the patient ID (Step S2021), obtains radiographing order information corresponding to the read patient ID out of the radiographing order information file 2161 (Step S2022), displays the obtained radiographing order information on the display unit 213 (Step S2023), reads the barcode attached to the cassette 250 used for radiographing, and obtains the cassette ID (Step S2024). Then, the CPU 211 relates the obtained cassette ID to the radiographing order information, stores them in the radiographing order information file 2161 (Step S2025), renews and stores the number of radiographic images on the basis of the cassette ID related to the radiographing order information and stored in the radiographing order information file 2161 (Step S2026).

Then, when the CPU 211 receives the instruction to edit the radiographing order information displayed on the display unit 213 through the input unit 212 (Step S2027; YES), the CPU 221 edits the radiographing order information according to inputted content, and stores the edited radiographing order information in the radiographing order information file 2161 (Step S2028).

Then, the CPU 211 determines whether the cassette ID is to be read or not continuously (Step S2029). When the cassette ID is to be read continuously (Step S2029; YES), the CPU 211 returns to Step S2024, and performs the above-described processing continuously. On the other hand, when the cassette ID is not to be read (Step S2029; NO), the CPU 211 terminates the radiographing start processing.

Screens displayed on the display unit 213 of the portable terminal 210 according to the above-described radiographing start processing are the same as the screens (FIGS. 10A and 10B) displayed on the display unit 113 of the portable terminal 110 according to the radiographing start processing in the first embodiment, and it is omitted to show them in figures and explain them.

Next, radiographing termination processing for transmitting radiographing order information and the cassette ID from the portable terminal 210 to the control apparatus 220 in the room after radiographing will be explained, as characteristic processing of the present invention.

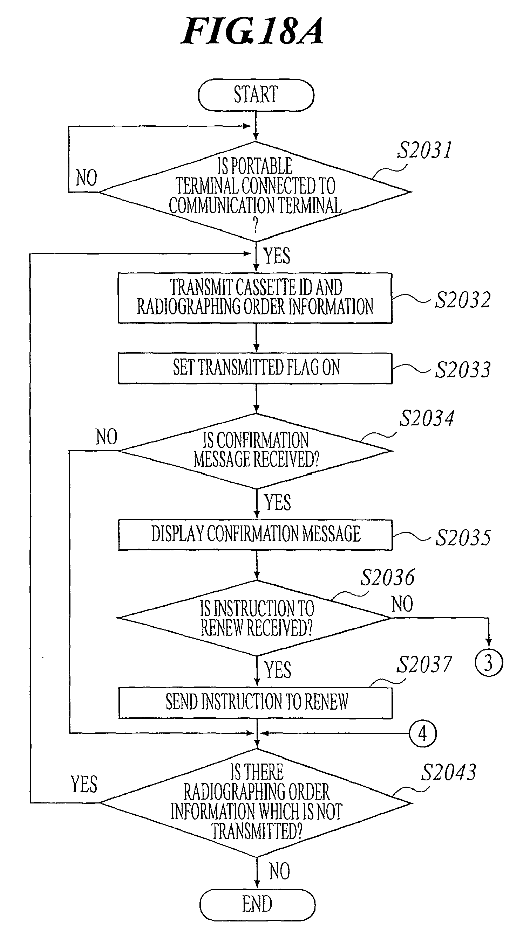

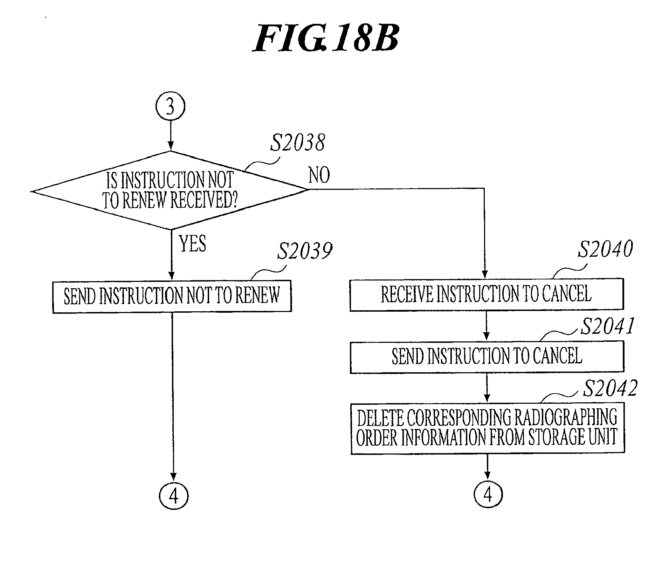

FIGS. 18A and 18B are flowcharts showing radiographing termination processing performed by the CPU 211 of the portable terminal 210. As shown in FIGS. 18A and 18B, the CPU 211 determines whether the portable terminal 210 is connected to the communication terminal 210-1 or not according to a detection signal outputted from the communication terminal 210-1 (Step S2031). When the portable terminal 210 is connected to the communication terminal 210-1 (Step S2031; YES), the CPU 211 obtains the radiographing order information and the cassette ID from the radiographing order information file 2161, and transmits them to the control apparatus 220 (Step S2032).

Then, the CPU 211 sets the transmitted flag for the radiographing order information including the transmitted cassette ID ON (Step S2033). The CPU 211 determines whether to receive the confirmation message from the control apparatus 220 or not (Step S2034). The confirmation message is displayed when the transmitted radiographing order information disagrees with the radiographing order information stored in the control apparatus 220. More specifically, when the transmitted radiographing order information is different from the radiographing order information stored in the control apparatus 220 in data, the message for confirming whether to renew the radiographing order information stored in the control apparatus 220 to the transmitted radiographing order information or not is displayed. When the transmitted radiographing order information does not include the cassette ID, and the number of photographic images is 0, the message for confirming whether to cancel the radiographing order information or not is displayed.

When the CPU 211 does not receive the conformation message (Step S2034; NO), the CPU 211 determines that the transmitted radiographing order information agrees with the radiographing order information stored in the control apparatus 220, and moves to Step S2043.

On the other hand, when the CPU 211 receives the confirmation message (Step S2034; YES), the CPU 211 displays the received confirmation message on the display unit 213 (Step S2035), and determines whether various instructions are received through the input unit 212 or not (Step S2035). First, when the operator inputs the instruction to renew the radiographing order information (Step S2036; YES), the CPU 211 sends the instruction to renew the radiographing order information to the control apparatus 220 (Step S2037).

When the operator inputs the instruction not to renew the radiographing order information (Step S2038; YES), the CPU 211 sends the instruction not to renew the radiographing order information to the control apparatus 220 (Step S2039). When the operator inputs the instruction to cancel the radiographing order information (Step S2040), the CPU 211 sends the instruction to cancel the radiographing order information to the control apparatus 220 (Step S2041), and deletes the corresponding radiographing order information from the radiographing order information file 2161 (Step S2042).

Then, the CPU 211 moves to Step 2043, and determines whether radiographing order information which is not transmitted is included in the radiographing order information file 2161 or not (Step S2043). When the radiographing order information which is not transmitted is included (Step S2043; YES), the CPU 211 moves to Step S2032, and performs the above-described processing continuously. On the other hand, when all radiographing order information included in the radiographing order information file 2161 and the cassette IDs for the radiographing order information are transmitted (Step S2043; NO), the CPU 211 terminates the radiographing termination processing.

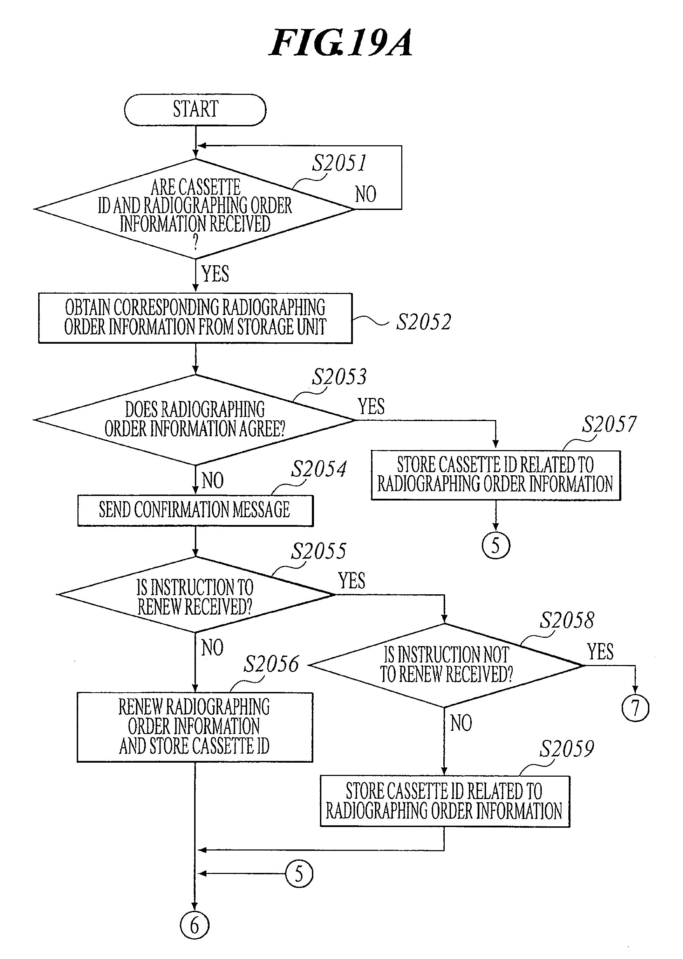

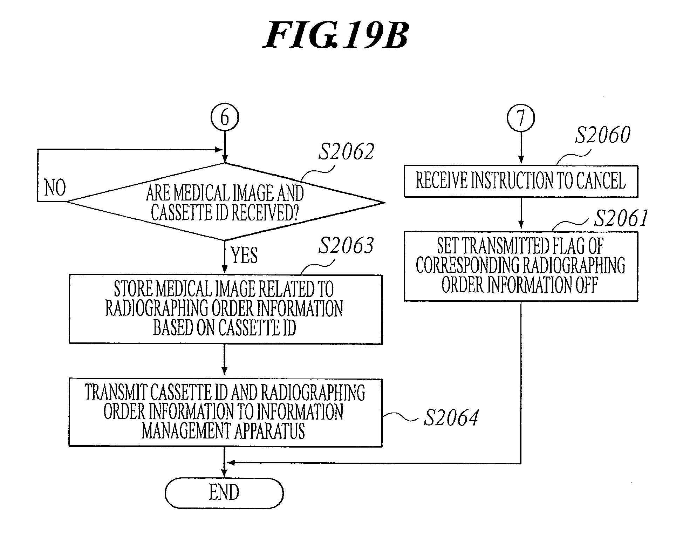

FIGS. 19A and 19B are flowcharts showing radiographing termination processing performed by the CPU 221 of the control apparatus 220. As shown in FIGS. 19A and 19B, when the CPU 221 receives the cassette ID related to radiographing order information from the portable terminal 210 through the communication terminal 210-1 (Step S2051; YES), the CPU 221 obtains radiographing order information corresponding to the received radiographing order information from the radiographing order information file 2261 (Step S2052). Then, the CPU 221 compares the radiographing order information received from the portable terminal 210 with the radiographing order information obtained from the radiographing order information 2261, and determines whether the radiographing order information agrees with each other or not (Step S2053).

Herein, the case the radiographing order information does not agree means the case the number of radiographic images stored in the received radiographing order information is different from the number of radiographic images stored in the radiographing order information obtained from the radiographing order information file 2261, and in the case, it is determined that the radiographing order information does not agree. Further, in the case the number of radiographic images stored in the received radiographing order information is 0 because radiographing is not carried out at all, it is determined that the radiographing order information does not agree. That is, in case the cassette ID related to the radiographing order information is not stored, the CPU 221 determines that radiographing is not carried out, and that the radiographing order information does not agree. The data as an object of determination is not limited to the number of radiographic images. The radiographing order information may be determined whether to agree or not on the basis of data stored in various items of the radiographing order information edited by the portable terminal 210.

When the radiographing order information agrees (Step S2053; YES), the CPU 221 relates the cassette ID related to the received radiographing order information to the radiographign order information stored in the radiographing order information file 2261, stores them (Step S2057), and moves to Step S2062.

On the other hand, when the radiographing order information does not agree (Step S2053; NO), the CPU 221 sends the confirmation message for confirming whether to renew the content of the radiographing order information stored in the radiographing order information file 2261 to the content of the radiographing order information received and store it or not, or for confirming whether to cancel the radiographing order information or not, to the portable terminal 210 (Step S2054). Then, the CPU 221 is determines whether the instruction to renew the radiographing order information is received from the portable terminal 210 or not (Step S2055). When the CPU 221 receives the instruction to renew the radiographing order information (Step S2055; YES), the CPU 221 renews the radiographing order information stored in the radiographing order information file 2261 of the storage unit 226 to the content of the radiographing order information received from the portable terminal 210. Further, the CPU 221 stores the cassette ID related to and received with the renewed radiographing order information in the radiographing order information file 2261 (Step S2056).

When the CPU 221 receives the instruction not to renew the radiographing order information from the portable terminal 210 (Step S2058; YES), the CPU 221 relates the received cassette ID to the radiographing order information and stores them without renewing the radiographing order information stored in the radiographing order information file 2261 (Step S2059).

When the CPU 221 receives the instruction to cancel the radiographing order information from the portable terminal 210 (Step S2060), the CPU 221 sets the transmitted flag of the corresponding radiographing order information OFF without storing the received radiographing order information and the received cassette ID in the radiographing order information file 2261 (Step S2061).

Thereafter, the CPU 221 determines whether to receive the medical image and the cassette ID from the medical image reading apparatus 240 or not (Step S2062). When receiving the medical image and the cassette ID (Step S2062; YES), the CPU 221 relates the medical image to the radiographing order information based on the received cassette ID, and stores them in the storage unit 226 (Step S2063). Then, when the CPU 221 obtains the radiographing order information and the cassette ID from the radiographing order information file 2261, and transmits them to the information management apparatus 260 according to the instruction by the operator (Step S2064), the CPU 221 terminates the radiographing termination processing.

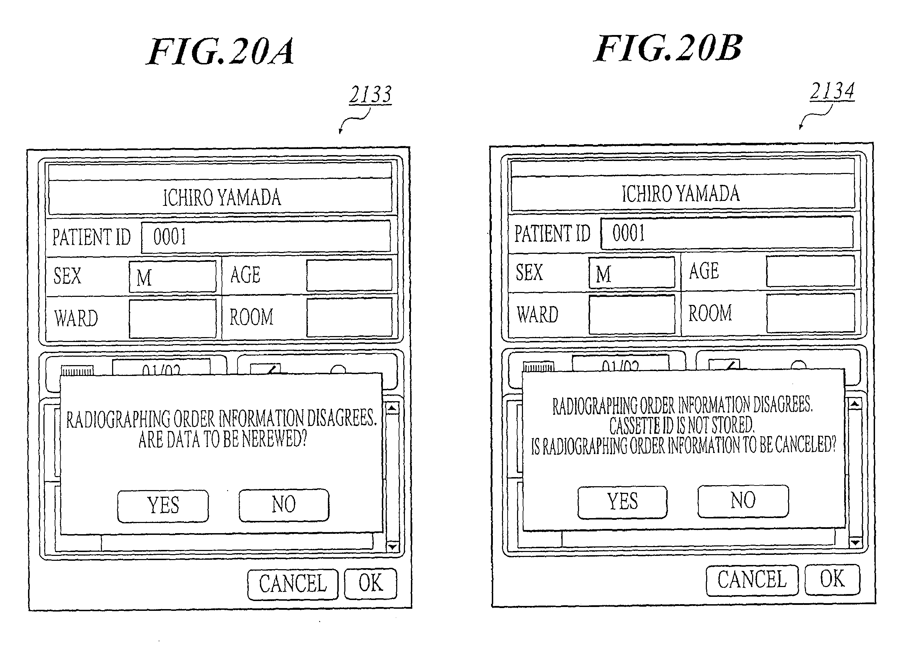

Screens displayed on the display unit 213 of the portable terminal 210 according to the above-described radiographing termination processing will be explained with reference to FIGS. 20A and 20B. FIGS. 20A and 20B are views showing confirmation screens 2133 and 2134 displayed on the portable terminal 210 when the radiographing order information transmitted to the control apparatus 220 and the radiographing order information stored in the control apparatus 220 disagree with each other.

FIG. 20A is a view showing a display screen 2133 on which a massage for confirming whether to renew the radiographing order information stored in the control apparatus 220 or not is displayed. As shown in FIG. 20A, the area for displaying the patient information and the area for displaying the radiographing order information are provided on the display screen 2133. The confirmation message is displayed on the area on which the corresponding radiographing order information is displayed.

More specifically, the confirmation message shows text data "RADIOGRAPHING ORDER INFORMATION DISAGREES. ARE DATA TO BE RENEWED?". Further, text data "YES" and "NO" are displayed on a lower section to input the instruction to confirm the renew. When "YES" is operated, the instruction to renew the radiographing order information is sent to the control apparatus 220. When "NO" is operated, the instruction not to renew the radiographing order information is sent to the control apparatus 220.

FIG. 20B is a view showing a display screen 2134 on which a massage for confirming whether to cancel the radiographing order information stored in the portable terminal 210 or not is displayed. As shown in FIG. 20B, the area for displaying the patient information and the area for displaying the radiographing order information are provided on the display screen 2134. The confirmation message is displayed on the area on which the corresponding radiographing order information is displayed.

More specifically, the confirmation message shows text data "RADIOGRAPHING ORDER INFORMATION DISAGREES. CASSETE ID IS NOT STORED. IS RADIOGRAPHING ORDER INFORMATION TO BE CANCELLED?". Further, text data "YES" and "NO" are displayed on a lower section to input the instruction to confirm the cancel. When "YES" is operated, the cancel instruction is sent to the control apparatus 220.

The portable processing screen displayed on the display unit 223 of the control apparatus 220 when the radiographing is carried out by using the portable terminal 210 is the same as the portable processing screen (with reference to FIG. 14) displayed on the display unit 123 of the control apparatus 120 according to the first embodiment, and it is omitted to show it in figures and explain it.

As described above, according to the medical image radiographing system 200 in the second embodiment, in case the radiographing is carried out based on the radiographing order information in the hospital room, when the radiographing state is required to be changed according to the condition of the patient or the like, the radiographing order information stored in the portable terminal 210 is edited by the portable terminal 210, and the content based on the radiographing state is stored in the portable terminal 210. After radiographing, when the portable terminal 210 is connected to the communication terminal 210-1, the portable terminal 210 transmits the radiographing order information and the cassette ID stored in the radiographing order information file 2161 to the control apparatus 220. Then, the control apparatus 220 determines whether the received radiographing order information agrees with the radiographing order information stored in the radiographing order information file 2261 or not.

When the received radiographing order information disagrees with the stored radiographing order information, the control apparatus 220 sends the confirmation message for confirming whether to renew the stored radiographing order information or not, or whether to cancel the received radiographing order information or not, to the portable terminal 210, and receives the instruction to renew, not to renew, or to cancel the radiographing order information from the portable terminal 210. Then, the control apparatus 220 renews the radiographing order information, relates the radiographing order information to the cassette ID, cancels the radiographing order information or the like, according to various instructions received from the portable terminal 210. When the received radiographing order information agrees with the stored radiographing order information, the control apparatus 220 relates the received cassette ID to the radiographing order information and stores them.

Consequently, when the radiographing is carried out in the hospital room, in case the radiographing state is changed according to the condition of the patient, the portable terminal 210 can edit the radiographing order information stored in the storage unit 216, and store the radiographing order information according to the radiographing state at the time. Further, when the portable terminal 210 transmits the radiographing order information to the control apparatus 220 after radiographing, the control apparatus 220 can renew the radiographing order information stored therein on the basis of the edited radiographing order information, relate the cassette ID to the renewed radiographing order information and store them. As a result, when the radiographing state is changed, because the portable terminal 210 can store the changed content exactly and accurately, and the control apparatus 220 can reflect and manage the changed content after radiographing order information, it is possible to manage the radiographing order information completely. Further, because it is unnecessary to input the radiographing order information added, changed or the like in the portable terminal 210 to the control apparatus 220 again, it is possible to save trouble and time required to input the radiographing order information, and to increase efficiency in the operation of the operator.

Further, the control apparatus 220 determines whether the radiographing order information received from the portable terminal 210 agrees with the radiographing order information stored in the radiographing order information file 2261 or not. When they disagree with each other, because the control apparatus 220 sends the confirmation message to the portable terminal 210, the operator can confirm that the radiographing order information is edited, and then send the instruction to renew the radiographing order information. Further, even when the radiographing order information stored in the portable terminal 210 is changed according to the radiographing state, the processing is not suspended due to the factor that the radiographing order information disagrees.

Further, when the control apparatus 220 receives the instruction not to renew the radiographing order information from the portable terminal 210, the control apparatus 220 can relate the received cassette ID to the radiographing order information stored in the radiographing order information file 2261 and store them without renewing the radiographing order information. Consequently, when the radiographing order information stored in the portable terminal 210 is edited due to the factor undesired by the operator, it is possible to instruct whether to renew the radiographing order information or not according to the decision of the operator, and to manage the radiographing order information properly.

Further, when the portable terminal 210 receives the instruction to cancel the radiographing order information, the portable terminal 210 sends the instruction to cancel the radiographing order information to the control apparatus 220, and cancels the corresponding radiographing order information from the radiographing order information file 2161. On the other hand, when the control apparatus 220 receives the instruction to cancel the radiographing order information, the control apparatus 220 sets the transmitted flag for the corresponding radiographing order information OFF. Consequently, the control apparatus 220 can again manage the radiographing order information which is transmitted to the portable terminal 210 but based on which the radiographing is not carried out. As a result, it is possible to prevent the operator or the like from forgetting to radiographing. Further, it is possible to distribute the operation and to increase efficiency in the operation.

When the received radiographing order information agrees with the stored radiographing order information, because the control apparatus 220 relates the received cassette ID to the radiographing order information stored in the radiographing order information file 2261 and stores them, the control apparatus 220 can relate and manage the radiographing order information and the caste ID without the instruction by the operator. Further, when the control apparatus 220 receives the medical image and the cassette ID related to the medical image from the medical image reading apparatus 240, the control apparatus 220 relates the medical image to the radiographing order information based on the cassette ID and manages them. Consequently, when there does not occur trouble, it is possible that the control apparatus 220 relates the cassette ID to the radiographing order information efficiently, relates the medical image to the radiographing order information and manages them with saving trouble of the operator.

Further, the control apparatus 220 transmits the radiographing order information and the cassette ID stored in the radiographing order information file 2261 according to the above-described processing to the information management apparatus 260. Accordingly, when the radiographing is carried out actually, because the radiographing order information is renewed and stored, the control apparatus 220 can transmit the radiographing order information having the renewed content to the information management apparatus 260. Further, because the information management apparatus 260 can manage the radiographing order information or the medical image on the basis of the newest information, the information management apparatus can share the information in the whole system, and use the system efficiently.

Although the present invention has been explained according to the above-described second embodiment which is an example of the medical image photographing system or the medical image management method suitable to the present invention, it should also be understood that the present invention is not limited to the embodiment.

For example, the system structure of the medical image radiographing system 200 as described above is an example of the present invention, and the present invention is not limited to the system structure. Like the case of the medical image radiographing system 100 according to the first embodiment, the communication terminal 210-1 for controlling the communication between the portable terminal 210 and the control apparatus 220 may not be connected to the control apparatus 220 directly. The communication terminal 210-1 may be connected to the network N, and communicate with a plurality of control apparatuses 220 through the network N arbitrarily. The structure is the same as one of the medical image radiographing system 500 shown in FIG. 15, and it is omitted to show it in figures and to explain it.

The present invention has been explained according to the second embodiment wherein the control apparatus 220 sends the confirmation message to the portable terminal 210, and the portable terminal 210 inputs various instructions and sends them to the control apparatus 220, as an example. However, the confirmation message may be displayed on the display unit 223 of the control apparatus 220, and various instructions may be inputted through the input unit 222 of the control apparatus 220. Further, the radiographing order information may be edited through the input unit 222 of the control apparatus 220, and renewed according to the radiographing state.

Further, the control apparatus 220 may have a structure for always renewing the radiographing order information stored in the radiographing order information file 2261 to the received radiographing order information and storing the renewed radiographing order information when the received radiographing order information disagrees with the radiographing order information stored in the radiographing order information file 2261. Further, the control apparatus 220 may have a structure for processing any one of setting for always renewing the radiographing order information and setting for transmitting the confirmation message to the portable terminal 210 and renewing the radiographing order information according to the instruction by the portable terminal 210, according to user setting.

Further, it should also be understood that the detailed structure and the detailed operation of each element of the medical image radiographing system 200 according to the second embodiment are not limited to the embodiment and various changed and modifications may be made to the invention without departing from the gist thereof.

According to the present invention, for example, when the radiographing state is changed according to the condition of the patient or the like, it is possible to edit the radiographing order information according to the changed content by the portable terminal. Further, because the portable terminal transmits the edited radiographing order information to the control apparatus, and the control apparatus renews the radiographing order information on the basis of the received radiographing order information, it is possible to manage the identification information on the cassette on the basis of the newest radiographing order information.

Further, when the radiographing order information received from the portable terminal disagrees with the radiographing order information stored in the storage unit, the control apparatus can send the confirmation message to the portable terminal, renew and store the radiographing order information according to the instruction received from the portable terminal. For example, when the radiographing order information is edited according to the radiographing state, the operator can confirm the edited radiographing order information, send the instruction to renew the radiographing order information, and make the control apparatus renew the radiographing order information. As a result, it is possible to improve confidence in the radiographing order information.

Further, when the control apparatus receives the instruction not to renew the radiographing order information, the control apparatus does not renew the radiographing order information. Consequently, in case the radiographing order information stored in the portable terminal is edited according to the factor undesired by the operator, it is possible to manage the radiographing order information properly without renewing the radiographing order information stored in the control apparatus by mistake.

Further, when the radiographing order information disagrees, the control apparatus sends the confirmation message for confirming whether to cancel the radiographing order information or not to the portable terminal, and receives the instruction to cancel the radiographing order information or not from the portable terminal. When receiving the instruction to cancel the radiographing order information, the control apparatus sets the transmission history of the corresponding radiographing order information to be not transmitted, and stores the transmission history. Consequently, for example, it is possible that the control apparatus again manages the radiographing order information for the radiographing which is not carried out. As a result, it is possiblt ot prevent the operator or the like from forgetting to radiographing, and distribute the operation again.

Further, when the control apparatus transmits the radiographing order information and the identification information on the cassette stored in the storage unit to the information management apparatus, the information management apparatus can manage the radiographing order information and the identification information of the cassette received from a plurality of control apparatuses in a concentrated way. That is, because the information management apparatus receives the radiographing order information and the identification information on the cassette which are renewed to newest information, the information management apparatus can manage the whole medical image radiographing system efficiently.

### [Third Embodiment]

According to the second embodiment, the medical image radiographing system capable of properly modifying the radiographing order information in the portable terminal has been explained. According to a third embodiment, a medical image radiographing system capable of efficiently adding the radiographing order information in a portable terminal will be explained.

Hereinafter, the third embodiment of the present invention will be explained in detail, with reference to figures. A definition of the present invention is not limited to the figures, like the cases of the first and second embodiments. As an example of a characteristic embodiment of the present invention, a medical image radiographing system 300 for radiographing in a ward with a portable terminal which is portable and a radiographing apparatus which is portable will be explained.

First, a structure of the third embodiment will be explained.

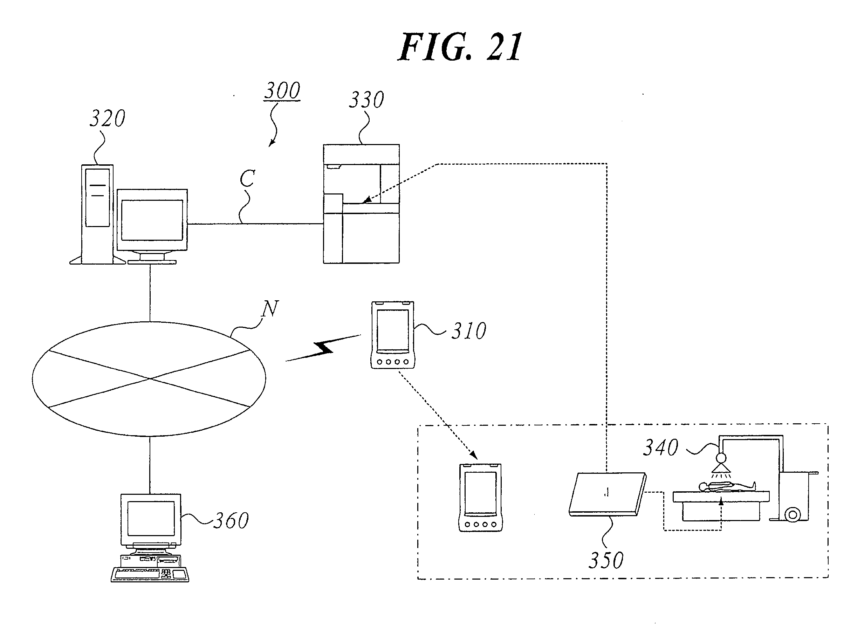

FIG. 21 is a conceptual view showing a system structure of the medical image radiographing system 300 of the present invention. As shown in FIG. 21, the medical image radiographing system 300 comprises a control apparatus 320, an information management apparatus 360, a medical image reading apparatus 330, a portable terminal (for example, PDA (Personal Digial Assistance)) 310, a portable radiographing apparatus 340, a cassette 350 or the like, substantially like the medical image radiographing system 100 according to the first embodiment, and the medical image radiographing system 200 according to the second embodiment. In the medical image radiographing system 300, the control apparatus 320, the information management apparatus 360 and the portable terminal 310 are connected to each other through a network N so that data are transmitted between them. The control apparatus 320 is connected to the medical image reading apparatus 330 through a cable C. Although FIG. 21 shows an example that one control apparatus 320, one information management apparatus 360, one medical image reading apparatus 330, one portable terminal 310 and one portable radiographing apparatus 340 are connected, the number of each apparatus is not limited to one specially.

The control apparatus 320 is connected to the medical image reading apparatus 330 through the cable C, and connected to the information management apparatus 360 and the portable terminal 310 through the network N. The control apparatus 320 receives radiographing order information from the information management apparatus 360, extracts radiographing order information for radiographing with X-ray in the hospital room from the received radiographing order information, and transmits the extracted radiographing order information to the portable terminal 310. Further, the control apparatus 320 receives added radiographing order information from the portable terminal 310, and adds the received radiographing order information to a radiographing order information file 3161, and renews the radiographing order information. Further, the control apparatus 320 controls the medical image reading apparatus 330 according to the renewed radiographing order information, makes the medical image reading apparatus 330 read radiation image information accumulated in a cassette 350 inserted therein, receives medical image data from the medical image reading apparatus 330, and performs image processing, display processing or the like to the medical image data. Further, the control apparatus 320 transmits the added radiographing order information to the information management apparatus 360.

The information management apparatus 360 is an accepting terminal for the HIS or the RIS, and comprises a control unit such as a CPU or the like, a storage unit, an input unit, a communication control unit or the like. The information management apparatus 360 stores inputted radiographing order information on radiographing of the medical image in a radiographing order information DB (Daba Base), and transmits the inputted radiographing order information to the control apparatus 320 through the network N. The radiographing order information is information for accepting radiographing of the medical image in the medical image radiographing system 300, for example, the patient ID which is an identification code assigned for specifying the patient to be examined for every patient, the patient name, the age, the room, the department, the doctor name in charge, the radiographing condition, the reading apparatus type or the like. Further, information management apparatus 360 adds the radiographing order information transmitted from the control apparatus 310 in the radiographing order information DB, and renews the radiographing order information DB.

The medical image reading apparatus 330 is a cassette type of image reading apparatus using the cassette 350. The cassette 350 incorporates the photostimulable phosphor substance sheet in which a part of radiation energy is accumulated. When an object to be examined is placed between the portable radiographing apparatus 340 and the cassette 350 in the hospital room or the like, and X-ray is irradiated from the portable radiographing apparatus 340 to the cassette 350 side, radiation image information of the object with X-ray is accumulated in the photostimulable phosphor substance sheet. When the cassette 350 used for X-ray radiographing is inserted in the medical image reading apparatus 330, the medical image reading apparatus 330 irradiates excitation light to the photostimulable phosphor substance sheet, converts stimulated light emitted from the sheet photoelectrically, converts an analog image signal obtained to a digital signal, and transmits the signal to the control apparatus 320 through the cable C as medical image data.

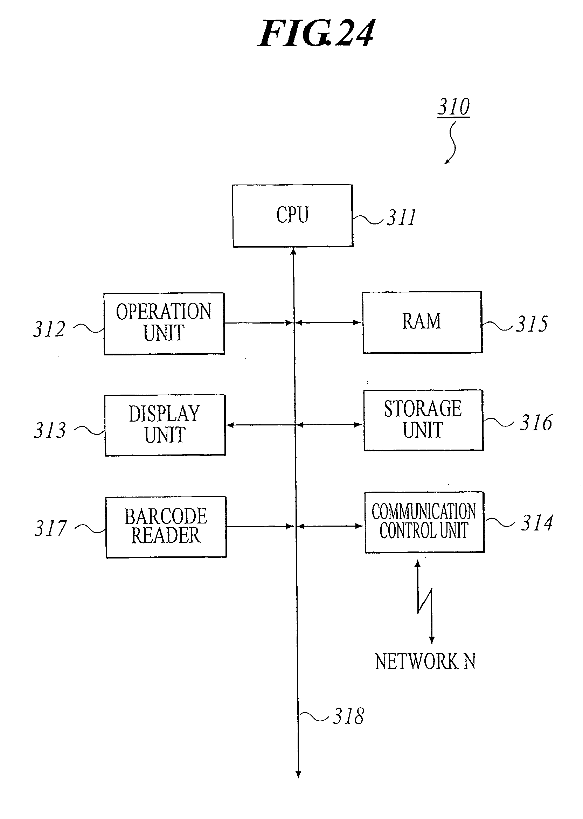

The portable terminal 310 receives the radiographing order information for X-ray radiographing in the room from the control apparatus 320 through the network N. When receiveing the patient ID, the portable terminal 310 searches the radiographing order information of the patient, and displays the radiographing condition on a display unit 323 (shown in FIG. 24). Further, the portable terminal 310 has a radiographing order information addition screen for adding the radiographing order information, and transmits the added radiographing order information to the control apparatus 320. The portable terminal 310 is any type of portable communication terminal, for example, a portable telephone, a PHS (Personal Handy-phone System), a note type PC or the like.

The portable radiographing apparatus 340 is an X-ray radiographing apparatus which is movable. When the object to be examined is placed between the portable radiographing apparatus 340 and the cassette 350, the portable radiographing apparatus 340 radiographs the object with X-ray by irradiating X-ray to the cassette 350.

The network N is a communication network constructed by using a private line or an existing general public network, and can adopt various line states such as a LAN, a WAN or the like, like the network N according to the first and second embodiments. The network N includes various types of communication networks such as a telephone network, the ISDN, a private line, a portable communication network, a communication satellite network, a CATV network or the like, an Internet service provider for connecting them, and the like. Preferably, the network N is a network making secure capable of being accepted by only a specific user in view of confidence in information management. Communication between apparatuses or terminals may be wired or wireless.

Next, each apparatus which is one of main components in the medical image radiographing system 300 will be explained in detail.

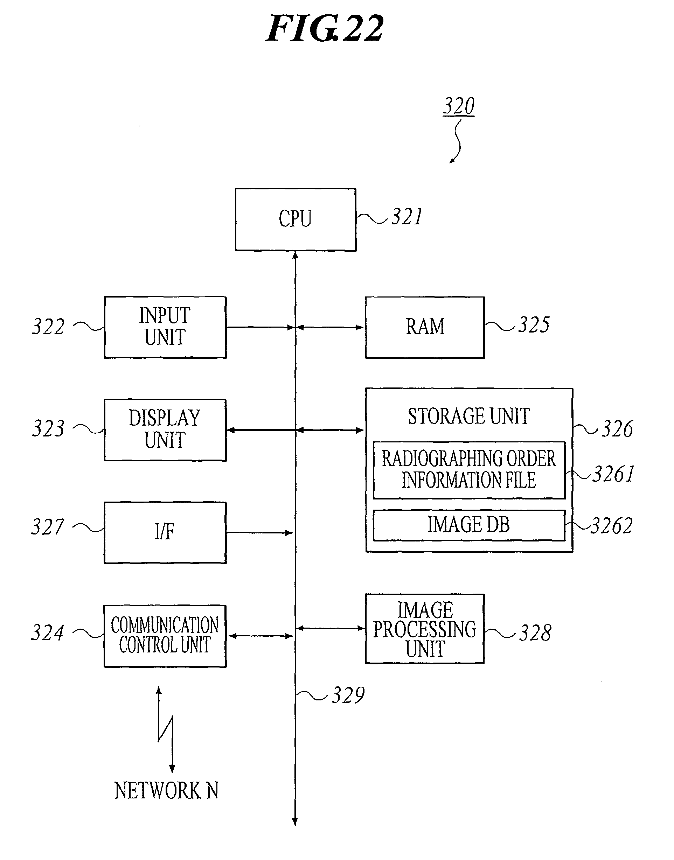

FIG. 22 is a block diagram showing a functional structure of the control apparatus 320. As shown in FIG. 22, the control apparatus 320 comprises a CPU 321, an input unit 322, a display unit 323, an I/F 327, a communication control unit 324, a RAM (Random Access Memory) 325, a storage unit 326, an image processing unit 328 and so on, which are connected to each other through a bus 329, substantially like the control apparatus 120 according to the first embodiment.

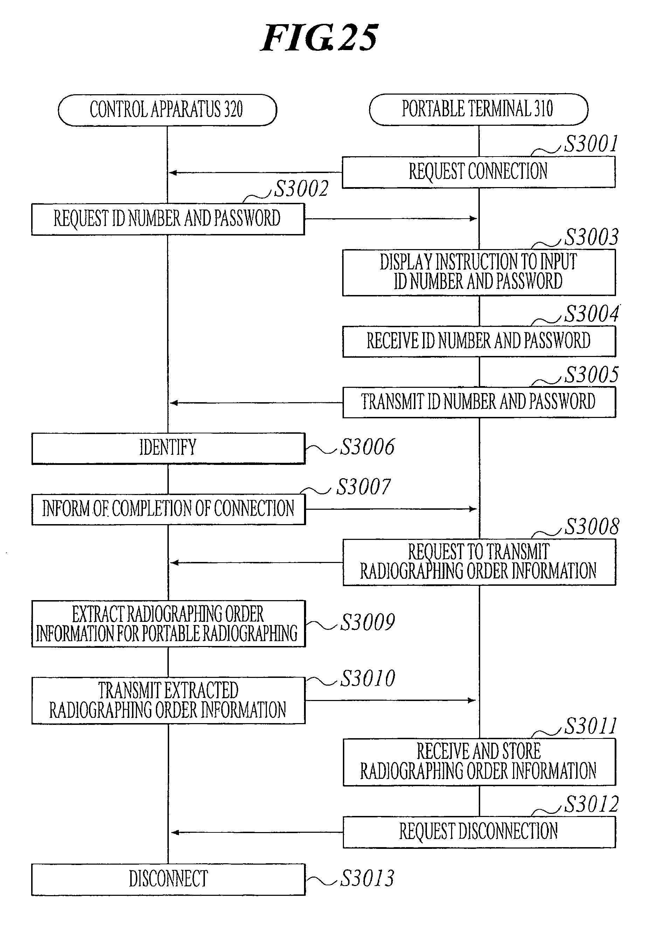

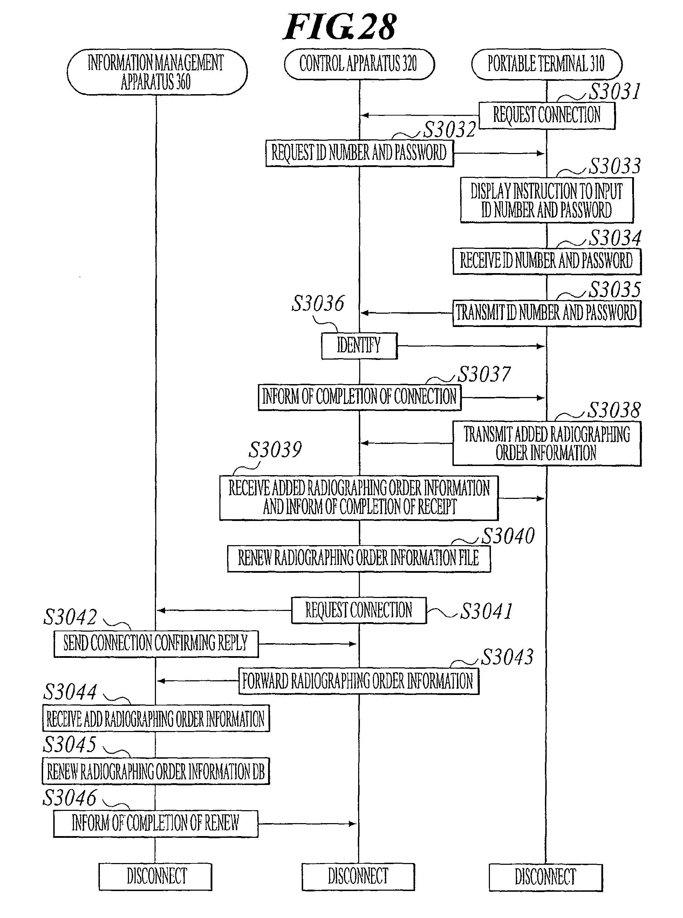

The CPU 321 reads a system program or various control programs stored in the storage unit 326, expands the program in the RAM 325, and controls each unit according to the control program in a concentrated way. Further, the CPU 321 performs various processing such as radiographing order information transmission processing (FIG. 25), or radiographing order information addition processing B (FIG. 28) as follows according to the program expanded in the RAM 325, stores processing results in the RAM 325 temporarily, and displays the processing results on the display 323.

For example, when receiving radiographing order information transmission request from the portable terminal 310, the CPU 321 extracts radiographing order information for radiographing which is not carried out for the "type" of medical image reading apparatus 330 from a radiographing order information file 3261, and transmits the extracted radiographing order information to the portable terminal 310.

Further, when receiving added radiographing order information from the portable terminal 310, the CPU 321 adds the radiographing order information in the radiographing order information file 3261 on the basis of the received content, renews the radiographing order information, and transmits the received radiographing order information to the information management apparatus 360.

Further, the CPU 321 controls the medical image reading apparatus 330 according to the renewed radiographing order information, makes the medical image reading apparatus 330 read radiation image information accumulated in the cassette 350 inserted therein, receives medical image data from the medical image reading apparatus 330, and executes image processing, display processing or the like to the medical image data.

The input unit 322, the display unit 323, the communication control unit 324, the RAM 325 and the storage unit 326 have substantially the same structures and functions as those of the input unit 122, the display unit 123, the communication control unit 124, the RAM 125 and the storage unit 326 of the control apparatus 120 according to the first embodiment, and it is omitted to explain them in detail.

The I/F 327 is an interface for connecting the control apparatus 320 to the medical image reading apparatus 330 through the cable C.

According to the third embodiment, the storage unit 326 stores a radiographing order information file 3261 for storing radiographing order information received from the information management apparatus 360, and an image DB 3262 for storing medical images read by the medical image reading apparatus 330.

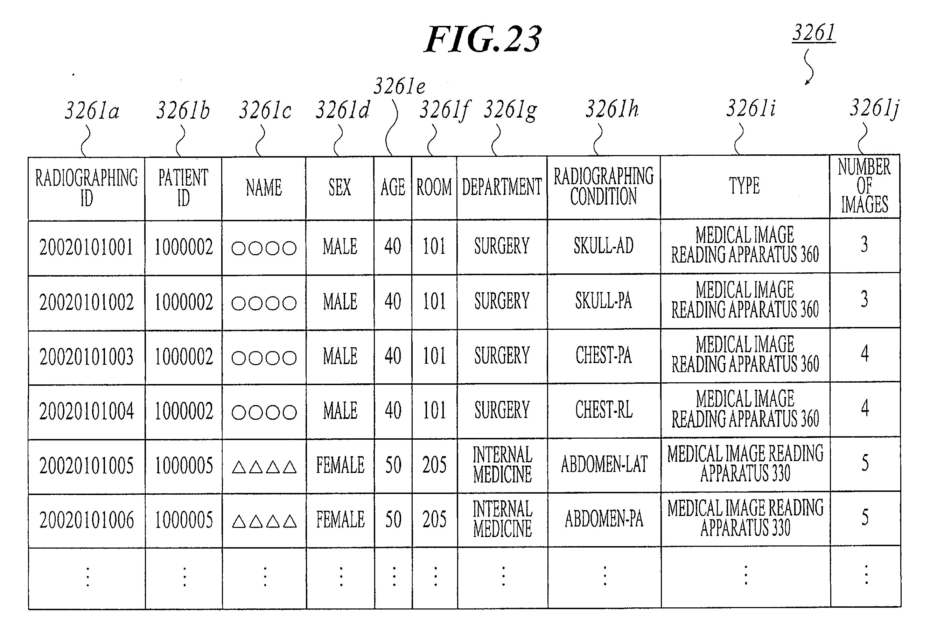

FIG. 23 is a table showing an example of a data structure of the radiographing order information 3261. As shown in FIG. 23, the radiographing order information file 3261 comprises items 3261a to 3261j for storing the radiographing ID, the patient ID, the patient name, the sex, the age, the room, the department, the radiographing condition, the type and the number of images, respectively, and data corresponding to each item are stored in each itme for every radiographing order information.

The items 3261a to 3261h and 3261j for the radiographing ID, the patient ID, the patient name, the sex, the age, the room, the department, the radiographing condition and the number of images store substantially the same data as those stored in the corresponding items of the radiographing order information file 1161 (FIG. 3) according to the first embodiment, and it is omitted to explain them in detail. The item 326i for the type stores data (for example, the medical image reading apparatus 360, the medical image reading apparatus 330) indicating the type of the reading apparatus.

The image processing unit 328 performs various image processing such as frequency processing, gradation processing, rotation processing, enlarge/reduce processing or the like to medical image data according to the instruction outputted from the CPU 321. Further, the image processing unit 328 performs compression processing for compressing image data in a predetermined coding system or expanding processing for decoding and expanding the compressed image data.

Next, an internal structure of the portable terminal 310 will be explained.

FIG. 24 is a block diagram showing a functional structure of the portable terminal 310. As shown in FIG. 24, the portable terminal 310 comprises a CPU 311, an operation unit 312, a display unit 313, a barcode reader 317, a RAM 315, a storage unit 316, a communication control unit 314 and so on, which are connected to each other through a bus 318, substantially like the portable terminal 110 according to the first embodiment.

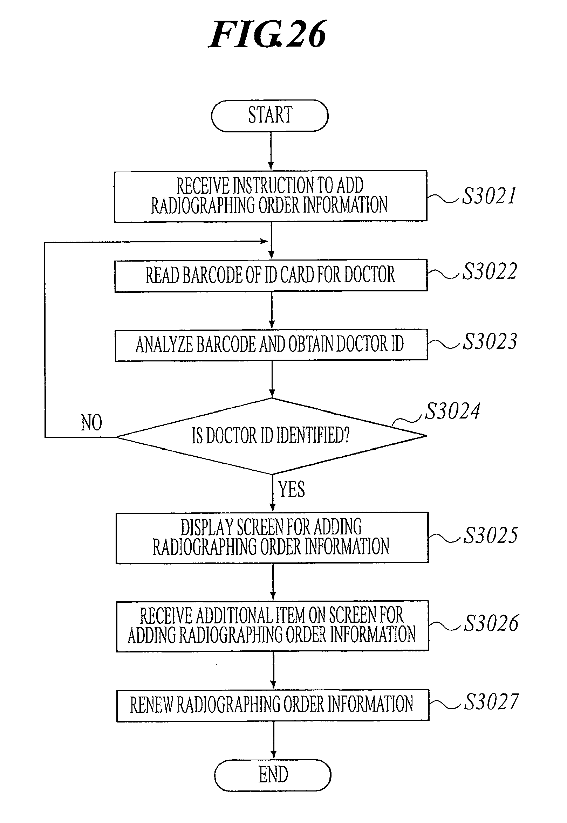

The CPU 311 reads a system program or various control programs stored in the storage unit 316, expands the program in the RAM 315, and controls operation of each unit according to the control program in a concentrated way. Further, the CPU 311 performs various processing such as radiographing order information transmission processing (FIG. 25), radiographing order information addition processing A (FIG. 26), or radiographing order information addition processing B (FIG. 28) according to the program expanded in the RAM 315, stores processing results in the RAM 315 temporarily, and displays the processing results on the display unit 313.

For example, the CPU 311 sends radiographing order information transmission request to the control apparatus 320 through the communication control unit 314, receives radiographing order information transmitted from the control apparatus 320, and stores the radiographing order information in the storage unit 316. Further, the CPU 311 displays the received radiographing order information on the display unit 313.

Further, the CPU 311 controls the barcode reader 317, makes the barcode reader 317 read a barcode displayed on a surface of an ID card for a doctor, analyzes the read barcode, obtaines a doctor ID as identification information of the doctor, and identifies the doctor. After identifying the doctor, the CPU 311 displays radiographing order information addition screens 3131 and 3132 on the display unit 313. When receiving an added matter, the CPU 311 adds the added matter to the radiographing order information stored in the storage unit 316 and renews the radiographing order information on the basis of content of the added matter. Further, the CPU 311 transmits the renewed radiographing order information to the control apparatus 320.

The operation unit 312 comprises a so-called touch panel for outputting position information inputted by bringing a finger or an exclusive stylus pen into touch on a transparent sheet panel covering a screen of the display unit 313, to the CPU 311 as an input signal. Further, the operation unit 312 comprises a power supply button, various function buttons or the like, and outputs a push signal corresponding to the button pushed to the CPU 311.

The display unit 313 comprises a LCD or the like. The display unit 313 displays instructions inputted through the operation unit 312, data or the like, according to an instruction of a display signal outputted from the CPU 311.

The barcode reader 317 comprises a light emitting element such as a semiconductor laser, a LED (light emitting diode) or the like, a photographing element or the like. The barcode reader 317 emits light from the light emitting element, irradiates the light to a predetermined surface, photographs an image in an irradiation direction, and photographs an image of the barcode according to control by the CPU 311. Then, the barcode reader 317 outputs the image photographed by the photographing element to the CPU 311.

The RAM 315 forms a storage area for temporarily storing the system program or the control program executable by the CPU 311 read out of the storage unit 316, inputted or outputted data, parameters or the like according to various processing performed by the CPU 311.

The storage unit 316 comprises a non-volatile semiconductor memory such as a flush memory or the like. The storage unit 316 stores a system program corresponding to the portable terminal 310, various application programs corresponding to the system program, various data, processing results or the like. The storage unit 316 stores the various programs in a readable program code format, and the CPU 311 performs operation according to the program codes in order.

The communication control unit 314 comprises a modem, a terminal adaptor, or the like. The communication control unit 314 controls wireless communication with a communication business company (which is not shown in figures) connected to the network N.

Next, the operation according to the third embodiment will be explained.

First, radiographing order information transmission processing will be explained.

FIG. 25 is a flowchart showing the radiographing order information transmission processing performed by the control apparatus 320 and the portable terminal 310 in the medical image radiographing system 300.

As show in FIG. 25, the CPU 311 of the portable terminal 310 sends connection request to the control apparatus 320 through the communication control unit 314 (Step S3001). When the CPU 321 of the control apparatus 320 receives the connection request from the portable terminal 310, the CPU 321 sends request of an ID number and a password to the portable terminal 310 (Step S3002). Herein, the ID number is an identification code assigned for every operator, for identifying whether the operator of the portable terminal 310 is an permitted person to transmit data between the portable terminal 310 and the control apparatus 320 such as a doctor, a radiologist, an operator instructed or the like or not. When receiving them through the communication control unit 314, the CPU 311 displays an instruction to input the ID number and the password on the display unit 313 (Step S3003). When the ID number and the password are inputted (Step S3004), the CPU 311 transmits the ID number and the password inputted through the communication control unit 314 to the control apparatus 320 (Step S3005). The CPU 321 identifies the ID number and the password received (Step S3006), and informs the portable terminal 310 of the completion of connection (Step S3007). When the ID number and the password inputted in Step S3004 are not those of the person permitted to access to the control apparatus 320, the connection is disconnected.

When receiving the completion of connection from the control apparatus 320, the CPU 311 sends radiographing order information transmission request through the communication control unit 314 to the control apparatus 320 (Step S3008). When receiving the radiographing order information transmission request from the portable terminal 310, the CPU 321 extracts radiographing order information for radiographing to be carried out in the hospital room, for example, radiographing order information having the type item 3261i storing the medical image reading apparatus 330, from the radiographing order information file 3261 (Step S3009), and transmits the extracted radiographing order information through the communication control unit 324 to the portable terminal 310 (Step S3010).

When receiving the radiographing order information from the control apparatus 320, the CPU 311 stores the received radiographing order information in the storage unit 316 (Step S3011). Then, when the CPU 311 sends disconnection request through the communication control unit 314 to the control apparatus 320 (Step S3012), the CPU 321 receives the disconnection request and disconnects the communication (Step S3013).

According to the above-described radiographing order information transmission processing, the control apparatus 320 transmits the radiographing order information to the portable terminal 310. The radiologist or the like takes the portable terminal 310 storing the radiographing order information with him when radiographing the patient with X-ray in the hospital room, makes the portable terminal 310 display the radiographing order information on the display unit 313, and confirms the radiographing condition such as the patient to be radiographed with X-ray, the radiographing order information for every patient, the radiographic part or the like.

Next, radiographing order information addition processing A for adding radiographing order information in the portable terminal 310 will be explained.

FIG. 26 is a flowchart showing the radiographing order information addition processing A performed by the CPU 311 of the portable terminal 310.

As shown in FIG. 26, when receiving an instruction to add radiographing order information through the operation unit 312 (Step S3021), the CPU 311 drives the barcode reader 317, and makes the barcode reader 317 read the barcode displayed on the surface of the ID card for the doctor (Step S3022). The CPU 311 analyzes the read barcode, and obtains information indicated by the barcode, that is the doctor ID (Step S3032). When identifying that the obtained doctor ID is a doctor ID permitted to add radiographing order information (Step S3024; YES), the CPU 311 displays the addition screen for radiographing order information on the display unit 313 (Step S3025). When an additional item is inputted on the addition screen for radiographing order information (Step S3026), the CPU 311 stores the added radiographing order information in the RAM 315 temporarily, adds the added radiographing order information in the radiographing order information stored in the storage unit 316, and renews the radiographing order information (Step S3027). When the doctor ID inputted in Step S3024 is not identified (Step S3024; NO), the CPU 311 displays that the doctor cannot add radiographing order information on the display unit 313, and returns to Step S3022.

The identification in Step S3024 is performed in order to certify that the doctor permits to add radiographing order information. The identification may be performed by transmitting the doctor ID written in an ID card incorporating a contact-type IC or a noncontact type IC to the portable terminal 310, instead of by reading the barcode of the ID card for the doctor as described above. Further, in order to make more secure, the identification may be performed by reading a physical characteristic of the operator such as a fingerprint, a retina or the like, and examining the physical characteristic.

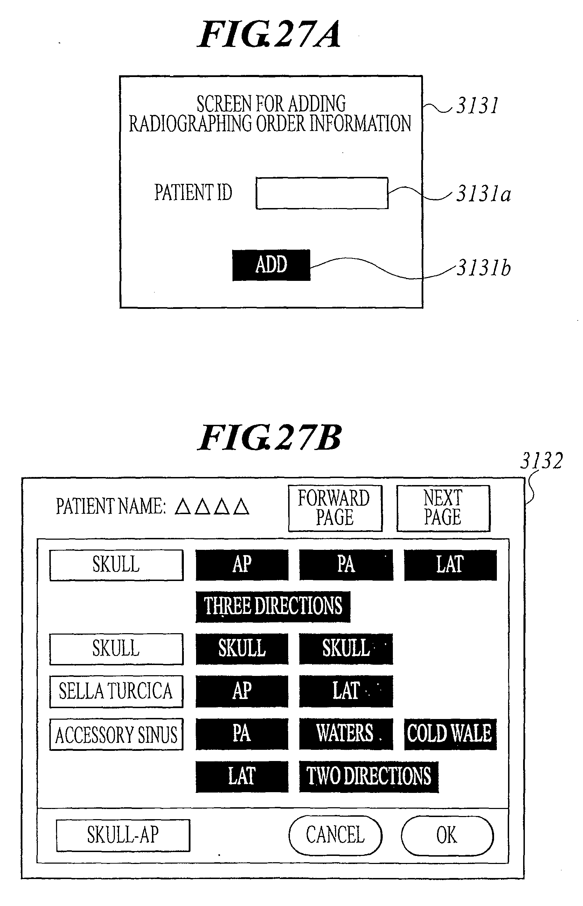

FIG. 27A is a view showing an example of the radiographing order information addition screen 3131 displayed in Step S3025 of FIG. 26. As shown in FIG. 27A, a patient ID input area 3131a for inputting the patient ID, and an add button 3131b for adding the radiographing order information are displayed on the radiographing order information addition screen 3131. When the patient ID is inputted, and the add button 3131b is pushed, the radiographing condition addition screen 3132 shown in FIG. 27B is displayed.

FIG. 27B is a view showing an example of the radiographing condition addition screen 3132 for adding the radiographing condition. As shown in FIG. 27B, the patient name is displayed at an upper section on the radiographing condition addition screen 3132. Therefore, the doctor can confirm the patient to add the radiographing order information. Further, buttons indicating the radiographing condition including the radiographic part and the radiographing direction are displayed at a middle section on the radiographing condition addition screen 3132. Therefore, when one button is selected and pushed, the inputted radiographing condition is displayed on a display area at a lower section on the radiographing condition addition screen 3132. When an OK button is pushed in state the radiographing condition is displayed, the radiographing order information of the radiographing condition inputted is added to the patient displayed at the upper section on the screen 3132.

Next, radiographing order information addition processing B for adding the radiographing order information added by the portable terminal 310 to the control apparatus 320 and the information management apparatus 360 will be explained with reference to a flowchart shown in FIG. 28.

The CPU 311 of the portable terminal 310 sends connection request through the communication control unit 314 to the control apparatus 320 (Step S3031). When receiving the connection request from the portable terminal 310, the CPU 321 of the control apparatus 320 sends request of the ID number and the password to the portable terminal 310 (Step S3032). Herein, the ID number is an identification code assigned for every operator, for identifying whether the operator of the portable terminal 310 is an permitted person to transmit data between the portable terminal 310 and the control apparatus 320 such as a doctor, a radiologist, an operator instructed or the like or not. When receiving them through the communication control unit 314, the CPU 311 displays an instruction to input the ID number and the password on the display unit 313 (Step S3033). When the ID number and the password are inputted (Step S3034), the CPU 311 transmits the ID number and the password inputted through the communication control unit 314 to the control apparatus 320 (Step S3035). The CPU 321 identifies the ID number and the password received (Step S3036), and informs the portable terminal 310 of the completion of connection (Step S3037). When the ID number and the password inputted in Step S3034 are not those of the person permitted to access to the control apparatus 320, the connection is disconnected.

When receiving the completion of connection from the control apparatus 320, the CPU 311 transmites the added radiographing order information through the communication control unit 314 to the control apparatus 320 (Step S3038). When receiving the radiographing order information from the portable terminal 310, the CPU 321 informs the control apparatus 320 of the completion of receipt (Step S3039). The CPU 321 adds the received radiographing order information to the radiographing order information file 3261, renews the radiographing order information file 3261 (Step S3040), and requests the information management apparatus 360 to connect (Step S3041). When receiving the information on the completion of receipt, the CPU 311 disconnects the communication. The control unit of the information management apparatus 360 sends connection confirmation reply to the control apparatus 320 (Step S3042). When receiving the connection confirmation reply, the CPU 321 forwards the radiographing order information outputted from the portable terminal 310 to the information management apparatus 360 (Step S3043). When receiving the forwarded radiographing order information (Step S3044), the information management apparatus 360 adds the received radiographing order information, renews the radiographing order information DB (Step S3045), and informs the control apparatus 320 of completion of renew (Step S3046). When receiving the information on the completion of renew, the CPU 321 disconnects the communication with the information management apparatus 360.

The portable terminal 310 may transmit the radiographing order information to the control apparatus 320 by performing the radiographing order information addition processing B when one radiographing order information is added, or may transmit the radiographing order information at one time when some radiographing order information is addded.

As described above, according to the medical image radiographing system 300, when receiving the radiographing order information transmission request from the portable terminal 310, the control apparatus 320 extracts radiographing order information for X-ray radiographing in a hospital room from the radiographing order information stored in the radiographing order information file 3261, and transmits the extracted radiographing order information to the portable terminal 310. When receiving the instruction to add radiographing order information through the input unit 312, the portable terminal 310 reads the barcode of the ID card for the doctor by the barcode reader 317, identifies the doctor, and displays the screen for adding radiographing order information on the display unit 313. When the radiographing order information is inputted to be added, the portable terminal 310 adds the inputted radiographing order information in the storage unit 316, renews the radiographing order information, and transmits the added radiographing order information to the control apparatus 320. The control apparatus 320 adds the received radiographing order information in the radiographing order information file 3261, renews the radiographing order information file 3261, and forwards the received radiographing order information to the information management apparatus 360. The information management apparatus 360 adds the received radiographing order information in the radiographing order information DB, and renews the radiographing order information DB.

Consequently, it is possible to confirm the radiographing order information in the hospital room, and to add the radiographing order information rapidly and efficiently when it is required to add the radiographing order information in the hospital room. Further, in order to add the radiographing order information, because it is necessary to identify whether the doctor is permitted or not, it is possible to prevent the operator or the like from adding the radiographing order information without the permission of the doctor. Further, it is possible to adjust the content of the radiographing order information between apparatuses.

Although the present invention has been explained according to the above-described third embodiment which is a preferable example of the present invention, it should also be understood that the present invention is not limited to the embodiment.

For example, although it has been explained that the portable terminal 310 receives the radiographing order information from the control apparatus 320, the portable terminal 310 may receive the radiographing order information from the information management apparatus 360 directly.

Further, it should also be understood that the detailed structure and the detailed operation of the medical image radiographing system 300, or each element of the medical image radiographing system 300 according to the embodiment are not limited to the embodiment and various changed and modifications may be made to the invention without departing from the gist thereof.

According to the present invention, when X-ray radiographing is carried out in the hospital room, in case it is necessary to add radiographing order information in the hospital room, it is possible to add radiographing order information rapidly and efficiently. Further, if the identification information on the permitted doctor is not inputted, the screen for adding the radiographing order information is not displayed. Consequently, it is possible to prevent the operator or the like from adding the radiographing order information without the permission of the doctor, and to prevent illegal X-ray radiographing against the Radiologist Law without the permission of the doctor in the room.

Further, the medical image radiographing system comprises the information management apparatus for generating radiographing order information on the basis of radiographing reservation input, and the control apparatus reads radiographing order information from the information management apparatus through the communication network. Consequently, it is possible to input radiographing order information by the external information management apparatus.

Further, when receiving the added radiographing order information transmitted from the portable terminal, the control apparatus adds the received radiographing order information to the stored radiographing order information, and transmits the radiographing order information to the information management apparatus through the network. When receiving the added radiographing order information transmitted from the control apparatus, the information management apparatus adds the received radiographing order information to the stored radiographing order information. Consequently, it is possible to add the radiographing order information added by the portable terminal in the hospital room to the information management apparatus easily and rapidly.

Further, the medical image radiographing system comprises the information management apparatus for generating radiographing order information on the basis of radiographing reservation input, and the portable terminal can read the radiographing order information from the information management apparatus through the communication network. Consequently, it is possible that the portable terminal obtains the radiographing order information without the control apparatus.

Further, the control apparatus extracts radiographing order information for the radiographing using the cassette from the radiographing order information, and transmits the extracted radiographing order information to the portable terminal. Consequently, because only the radiographing order information for the radiographing using the cassette is transmitted to the portable terminal, it is possible to limit data processed by the portable terminal to information required to radiograph with X-ray in the hospital room. As a result, it is possible that the portable terminal performs the processing efficiently.

Further, when radiographing with X-ray in the hospital room, in case it is necessary to add radiographing order information in the hospital room, it is possible to provide the portable terminal capable of adding radiographing order information rapidly and efficiently. Further, according to the portable terminal, if the permitted doctor identification information is not inputted, the screen for adding radiographing order information is not displayed. Consequently, it is possible to prevent the operator or the like from adding radiographing order information without the permission of the doctor.

### [Fourth Embodiment]

According to the above-described first to third embodiments, the medical image radiographing system capable of modifying the radiographing order information in itself in the control apparatus or the portable terminal has been explained. According to a fourth embodiment, a medical image radiographing system capable of obtaining radiographing history information related to the radiographing order information when the control apparatus is not communicated with the portable terminal, will be explained.

Hereinafter, the fourth embodiment of the present invention will be explained in detail, with reference to figures. A definition of the present invention is not limited to the figures, like the cases of the first to third embodiments. As an example of a characteristic embodiment of the present invention, a medical image radiographing system 400 for radiographing in a ward with a portable terminal which is portable and a radiographing apparatus which is portable will be explained.

First, a structure of the fourth embodiment will be explained.

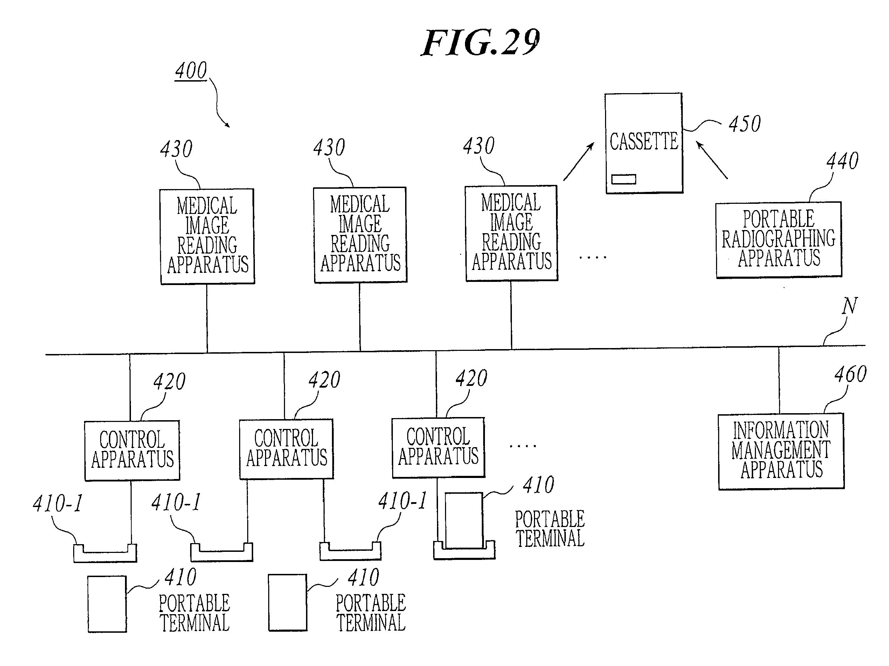

FIG. 29 is a conceptual view showing a system structure of the medical image radiographing system 400 of the present invention. As shown in FIG. 29, the medical image radiographing system 400 comprises a portable terminal 410, a communication terminal 410-1, a control apparatus 420, a medical image reading apparatus 430, a portable radiographing apparatus 440, a cassette 450, an information management apparatus 460 or the like, like the medical image radiographing system 100 according to the first embodiment.

The portable terminal 410 has substantially the same structure and function as those of the portable terminal 110 according to the first embodiment. Further, the portable terminal 410 obtains identification information (hereinafter, it will be called an operator ID.) on the operator, identification information (hereinafter, it will be called a cassette ID.) on the cassette 450, and identification information (hereinafter, it will be called a radiographing apparatus ID.) on the radiographing apparatus, relates them to the radiographing order information, and stores them, when starting radiographing.

The communication terminal 410-1 has substantially the same structure and function as those of the communication terminal 110-1 according to the first embodiment. For example, the communication terminal 410-1 controls transmission of radiographing order information from the control apparatus 420 to the portable terminal 410, or controls transmission of the operator ID, the cassette ID and the radiographing apparatus ID from the portable terminal 410 to the control apparatus 420.

The control apparatus 420 receives radiographing order information from the information management apparatus 460, and manages the medical image on the basis of the received radiographing order information. Further, the control apparatus 420 manages a radiographing history of the medical image on the basis of information received from the portable terminal 410 and the medical image reading apparatus 430. For example, the control apparatus 420 transmits the radiographing order information outputted from the information management apparatus 460 through the communication terminal 410-1 to the portable terminal 410. Further, the control apparatus 420 receives the operator ID, the cassette ID and the radiographing apparatus ID related to the radiographing order information from the portable terminal 410 after radiographing. Further, the control apparatus 420 receives the medical image, and the cassette ID and identification information (hereinafter, it will be called a reading apparatus ID.) on the reading apparatus related to the medical image from the medical image reading apparatus 430. Then, the control apparatus 420 relates the medical image, the cassette ID, and the radiographing apparatus ID and the reading apparatus ID to each other, and manages them, on the basis of the radiographing order information.

The medical image reading apparatus 430 has substantially the same structure and function as those of the medical image reading apparatus 130 according to the first embodiment. For example, the medical image reading apparatus 430 reads the cassette ID attached to the cassette 450, and transmits the cassette ID and the own reading apparatus ID related to the medical image to the control apparatus 420.

The portable radiographing apparatus 440, the cassette 450 and the information management apparatus 460 have substantially the same structures and functions as those of the portable radiographing apparatus 140, the cassette 150 and the information management apparatus 160 according to the first embodiment, respectively, and it is omitted to explain them in detail.

Next, each apparatus which is one of main components in the present invention will be explained in detail.

The portable terminal 410 is substantially the same structure as one of the portable terminal 110 according to the first embodiment, and it is omitted to show it in figures. The portable terminal 410 comprises a CPU 411, an operation unit 412, a display unit 413, an I/F 414 which is the communication section, a RAM 415, a storage unit 416, a barcode reader 417 and so on, which are connected to each other through a bus 418.

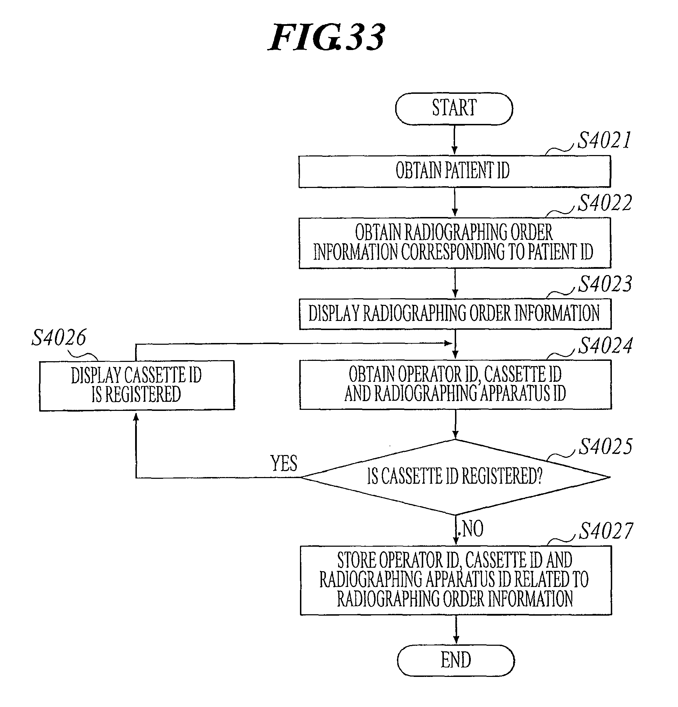

The CPU 411 has substantially the same function as one of the CPU 111 of the portable terminal 110. The CPU 411 reads a radiographing preparation processing program and a radiographing start processing program out of the storage unit 416, and executes radiographing preparation processing (with reference to FIG. 5B) and radiographing start processing (FIG. 33) according the programs. The detail of each processing will be explained as follows.

The display unit 413 has substantially the same structure and function as those of the display unit 113 of the portable terminal 110. The display unit 413 displays various information such as radiographing order information, the obtained patient ID, the cassette ID, the operator ID, the radiographing apparatus ID or the like, on the basis of a display instruction outputted from the CPU 411.

The I/F 414 has substantially the same structure and function as those of the I/F 114 of the portable terminal 110. The I/F 114 transmits the operator ID and the radiographing apparatus ID besides the cassette ID related to the radiographing order information to the control apparatus 420, after radiographing.

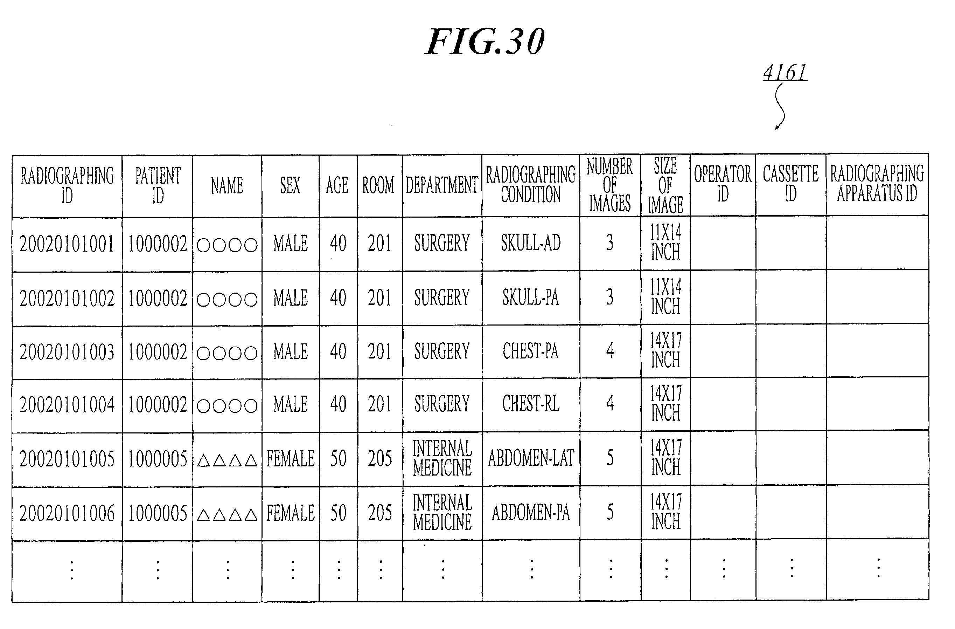

The storage unit 416 has substantially the same structure and function as those of the storage unit 116 of the portable terminal 110. The storage unit 416 stores a radiographing order information file 4161 for storing radiographing order information received from the control apparatus 420. The radiographing order information file 4161 will be explained with reference to FIG. 30.

FIG. 30 is a table showing an example of a data structure of the radiographing order information file 4161. As shown in FIG. 30, the radiographing order information file 4161 has items for storing the radiographing ID, the patient ID, the name, the sex, the age, the room, the department, the radiographing condition, the number of images and the cassette ID, and further a size of the image, the operator ID obtained when starting radiographing and the radiographing apparatus ID, respectively. The radiographing order information file 4161 stores data corresponding to each item for every radiographing order information.

The items for the radiographing ID, the patient ID, the name, the sex, the age, the room, the department, the radiographing condition, the number of images and the cassette ID store the same data as those stored in the corresponding items of the radiographing order information file 1161 (with reference to FIG. 3) according to the first embodiment, and it is omitted to explain them in detail. The item for the size (for example, 11x14 inch, 14x 17 inch or the like) of the image stores the size of the medical image to be radiographed. The item for the operator ID stores an identification code (for example, information read out of the barcode attached to the ID card which the operator takes with him) assigned for every operator to specify the operator which carries out the radiographing. The item for the radiographing apparatus ID stores an identification code (for example, information read out of the barcode attached to the portable radiographing apparatus 440) assigned for every radiographing apparatus to specify the portable radiographing apparatus 440 which radiogaphs the patient.

The data of the operator ID, the cassette ID and the radiographing apparatus ID as described above are not stored in the radiographing order information file 4161 before starting radiographing. Each data read out of the barcode by the barcode reader 417 when starting radiographing are stored in the radiographing order information file 4161.

The radiographing order information may include various patient information, for example, a name of a doctor in charge, warning information for warning infection or the like, drug allergy, pregnancy, additional medical history, special care necessity such as a wheelchair, a stretcher or the like, clinical diagnosis, privileged items or the like, besides the patient ID, the name, the sex and the age, as the patient information. Further, the radiographing order information may include various radiographing information, for example, a radiographing method (simple radiography, contrast radiography or the like), the appointed radiographing date or the like, besides the radiographing condition, the radiographing apparatus, the number of images and the size of the image, as the radiographing information. Further, the radiographing order information may be displayed when a password is inputted as the occasion may demand.

The barcode reader 417 has substantially the same structure and function as those of the barcode reader 117 of the portable terminal 110. The barcode reader 417 is an example of the obtaining section for obtaining the operator ID, the radiographing apparatus ID or the like besides the caste ID, and comprises a scanner which is an optical reading apparatus. For example, the barcode reader 417 obtains the cassette ID by reading the barcode attached to the cassette 450 in which the medical image is recorded, the patient ID by reading the barcode attached to the bedside of the patient or a part of the body of the patient, the operator ID by reading the barcode attached to the ID card or the like for the operator, and the radiographing apparatus ID by reading the barcode attached to the radiographing apparatus, when radiographing.

The operation unit 412 and the RAM 415 have substantially the same structures and functions as those of the operation unit 112 and the RAM 115 of the portable terminal 110, and it is omitted to explain them in detail.

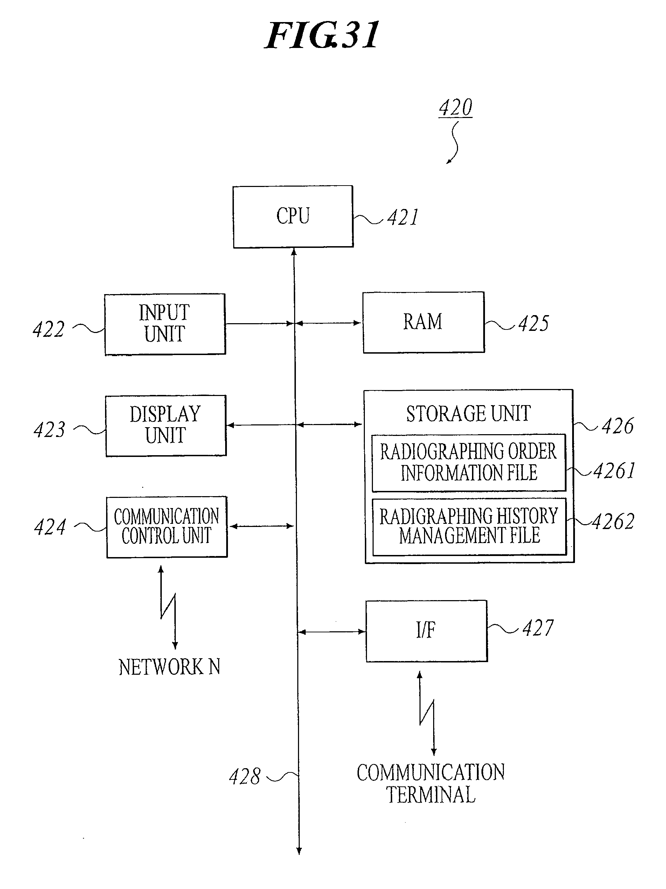

The control apparatus 420, as shown in FIG. 31, has substantially the same structure as one of the control apparatus 120 according to the first embodiment. The control apparatus 420 comprises a CPU 421, an input unit 422 which is the input section, a display unit 423 which is the display, a communication control unit 424, a RAM 425, a storage unit 426, an I/F 427 which is the communication section and so on, which are connected to each other through a bus 428.

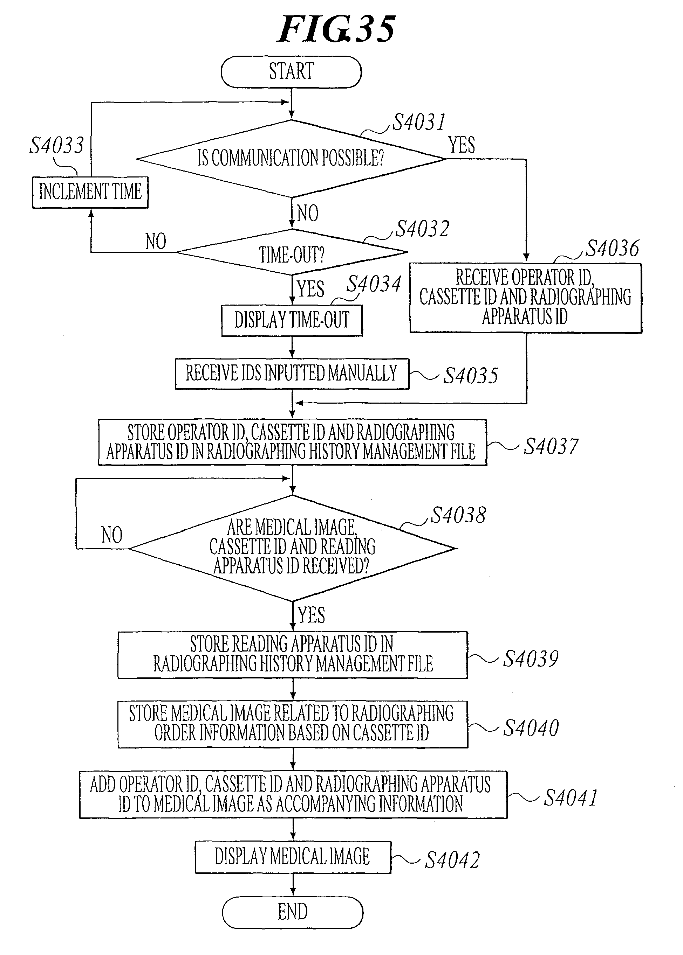

The CPU 421 has substantially the same function as one of the CPU 121 of the control apparatus 120. The CPU 421 reads a radiographing preparation processing program and a radiographing termination processing program out of the storage unit 426, and executes radiographing preparation processing (with reference to FIG. 5A) and radiographing termination processing (FIG. 35) according the programs, as follows. Each processing will be explained in detail , as follows.

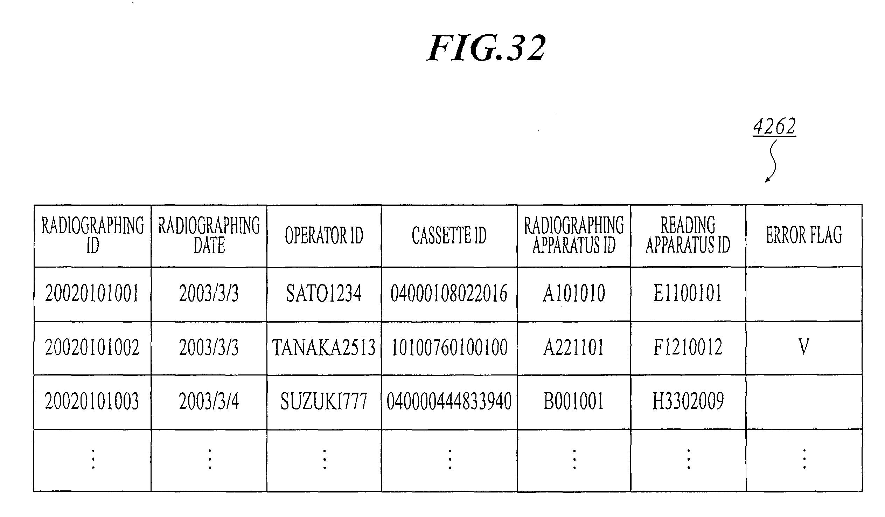

The storage unit 426 has substantially the same structure and function as those of the storage unit 126 of the control apparatus 120. The storage unit 426 stores a radiographing order information file 4261 for storing radiographing order information received from the information management apparatus 460, and a radiographing history management file 4262 for managing radiographing history of the medical image. The radiographing order information file 4261 has substantially the same structure as one of the above-described radiographing order information file 4161 (shown in FIG. 30), and it is omitted to show it in figures or explain it in detail. The radiographing history management file 4262 will be explained with reference to FIG. 32.

FIG. 32 is a table showing an example of a data structure of the radiographing history management file 4262. As show in FIG. 32, the radiographing history management file 4262 has items for storing the radiographing ID, the radiographing date, the operator ID, the cassette ID, the radiographing apparatus ID, the reading apparatus ID and an error flag.

The identification code (for example, 20020101001, ...) assigned for every radiographing to specify radiographing stored in the above-described radiographing order information file 4161 is stored in the item for the radiographing ID. The radiographing ID is related to the above-described radiographing order information. Information (for example, 2003/3/3, ...) indicating the date the medial image is radiographed is stored in the item for the radiographing date.

Identification information (for example, sato1234, ...) assigned for every operator to specify the operator who radiographs the medical image is stored in the item for the operator ID. Identification information (for example, 04000108022016, ...) assigned for every cassette to specify the cassette 450 storing the medical image is stored in the item for the cassette ID. Identification information (for example, A101010, ...) assigned for every radiographing apparatus to specify the radiographing apparatus or the portable radiographing apparatus 440 which radiographs the medical image is stored in the item for the radiographing apparatus ID.

Identification information (for example, E1100101, ...) assigned for every reading apparatus to specify the medical image reading apparatus 430 which reads the medical image out of the cassette 450 is stored in the item for the reading apparatus ID. A check flag for showing whether there occurs error in the medical image or not is stored in the item for the error flag.

The radiographing ID, the radiographing date, the operator ID, the cassette ID and the radiographing apparatus ID as described above, are information transmitted from the portable terminal 410 in the following radiographing termination processing. The reading apparatus ID is information transmitted from the medical image reading apparatus 430. The check of the error flag is information inputted by the operator or the doctor according to circumstances. The information stored in the radiographing history management file 4262 is not limited to the above-described example, and may store various information as necessary.

The I/F 427 has substantially the same structure and function as those of the I/F 127 of the control apparatus 120. For example, the I/F 427 transmits the radiographing order information to the portable terminal 410 before starting radiographing, and receives the operator ID, the cassette ID and the radiographing apparatus ID related to the radiographing order information from the portable terminal 410 after radiographing.

The input unit 422, the display unit 423, the communication control unit 424 and the RAM 425 have substantially the same structures and functions as those of the input unit 122, the display unit 123, the communication control unit 124 and the RAM 125 of the control apparatus 120, and it is omitted to explain them in detail.

Next, the medical image reading apparatus 430 will be explained. The functional structure of the medical image reading apparatus 430 is substantially the same as one of the control apparatus 420 as described above. The same names are attached to the same elements as those of the control apparatus 420, and it is omitted to show them in figures and to explain them in detail. That is, the medical image reading apparatus 430 comprises a CPU 431, an input unit 432, a display unit 433, a communication control unit 434, a RAM 435, a storage unit 436, an image reading unit 437, a barcode reader 438 and so on, which are connected to each other through a bus 439.

The image reading unit 437 irradiates excitation light to a photostimulable phosphor substance layer of the cassette 450, emits phosphor light corresponding to radiation energy accumulated, converts the emitted phosphor light photoelectrically, and obtains radiation image data.

The barcode reader 438 is an example of the obtaining section for obtaining the cassette ID, and comprises a scanner which is an optical reading apparatus. The barcode reader 438 reads a barcode attached on a surface of the cassette 450 installed in the image reading unit 437.

The CPU 431 obtains the medical image read by the image reading unit 437, the cassette ID read by the barcode reader 438, and the reading apparatus ID for assigning the medical image radiographing apparatus reading the medical image, that is, the own apparatus only one-sidedly. The CPU 431 controls the communication control unit 434, relates the cassette ID and the reading apparatus ID to the medical image and transmits them to the control apparatus 420. Herein, preferably, the cassette ID and the reading apparatus ID related to the medical image are transmitted based on, for example, the DICOM (Digital Imaging and Communications Medicine) protocol.

Next, the operation according to the fourth embodiment will be explained.

The program realizable of each function described in the following flowcharts is stored in the storage unit 416 of the portable terminal 410, the storage unit 426 of the control apparatus 420 or the storage unit 436 of the medical image reading apparatus 430 in a format of program codes readable by a computer. Therefore, the CPU 411 of the portable terminal 410, the CPU 421 of the control apparatus 420 or the CPU 431 of the medical image reading apparatus 430 performs the operation according to the program codes of the programs expanded in the RAM 415, the RAM 425 or the RAM 435 in order.

The CPU 421 of the control apparatus 420 and the CPU 411 of the portable terminal 410 perform radiographing preparation processing for transmitting radiographing order information from the control apparatus 420 to the portable terminal 410 in a radiographing room as preparation processing for radiographing.

The CPU 421 of the control apparatus 420 performs the radiographing preparation processing by expanding a radiographing preparation program read by the CPU 421 out of the storage unit 426 in the RAM 425, when triggered by input of an instruction by the operator or the like to execute the radiographing preparation processing to the input unit 422 of the control apparatus 420.

Further, the radiographing order information may be previously transmitted from the information management apparatus 460 to the control apparatus 420 through the network N, and the control apparatus 420 may store it in the storage unit 426. Further, the radiographing order information may be inputted in the control apparatus 420, and the control apparatus 420 may store it in the storage unit 426. Furthermore, the radiographing order information may be displayed on the display unit 423.

The CPU 411 of the portable terminal 410 performs the radiographing preparation processing by expanding a radiographing preparation program read by the CPU 411 out of the storage unit 416 in the RAM 415, when triggered by connecting the portable terminal 410 to the communication terminal 410-1 by the operator or the like. Therefore, the operator radiographing the patient can radiograph and prepare the radiographing with reference to the radiographing order information displayed on the portable terminal 410.

The radiographing preparation processing performed by the CPU 421 of the control apparatus 420 and the CPU 411 of the portable terminal 410 is the same as the processing (shown in FIGS. 5A and 5B) performed by the CPU 121 of the control apparatus 120 and the CPU 111 of the portable terminal 110 according to the first embodiment, and it is omitted to explain it. Further, screens displayed on the display unit 423 of the control unit 420 according to the radiographing preparation processing are the same as the screens (shown in FIGS. 6 to 8B) displayed on the display unit 123 of the control apparatus 120 according to the first embodiment, and it is omitted to explain them.

Next, radiographing start processing for relating the patient ID, the operator ID, the cassette ID and the radiographing apparatus ID to radiographing order information, and recording them, performed by the portable terminal 410 in a room for radiographing before starting radiographing will be explained.

FIG. 33 is a flowchart showing radiographing start processing performed by the portable terminal 410. The CPU 411 performs the radiographing start processing by expanding a radiographing start program read by the CPU 411 out of the storage unit 416 in the RAM 415, when triggered by input of an instruction by the operator or the like to execute the radiographing start processing to the input unit 412 of the portable terminal 410. First, the CPU 411 controls the barcode reader 417, reads the barcode attached to the bed side of the patient or a part of the body of the patient, and obtains the patient ID (Step S4021). Then, the CPU 411 obtains radiographing order information corresponding to the read patient ID out of the storage unit 416 (Step S4022).

Then, the CPU 411 displays the obtained radiographing order information on the display unit 413 (Step S4023), controls the barcode reader 417, reads the barcodes attached to the operator radiographing the patient, the cassette 450 and the portable radiographing apparatus 440, and obtains the operator ID, the cassette ID and the radiographing apparatus ID (Step S4024). Then, the CPU 411 determines whether the cassette ID obtained in Step S4024 is registered or not (Step S4025). The processing in Step S4025 is performed to detect double registration of the cassette in case the operator prepares a plurality of cassettes. More specifically, the CPU 412 determines whether the obtained cassette ID is stored (registered) in the storage unit 416 or not.

When the obtained cassette ID is registered (Step S4025; YES), the CPU 411 displays that the cassette ID is registered on the display unit 413 (Step S4026), and returns to Step S4024. When the obtained cassette ID is not registered (Step S4025; NO), because the obtained cassette ID is not registered, the CPU 411 relates the operator ID, the cassette ID and the radiographing apparatus ID obtained to the radiographing order information, stores them in the storage unit 416 (Step S4027), and terminates the radiographing start processing.

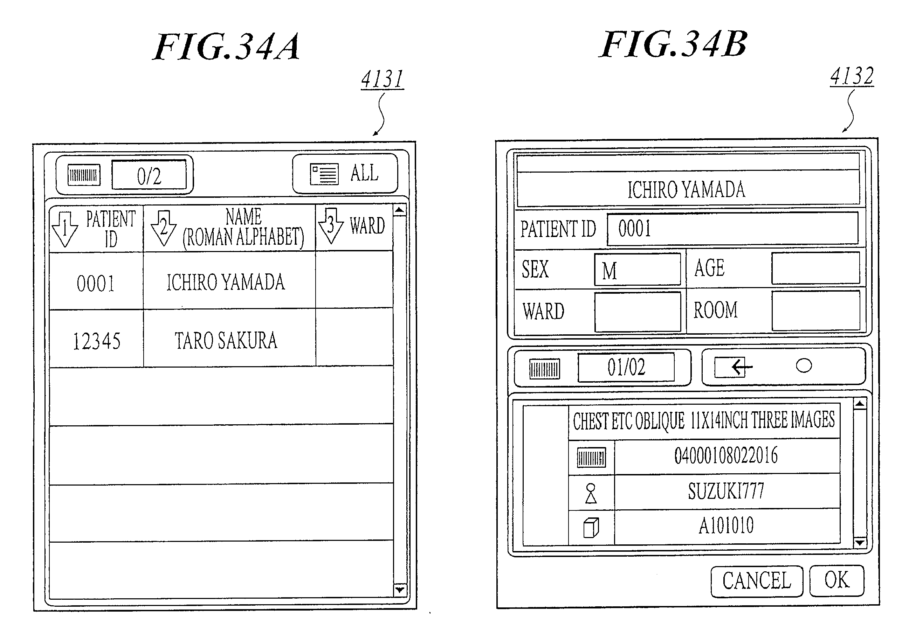

Screens displayed on the display unit 413 of the portable terminal 410 according to the above-described radiographing start processing will be explained with reference to FIGS. 34A and 34B.

FIG. 34A is a view showing a patient list screen 4131 for displaying a list of patients registered in the portable terminal 410. As shown in FIG. 34A, like the patient list screen 1131 (shown in FIG. 10A) according to the first embodiment, an area for showing patient information is provided on the patient list screen 4131. Items for displaying the patient ID, the name and the ward are provided in the area. More specifically, numeral data "0001" are displayed in the item of the patient ID, and text data "Ichiro Yamada" are displayed in the item for the name.

FIG. 34B is a view showing a display screen 4132 for displaying radiographing order information of the patient corresponding to the patient ID read by the barcode reader 417 of the potable terminal 410. As shown in FIG. 43B, like the display screen 1132 (shown in FIG. 10B) for displaying the radiographing order information of the patient according to the first embodiment, an area for displaying patient information and an area for displaying radiographing information are provided on the display screen 4132. Items for displaying the name, the patient ID, the sex, the age, the ward and the room are provided in the area for displaying patient information, and corresponding data are displayed on each item.

Further, items for displaying the radiographing condition and the cassette ID, and further the operator ID, the radiographing apparatus ID, the number of images and the size of the image are provided in the area for displaying radiographing information, and data obtained by the barcode reader 417 of the portable terminal 410 are displayed in the item for the cassette ID, the operator ID and the radiographing apparatus ID at the same time the barcodes are read by the barcode reader 417. More specifically, text data "chest etc oblique 11x14 inch three images" are displayed in the item for the radiographic part and the size of the image, and numeral data "04000108022016" are displayed in the item for the cassette ID. Text data and numeral data "suzuki777" are displayed in the item for the operator ID, and text data and numeral data "A101010" are in the item for the radiographing apparatus ID.

Therefore, the operator radiographs the patient corresponding to the radiographing order information by using the portable radiographing apparatus 440 and the cassette 450 registered according to the radiographing start processing, with reference to the radiographing order information displayed on the portable terminal 410. The portable terminal 410 transmits the operator ID, the cassette ID and the radiographing apparatus ID related to the radiographing order information for radiographing which is carried out to the control apparatus 420. The medical image reading apparatus 430 reads the medical image, the cassette ID and the reading apparatus ID out of the cassette 450 used for radiographing, and transmits them to the control apparatus 420.

Next, radiographing termination processing for obtaining the operator ID, the cassette ID and the radiographing apparatus ID as the radiographing history information related to the radiographing order information for radiographing which is carried out from the portable terminal 410, and obtaining the medical image, the cassette ID and the reading apparatus ID read from the medical image reading apparatus 430 after radiographing in the control apparatus 420, will be explained.

FIG. 35 is a flowchart showing radiographing termination processing performed by the control apparatus 420. As shown in FIG. 35, the CPU 211 performs the radiographing termination processing by expanding a radiographing termination program read by the CPU 421 out of the storage unit 426 in the RAM 425, when triggered by connecting the portable terminal 410 to the communication terminal 410-1. First, the CPU 421 determines whether the communication between the portable terminal 410 and the communication terminal 410-1 (and the I/F 427) is established to communicate with the portable terminal 420 or not (Step S4031). For example, when the operator drops the portable terminal 410, when the I/F 414 of the portable terminal 410 breaks down without an external factor or the like, there may occur the state the communication is impossible.

When the communication is impossible (Step S4031; NO), the CPU 421 determines whether the timer provided in the RAM 425 passes a predetermined time to be time-out or not (Step S4032). When the CPU 421 performs Step S4032 for the first time, the timer of software (program) stored in the RAM 421 is set. The timer is not limited to the software in the present embodiment, but may be a timer of hardware.

When the timer is not time-out (Step S4032; NO), the CPU 421 increments (counts up) the timer of the RAM 425 (Step 4033), and returns to Step S4031. When the timer is time-out (Step S4032; YES), the CPU 421 displays "time-out" showing that the time the communication is not established passes the predetermined time on the display unit 423 (Step S4034). Then, the CPU 421 displays the screen for inputting the operator ID, the cassette ID and the radiographing apparatus ID stored in the communication terminal 410 by the operator or the like on the display unit 423, and receives the IDs inputted through the input unit 422 by the operator or the like (manually) (Step S4035). The operator or the like makes the portable terminal 410 display the operator ID, the cassette ID and the radiographing apparatus ID, and inputs various information through the input unit 422 of the control apparatus 420 with reference to the IDs displayed.

When the communication is possible (Step S4031; YES), the CPU 421 receives the operator ID, the cassette ID and the radiographing apparatus ID through the communication terminal 401-1 from the portable terminal (Step S4036). Then, the CPU 421 relates the operator ID, the cassette ID and the radiographing apparatus ID inputted in Step S4035, or the operator ID, the cassette ID and the radiographing apparatus ID received in Step S4036 to the radiographing order information, stores them in the radiographing history management file 4262, and displays them on the display unit 423 (Step S4037). The CPU 421 sets the receipt (or input) terminatted flag of the radiographing order information for the operator ID, the cassette ID and the radiographing ID inputted or received ON.

Then, when receiving the medical image, the cassette ID and the reading apparatus ID from the medical image reading apparatus 430 (Step S4038; YES), the CPU 421 relates the received reading apparatus ID to the radiographing order information and stores them in the radiographing history management file 4262, on the basis of the cassette ID (Step S4039). The CPU 421 relates the medical image to the radiographing order information and stores them in the storage unit 426, on the basis of the cassette ID related to the medical image and the cassette ID related to the radiographing order information (Step S4040).

Then, CPU 421 adds the operator ID, the cassette ID, the radiographing apparatus ID and the reading apparatus ID corresponding to the medical image, to the medical image as accompanying information (Step S4041). Preferably, the accompanying information to be added to the medical image is added to the medical image according to the DICOM protocol. Then, the CPU 421 displays the received medical image on the display unit 423 (Step S4042), and terminates the radiographing termination processing.

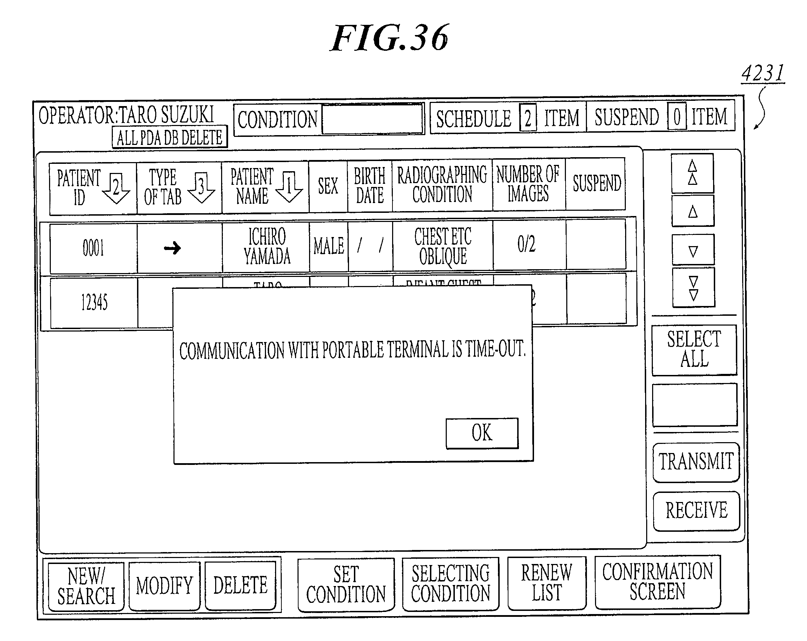

A time-out screen 4231 displayed on the display unit 423 of the control apparatus 420 according to the above-described radiographing termination processing will be explained with reference to FIG. 36. FIG. 36 is a view showing the time-out screen 4231. As shown in FIG. 36, when the communication between the portable terminal 410 and the communication terminal 410-1 is not established, and becomes time-out, the time-out screen 4231 is displayed on the display unit 423. When a "OK" button is selected, a manual input screen (which is not shown in figures) is displayed, and the operator or the like inputs the operator ID, the cassette ID and the radiographing apparatus ID.

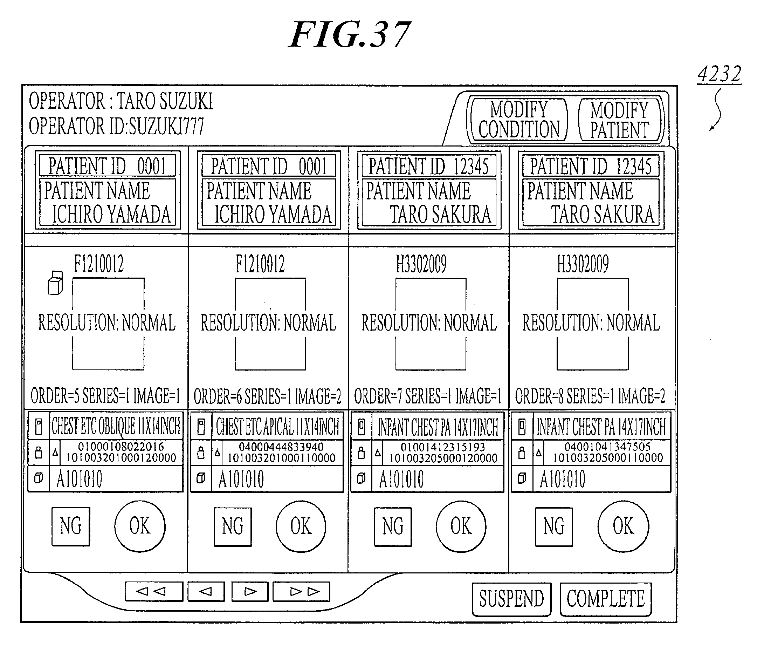

The medical image displayed on the display unit 423 of the control apparatus 420 will be explained with reference to FIG. 37. FIG. 37 is a view showing a portable processing screen 4232 displayed on the display unit 423 when the radiographing is carried out by using the portable terminal 410. As shown in FIG. 37, medical images for a plurality of patients registered in one portable terminal 410 are displayed on one screen of the portable processing screen 4232.

That is, like the portable processing screen 1237 according to the first embodiment, an area for displaying the operator information, and an area for displaying the patient information, the medical image and the radiographing information for every patient are provided on the portable processing screen 4232. Four types of medical images can be displayed on one portable processing screen 4232. More specifically, the item for displaying text data "TARO SUZUKI" as the name of the operator and further an item for displaying text data and numeral data "suzuki777" as the operator ID are provided in the area for displaying the operator information at a left upper end section of the portable processing screen 4232. The medical image displayed at a left end section will be explained as the medical image displayed for every patient. An item for displaying numeral data "0001" as the patient ID and an item for displaying text data "ICHIRO YAMADA" as the name are provided in the area for displaying the patient information.

An area for displaying the medical image is provided at a lower section. The medical image, an item for displaying numeral data "F1210012" as the reading apparatus ID, and an item for displaying text data "NORMAL" as the resolution are provided in the area. Further, an area for displaying the radiographing information is provided at a much lower section. An item for displaying text data "CHEST ETC OBLIQUE 11X14 INCH" as the radiographic part and the size of the image, an item for displaying numeral data "01000108022016" as the cassette ID, and an item for displaying text data and numeral data "A101010" as the radiographing apparatus ID are provided in the area.

Further, instruction buttons for inputting "NG" and "OK" are provided at a lower section of the area for displaying the radiographing information, and it is inputted whether the displayed medical image is abnormal or not. That is, in case the medical image is abnormal, when the "NG" instruction button is instructed through the input unit 422, the error flag of the radiographing history management file 4262 of the corresponding medical image is set ON.

The control apparatus 420 can display a list of identification information such as the operator ID, the cassette ID, the radiographing apparatus ID, the reading apparatus ID or the like for the medical image having the error flag which is set ON, on the basis of the radiographing history file 4262 storing that the medical image is abnormal or not. At the time, for example, the CPU 421 can sort and display the medical image which is abnormal for every identification information. Accordingly, the control apparatus 420 can extract the operator, the cassette, the radiographing apparatus or the reading apparatus wherein the abnormality occurs frequently, trace to the cause of the abnormality accurately, and get rid of the cause rapidly.

As described above, according to the medical image radiographing system 400 in the fourth embodiment, as the radiographing preparation, when the control apparatus 420 transmits the radiographing order information to the portable terminal 410, the portable terminal 410 receives, stores and displays the radiographing order information. Further, before starting radiographing, the portable terminal 410 reads the patient ID of the patient to be examined, the operator ID of the operator radiographing the patient, the cassette ID of the cassette storing the medical image, and the radiographing apparatus ID of the radiographing apparatus recording the medical image, and relates them to the radiographing order information.

After radiographing, the portable terminal 410 transmits the operator ID, the cassette ID and the radiographing apparatus ID related to the radiographing order information to the control apparatus 420. On the other hand, the medical image reading apparatus 430 reads the medical image and the cassette ID out of the cassette 450 recording the medical image, and transmits the cassette ID and the reading apparatus ID related to the medical image to the control apparatus 420.

Then, when the communication with the portable terminal 410 is established, the control apparatus 420 receives the operator ID, the cassette ID and the radiographing apparatus ID. On the other hand, when the communication with the portable terminal 410 is not established, and the time is out, the control apparatus 420 obtains the operator ID, the cassette ID and the radiographing apparatus ID inputted through the input unit 422 manually. The control apparatus 420 relates the operator ID, the cassette ID and the radiographing apparatus ID received from the portable terminal 410 or inputted manually, and the reading apparatus ID received from the medical image reading apparatus 430 to the radiographing order information, stores the operator ID, the cassette ID, the radiographing apparatus ID and the reading apparatus ID in the radiographing history management file 4262, relates the medical image to the radiographing order information on the basis of the cassette ID and manages them.

Accordingly, even when the communication between the portable terminal 410 and the communication terminal 410-1 is not established, because the control apparatus 420 can obtain the operator ID, the cassette ID and the radiographing apparatus ID inputted manually, the control apparatus 420 can obtain information required to manage the radiographing certainly. Consequently, the control apparatus 420 can store and manage the obtained information in the radiographing history file 4262. Further, when the communication between the portable terminal 410 and the communication terminal 410-1 is established, the control apparatus 420 can receive and obtain the operator ID, the cassette ID and the radiographing apparatus ID through the communication terminal 410-1 from the portable terminal 410 certainly. Further, the control apparatus 420 can make the operator or the like input the operator ID, the cassette ID and the radiographing apparatus ID certainly, and obtain them with reference to display of the operator ID, the cassette ID and the radiographing apparatus ID inputted.

Further, in case the communication between the communication terminal 410-1 and the control apparatus 420 is not established, even when the portable terminal 410 is connected to the communication terminal 410-1, the control apparatus 420 does not change at all. In the case, in the control apparatus 420, the operator or the like selects any one of the normal radiographing mode and the portable radiographing mode on a menu screen such as the menu screen 1232 shown in FIG. 6, through the input unit 422, and selects a "new/search" key on a portable list screen or the like such as the portable list screen 1232 shown in FIG. 7. Then, the operator or the like inputs necessary information manually, with watching the screen displayed on the display unit 413 of the portable terminal 410, and inserts the cassette 450 used for radiographing into the medical image reading apparatus 430 after inputting the necessary information.

When information on a plurality of patients is inputted in the potable mode, the images for the patients are displayed on the display unit 423 of the control apparatus 420 after reading the cassette 450 as the portable processing screen 4232 shown in FIG. 37. When the information on the patient is inputted in the normal mode, a plurality of images for one patient are displayed and changed for every patient on the display unit 423, like the normal radiographing.

Although the present invention has been explained according to the above-described fourth embodiment which is an example of the medical image photographing system or the medical image management method suitable to the present invention, it should also be understood that the present invention is not limited to the embodiment.

For example, the system structure of the medical image radiographing system 400 as described above is an example of the present invention, and the present invention is not limited to the system structure. Like the case of the medical image radiographing system 100 according to the first embodiment, the communication terminal 410-1 for controlling the communication between the portable terminal 410 and the control apparatus 420 may not be connected to the control apparatus 420 directly. The communication terminal 410-1 may be connected to the network N, and communicate with a plurality of control apparatuses 420 through the network N arbitrarily. The structure is the same as one of the medical image radiographing system 500 shown in FIG. 15, and it is omitted to show it in figures and to explain it in detail.

Further the radiographing order information transmitted from the control apparatus 420 to the portable terminal 410 may include information (for example, an IP address for identifying an computer on the network or the like) on an apparatus to which the cassette ID related to the radiographing order information for the radiographing which is carried out is transmitted.

Further, after radiographing, in case the communication between the portable terminal 410 and the control apparatus 420 is not established, the display unit 413 of the portable terminal 410 breaks down, and the screen are not displayed on the display unit 413, the operator or the like cannot input the operator ID, the cassette ID and the radiographing apparatus ID through the input unit 422 of the control apparatus 420 with reference to the screen displayed on the display unit 413. In the case, the operator again obtains the cassette ID, the operator ID, the radiographing apparatus ID from the cassette 450 used for radiographing, the operator radiographing the patient and the portable radiographing apparatus 440 used for radiographing, and inputs the information through the input unit 422.

Further, the operator ID, the cassette ID, the radiographing apparatus ID or the like is not limited to be obtained by the barcode reader 417 of the portable terminal 410. The ID card for the operator, the cassette 450 or the portable radiographing apparatus 440 may comprise an IC chip recording identification information, and the portable terminal 410 may obtain the identification information recorded in the IC chip. In the case, the portable terminal 410 may comprise an IC reader (which is not shown in figures).

Further, the information stored in the radiographing history management file 4262 is not limited to the above-described example, and the radiographing history management file 4262 may store various information. For example, the radiographing history management file 4262 may store the radiographing order information, realte an identification number of the portable terminal 410, an identification number of the communication terminal 410-1, an image processing condition when processing the image, an operator ID for processing the image or the like, to the radiographing order information as information regarding a matter participating in the radiographing, and them. Further, the information may be related to the radiographing order information on the basis of various information such as the radiographing ID, the cassette ID, the patient ID or the like. Further, the relationship between the radiographing order information and the medical image may determined on the basis of various information so as to be clear.

Further, the time for obtaining the operator ID, the cassette ID or the radiographing apparatus ID is not limited to the above-described example. If the relationship to the radiographing order information is clear, the operator ID, the cassette ID or the radiographing apparatus ID may be obtained at any time. For example, in the radiographing preparation processing, the operator ID may be included in the radiographing order information transmitted from the control apparatus 420 to the portable terminal 410. In the case, in the radiographing start processing, the portable terminal 410 may identify only the operator ID. Further, in case of post-registration for registering the cassette ID after radiographing, the cassette ID may be not obtained before radiographing.

Further, it should also be understood that the detailed structure and the detailed operation of each element of the medical image radiographing system 400 according to the embodiment are not limited to the embodiment and various changed and modifications may be made to the invention without departing from the gist thereof.

According to the present invention, even when the communication with the portable terminal is not established, because the control apparatus obtains the radiographing history information related to the radiographing order information and inputted, it is possible that the control apparatus obtain the radiographing history information certainly.

Further, when the communication with the portable terminal is established, the control apparatus can obtain the radiographing history information certainly by receiving it from the portable terminal.

Further, because the control apparatus determines whether the communication with the portable terminal is established or not in state the portable terminal is connected to the communication terminal, it is possible that the control apparatus certainly determines whether the communication is established or not.

Further, it is possible to make the operator or the like input the radiographing history information certainly with reference to the display of the inputted radiographing history information. Consequently, it is possible that the control apparatus obtains it certainly.

The medical image radiographying system 100, 200, 300 or 400 may be a CR (Computed Radiography) apparatus, a FPD (Flat Panel Detector), a CT (Computed Tomography) apparatus, a MRI (Magnetic Resonance Imaging) apparatus, an ultrasonography diagnostic apparatus or the like. In the case, the medical image radiographing system may attach the UID (unique ID) determined in the DICOM protocol to each image data and manage it, instead of the cassette ID.

The entire disclosure of Japanese Patent Applications Nos. Tokugan 2002-317258 filed on October 31, 2002, Tokugan 2003-079711 filed on March 24, 2003, Tokugan 2003-079769 filed on March 24, 2003, and Tokugan 2003-091018 filed on March 28, 2003, including specifications, claims, drawings and summaries are incorporated herein by reference in their entireties.

## Claims

1. A medical image radiographing system comprising:
a control apparatus to correlate radiographing order information regarding medical radiographing with a medical image radiographed based on the radiographing order information to manage the radiographing order information and the medical image, wherein
the control apparatus includes:
a control section to correlate the radiographed medical image with radiographing order information including a patient ID, and to generate a portable processing screen which displays a plurality of medical images of a plurality of patients and a plurality of pieces of patient information respectively corresponding to the medical images by using the medical image correlated to the radiographing order information to control a display section to display the portable processing screen when radiographing of a patient using a portable radiographing apparatus is performed in a ward, or to generate an ordinary screen which displays a plurality of medical images of one patient by using the medical image correlated to the radiographing order information to control the display section to display the ordinary screen when radiographing is performed in a radiographing room.

2. The medical image radiographing system of claim 1, wherein
the control apparatus further includes a storage to store the radiographing order information, and
the control section controls the display section to display a portable mode screen when a portable terminal to obtain and display the radiographing order information stored in the storage is used to radiograph a patient.

3. The medical image radiographing system of claim 2, wherein
the portable processing screen displays the medical images regarding a plurality of patients registered in one portable terminal on one screen.

4. The medical image radiographing system of any one of claims 1-3, wherein
the control apparatus further includes a communication terminal which enables the portable terminal to obtain the radiographing order information stored in the storage when the portable terminal is attached to the communication terminal.

5. The medical image radiographing system of any one of claims 1-4, wherein
the control section controls the display section to display the portable mode screen which displays the radiographing order information when radiographing of a parient using the portable radiographing apparatus is performed in a ward or an ordinary mode screen which displays the radiographing order information when radiographing is performed in a radiographing room.
